# EUROPÄISCHE PATENTANMELDUNG

(11) **EP 0 310 909 A2**
(43) Veröffentlichungstag der Anmeldung: **12.04.1989**
(21) Anmeldenummer: 88115823.2
(22) Anmeldetag: 26.09.1988
(51) Int. Cl.: C07D 499/70, C07D 501/20, A61K 31/43, A61K 31/545

(54) **Heteroanellierte Phenylglycin-beta-Lactam-Antibiotika, Verfahren zu ihrer Herstellung und ihre Verwendung als Arzneimittel**

(30) Priorität: 05.10.1987 DE 3733626
(71) Anmelder: BAYER AG, 51368 Leverkusen (DE)
(72) Erfinder: Schmidt, Gunther, Dr., D-5600 Wuppertal 1 (DE); Metzger, Karl Georg, Dr., D-5600 Wuppertal 1 (DE); Zeiler, Hans-Joachim, Dr., D-5620 Velbert 15 (DE)

(57) **Zusammenfassung**

Die Erfindung betrifft β-Lactam-Antibiotika der allgemeinen Formel (I) 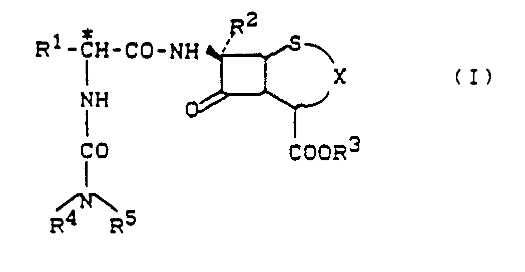 in welcher R¹, R², R³, R⁴ , R⁵ und X die in der Beschreibung angegebene Bedeutung haben, welche heteroanellierte Phenylglycin-Derivate in der Seitenkette besitzen, Verfahren zu ihrer Herstellung sowie ihre Verwendung als Arzneimittel, insbesondere als antibakteriell-wirksame Mittel.

## Beschreibung

Die Erfindung betrifft β-Lactam-Antibiotika, die heteroanellierte Phenylglycin-Derivate in der Seitenkette besitzen, Verfahren zu ihrer Herstellung sowie ihre Verwendung als Arzneimittel, insbesondere als antibakteriell-wirksame Mittel.

Es ist bekannt, daß verschiedene Vertreter von 6-Acylaminopenicillansäuren und 7-Acylaminocephalosporansäure-Derivaten ein sehr breites Wirkungsspektrum besitzen, so z.B. Piperacillin [US-Patent 4 087 424 (1978)], Mezlocillin [DE 21 52 968 (1973)], BRL 36 650 [EP 71 395 (1982)], Cefoperazon (T 1551) [DE 28 41 706 (1978); DE 26 00 880 (1976)].

Die vorliegende Erfindung betrifft heteroanellierte Phenylglycin-ß-Lactam-Antibiotika der allgemeinen Formel (I) 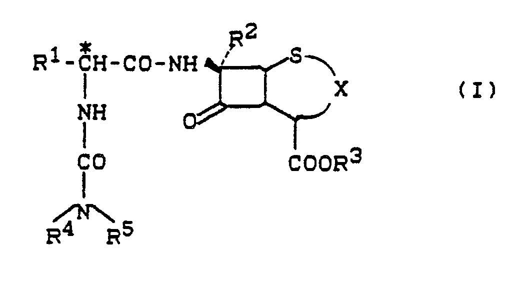 in welcher
R^{I -} für einen Rest der Formel 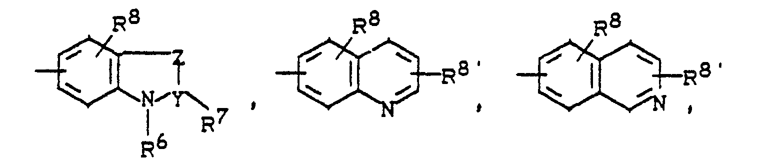 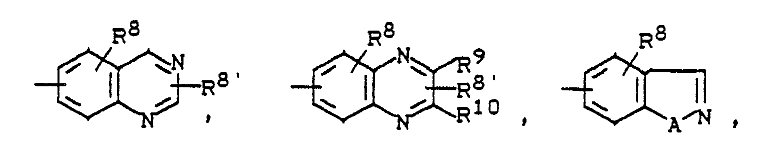 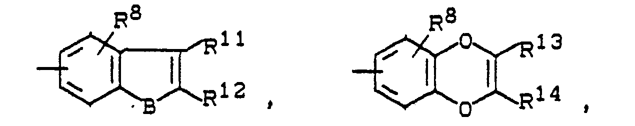 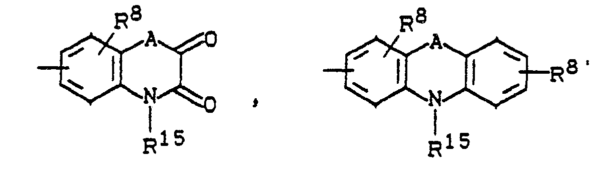 oder 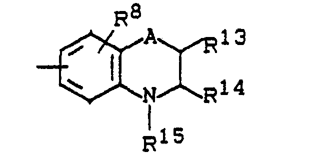 steht, worin
Y - für N oder CR¹⁶ steht,
oder die Gruppierung
Y-R^{7 -} für >= O oder >= N-R⁷ steht,
Z - für 0, S oder -NR¹⁷ steht,
A - für 0, S oder -NR'⁸ steht,
B - für O oder -NR¹⁵ steht,
_{R}⁶ - für Wasserstoff oder
   - für Hydroxy oder Amino steht, oder
   - für geradkettiges, verzweigtes oder cyclisches, gegebenenfalls durch Halogen, gegebenenfalls substituiertes Amino, Hydroxy, Cyano oder C₆-C₁₀-Aryl substituiertes Alkyl oder Alkenyl mit bis zu 10 Kohlenstoffatomen steht, oder
   - für gegebenenfalls substituiertes C₆-C₁₀-Aryl steht,
   R^{7 -} für Wasserstoff steht, oder
   - für gegebenenfalls substituiertes C₆-C₁₀-Aryl steht, oder
   - für geradkettiges, verzweigtes oder cyclisches Alkyl oder Alkenyl mit bis zu 10 Kohlenstoffatomen steht, das gegebenenfalls substituiert ist durch Halogen, Hydroxy, Alkoxy oder Alkoxycarbonyl mit jeweils bis zu 6 Kohlenstoffatomen, Cyano, Carboxy, gegebenenfalls substituiertes C₆-C₁₀-Aryl, Sulfo oder durch eine gegebenenfalls substituierte Aminogruppe,
oder
R⁶ und R⁷ gemeinsam eine Doppelbindung vervollständigen,
R⁸, R^{8'} gleich oder verschieden sind und
   - für Wasserstoff stehen, oder
   - für geradkettiges, verzweigtes oder cyclisches Alkyl, Alkoxy oder Alkylthio mit jeweils bis zu 8 Kohlenstoffatomen stehen, oder
   - für Trifluormethyl oder Trifluormethoxy stehen, oder
   - für Hydroxy, Mercapto, Nitro, Cyano oder Halogen stehen, oder
   - für eine gegebenenfalls substituierte Aminogruppe stehen,
R⁹, R¹⁰ gleich oder verschieden sind und
   - für Wasserstoff stehen, oder
   - für gegebenenfalls substituiertes C₆-C₁₀-Aryl stehen, oder
   - für eine gegebenenfalls substituierte Aminogruppe stehen, oder
   - für Hydroxy stehen, oder
   - für geradkettiges, verzweigtes oder cyclisches Alkoxy mit bis zu 8 Kohlenstoffatomen stehen, oder
   - für Acyl oder Acyloxy mit jeweils bis zu 7 Kohlenstoffatomen stehen, oder
   - für geradkettiges, verzweigtes oder cyclisches, gegebenenfalls substituiertes Alkyl mit bis zu 12 Kohlenstoffatomen stehen.
R¹¹, R¹² gleich oder verschieden sind und
   - für Wasserstoff stehen, oder
   - für gegebenenfalls substituiertes C₆-C₁₀-Aryl stehen, oder
   - für Heterocyclyl stehen, oder
   - für Hydroxy stehen, oder
   - für eine gegebenenfalls substituierte Aminogruppe stehen, oder
   - für geradkettiges, verzweigtes oder cyclisches Alkoxy mit bis zu 8 Kohlenstoffatomen, oder
   - für Acyl oder Acyloxy mit jeweils bis zu 7 Kohlenstoffatomen stehen, oder
   - für Alkoxycarbonyl mit bis zu 8 Kohlenstoffatomen stehen, oder
   - für gegebenenfalls substituiertes geradkettiges, verzweigtes oder cyclisches Alkyl mit bis zu 12 Kohlenstoffatomen stehen,
oder
R¹¹ und R¹² zusammen für die Gruppierung der Formel 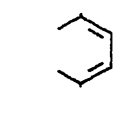 stehen,
R¹³, R¹⁴ gleich oder verschieden sind und
   - für Wasserstoff stehen, oder
   - für gegebenenfalls substituiertes geradkettiges, verzweigtes oder cyclisches Alkyl mit bis zu 12 Kohlenstoffatomen stehen, oder
   - für gegebenenfalls substituiertes C₆-C₁₀-Aryl stehen, oder
   - für Alkoxycarbonyl mit bis zu 8 Kohlenstoffatomen stehen,
R¹⁵ für Wasserstoff, oder
   - für gegebenenfalls substituiertes C₆-C₁₀-Aryl steht, oder
   - für geradkettiges, verzweigtes oder cyclisches Alkyl mit bis zu 8 Kohlenstoffatomen steht,
   R¹⁶ die gleiche Bedeutung hat wie R⁷ und außerdem - für Halogen, oder
   - für geradkettiges, verzweigtes oder cyclisches Alkoxy oder Alkylthio mit jeweils bis zu 8 Kohlenstoffatomen steht, oder
   - für eine gegebenenfalls substituierte Aminogruppe steht, oder
   - für geradkettiges, verzweigtes oder cyclisches Alkylsulfonyl mit bis zu 8 Kohlenstoffatomen steht, oder
   - für Phosphono, Sulfo oder Sulfamoyl steht, oder
   - für Mercapto, Hydroxy, Phenylthio oder Phenoxy steht, oder
   - für Guanidino, Amidino, Hydrazino oder Hydroxylamino steht, oder
   - für gegebenenfalls substituiertes Heterocyclyl steht, oder
   - für gegebenenfalls substituiertes Heterocyclyloxy oder Heterocyclylthio steht,
R¹⁷ die gleiche Bedeutung hat wie R¹⁶, jedoch nicht mit R⁷ eine Doppelbindung vervollständigt,
oder
R¹⁶ und R¹⁷ gemeinsam für eine gegebenenfalls durch Sauerstoff oder Schwefel unterbrochene C₂-C₄-Methylenkette steht
und
R¹⁸ die gleiche Bedeutung hat wie R¹⁵ und mit diesem gleich oder verschieden sein kann,
R^{2 -} für Wasserstoff, oder
   - für geradkettiges, verrzweigtes oder cyclisches Alkoxy oder Alkylthio mit jeweils bis zu 5 Kohlenstoffatomen steht, oder
   - für eine gegebenenfalls substituierte Aminogruppe steht, oder
   - für Formamido steht,
R^{3 -} für Wasserstoff, oder
   - für eine Carboxyschutzgruppe, oder
   - für einen in vivo spaltbaren Esterrest steht,
X - für eine Gruppe der Formel 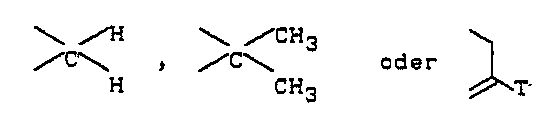 steht, oder die Gruppierung 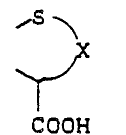 für die Gruppierung 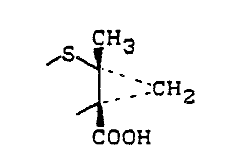 steht, worin worin

T-Wasserstoff oder Halogen bedeutet, oder
- geradkettiges, verzweigtes oder cyclisches Alkoxy oder Alkylthio mit jeweils bis zu 6 Kohlenstoffatomen bedeutet, oder
- geradkettiges, verzweigtes oder cyclisches Alkyl, Alkenyl oder Alkinyl mit jeweils bis zu 7 Kohlenstoffatomen bedeutet, die gegebenenfalls substituiert sein können durch Halogen, Hydroxy, Alkoxy oder Alkylthio mit bis zu 5 Kohlenstoffatomen, Aminocarbonyloxy, Acyloxy mit bis zu 10 Kohlenstoffatomen, oder durch einen Pyridiniumrest, der ein- oder mehrfach substituiert sein kann, oder durch einen Rest der Formel 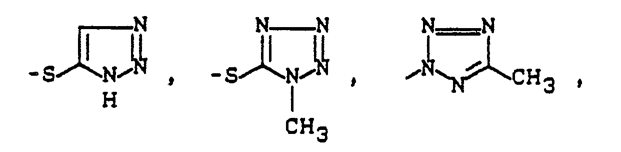 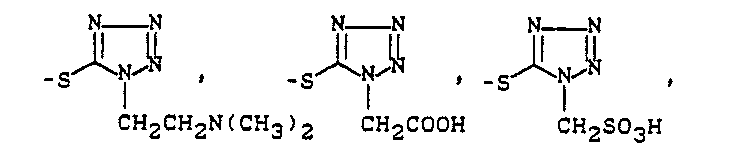 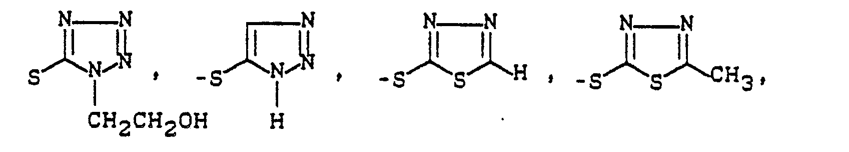 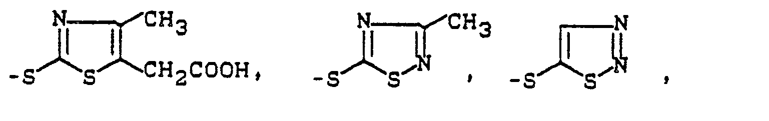 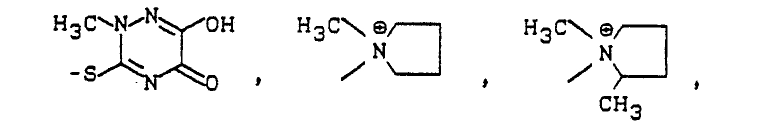 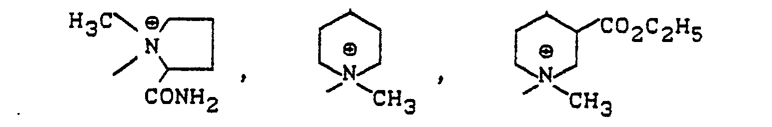 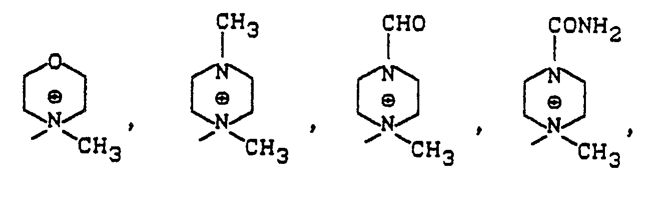 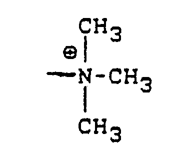 steht,
R^{4 -} für Wasserstoff steht, oder
   - für geradkettiges, verzweigtes oder cyclisches Alkyl mit bis zu 8 Kohlenstoffatomen steht, oder
   - für gegebenenfalls substituiertes C₆-C₁₀-Aryl steht,
R^{S -} für Wasserstoff steht, oder
   - für geradkettiges, verzweigtes oder cyclisches Alkyl mit bis zu 8 Kohlenstoffatomen steht, oder
   - für einen 5- bis 6-gliedrigen heterocyclischen Rest steht, der als Heteroatome ein bis zwei Stickstoffatome, ein Sauerstoff und/oder ein Schwefelatom enthalten kann und der mehrfach gleich oder verschieden substituiert sein kann durch geradkettiges, verzweigtes oder cyclisches Alkyl, Alkenyl, Alkinyl mit jeweils bis zu 6 Kohlenstöffatomen, Cycloalkyl mit 3 bis 7 Kohlenstoffatomen, Alkoxy, Alkenyloxy, Alkylthio, Alkenylthio, Alkylsulfonyl oder Alkenylsulfonyl mit jeweils bis zu 6 Kohlenstoffatomen, durch Aryl, Aryloxy, Arylthio, Arylsulfonyl mit jeweils 6 bis 10 Kohlenstoffatomen, durch eine gegebenenfalls substituierte Aminogruppe, durch Oxo, Hydroxy, Mercapto, Cyano, Nitro, durch Alkylimino mit bis zu 6 Kohlenstoffatomen, oder Arylimino mit 6 bis 10 Kohlenstoffatomen, oder
R⁴ und R⁵ zusammen mit dem Stickstoffatom einen Ring der Formel 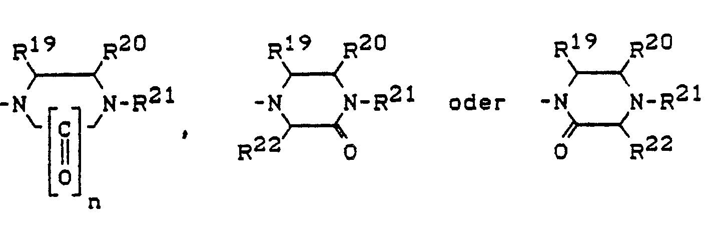 bildet, worin
R'⁹, R²⁰ gleich oder verschieden. sind und Wasserstoff, geradkettiges, verzweigtes oder cyclisches Alkyl oder Alkoxy mit jeweils bis zu 4 Kohlenstoffatomen, Hydroxy, Amino, C₆-C₁₀-Aryl oder Halogen bedeuten, oder
   - die Gruppierung der Formel 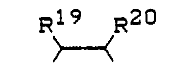 eine Gruppierung der Formel 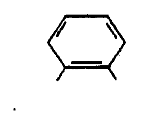 bedeutet,
R²¹- Wasserstoff bedeutet, oder
   - geradkettiges oder verzweigtes Alkyl oder Alkenyl mit jeweils bis zu 10 Kohlenstoffatomen bedeutet, oder
   - Cycloalkyl mit 3 bis 7 Kohlenstoffatomen bedeutet, wobei bei den genannten Resten ein oder zwei CHz-Gruppen durch CO, CS, COz, 0 oder S ersetzt sein können, oder
   - gegebenenfalls substituiertes C₆-C₁₀-Aryl oder C₇-C₁₄-Aralkyl bedeutet, oder
   - einen heterocyclischen Rest der Reihe Thienyl, Furyl, Pyridyl, Pyrimidyl, Oxazolyl, Thiazolyl, Oxadiazolyl, Thiadiazolyl, Benzoxazolyl, Benzthiazolyl, Benzoxadiazolyl, Benzthiadiazolyl, Chinolyl, Isochinolyl oder Indolyl bedeutet, wobei die genannten Heterocyclen bis zu 3-fach gleich oder verschieden substituiert sein können durch geradkettiges, verzweigtes oder cyclisches Alkyl, Alkoxy oder Alkylthio, Alkylsulfonyl mit jeweils bis zu 8 Kohlenstoffatomen, durch Trifluormethyl, Trifluormethoxy, Halogen, Nitro, Cyano, C₆-C₁₀-Aryl oder C7-C14-Aralkyl, oder
R²¹- eine Gruppe der Formel 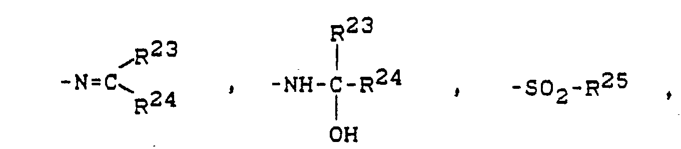 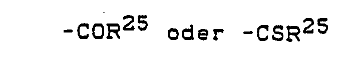 bedeutet,
R²²⁻ Wasserstoff oder geradkettiges, verzweigtes oder cyclisches Alkyl mit bis zu 6 Kohlenstoffatomen bedeutet,
R²³, R²⁴ gleich oder verschieden sind und Wasserstoff, Cyano, Carboxy, Alkoxycarbonyl mit bis zu 6 Kohlenstoffatomen bedeuten, oder
   - geradkettiges oder yerzweigtes Alkyl oder Alkenyl mit jeweils bis zu 8 Kohlenstoffatomen bedeuten, oder
   - Cycloalkyl mit 3 bis 7 Kohlenstoffatomen bedeuten, oder
   - gegebenenfalls substituiertes C₆-C₁₀-Aryl oder CrC14.-Aralkyl bedeuten, oder
   - einen Heterocyclus der Reihe Furyl, Thienyl, Pyridyl, Pyrimidyl, Oxazolyl, Thiazolyl, Oxadiazolyl, Thiadiazolyl, Benzoxazolyl oder Benzthiazolyl bedeuten, wobei die genannten Heterocyclen bis zu 3-fach gleich oder verschieden substituiert sein können durch geradkettiges, verzweigtes oder cyclisches Alkyl, Alkoxy oder Alkylthio mit jeweils bis zu 6 Kohlenstoffatomen, durch Halogen, Nitro, Cyano, Trifluormethyl oder Trifluormethoxy,
oder
R²³ und R²⁴ gemeinsam mit dem Kohlenstoffatom einen 3- bis 7-gliedrigen heterocyclischen Ring bilden, der als Heteroatome bis zu zwei Stickstoffe, ein Sauerstoff und/oder ein Schwefelatom enthalten kann, der gesättigt oder ungesättigt und benzokondensiert sein kann und der bis zu 3-fach gleich oder verschieden substituiert sein kann durch geradkettiges oder verzweigtes Alkyl oder Alkoxy mit jeweils bis zu 8 Kohlenstoffatomen, durch Cycloalkyl mit 3 bis 7 Kohlenstoffatomen, durch Halogen, Cyano, Nitro, Trifluormethyl oder Trifluormethoxy,
und
R²⁵- Hydroxy, geradkettiges oder verzweigtes Alkyl, Alkenyl oder Alkoxy mit jeweils bis zu 8 Kohlenstoffatomen bedeutet, oder
   - Cycloalkyl mit 3 bis 7 Kohlenstoffatomen bedeutet, oder
   - gegebenenfalls substituiertes C_{G}-Cio-Aryl oder C₇-C₁₄-Aralkyl bedeutet, oder
   - eine gegebenenfalls substituierte Aminogruppe bedeutet, oder
   - einen heterocyclischen Ring der Reihe Furyl, Thienyl, Pyridyl, Pyrimidyl, Thiazolyl, Oxazolyl, Thiadiazolyl oder Oxadiazolyl bedeutet, wobei die genannten heterocyclischen Reste bis zu 3-fach gleich oder verschieden substituiert sein können durch geradkettiges oder verzweigtes Alkyl, Alkoxy oder Alkylthio mit jeweils bis zu 6 Kohlenstoffatomen, durch Halogen, Nitro, Cyano, Trifluormethyl oder Trifluormethoxy
   und deren Salze.

Im Rahmen der oben angegebenen Definition steht Aryl bzw. Aralkyl im allgemeinen für einen Phenyl-oder Benzylrest, wobei die Phenylreste 1- bis 4-fach, bevorzugt 1- bis 3-fach gleich oder verschieden substituiert sein können. Als Substituenten seien zu nennen: Halogen, bevorzugt Fluor, Chlor oder Brom, geradkettiges, verzweigtes oder cyclisches Alkyl, Alkoxy, Alkylthio oder Alkylsulfonyl mit jeweils bis zu 10 Kohlenstoffatomen, bevorzugt bis zu 6 Kohlenstoffatomen, Halogenalkyl, Halogenalkoxy, Halogenalkylthio mit jeweils bis zu 7 Kohlenstoffatomen, bevorzugt mit bis zu 5 Kohlenstoffatomen und mit bis zu 5, bevorzugt bis zu 3 Chlor und/oder Fluoratomen, oder Nitro, Cyano, Benzyl, Sulfo, Amidino, Sulfamoyl, Carbamoyl oder eine gegebenenfalls substituierte Aminogruppe.

Gegebenenfalls substituiertes Alkyl im Rahmen der oben angegebenen Definition steht im allgemeinen für geradkettiges, verzweigtes oder cyclisches Alkyl mit bevorzugt bis zu 10 Kohlenstoffatomen, wobei als Substituenten in Frage kommen: Halogen, Alkoxy, Alkylthio mit jeweils bis zu 8 Kohlenstoffatomen, bevorzugt mit bis zu 6 Kohlenstoffatomen, Halogenalkylthio, Halogenalkoxy mit jeweils bis zu 8 Kohlenstoffatomen und bis zu 5, bevorzugt bis zu 3 Fluor- und/oder Chloratomen, Nitro, Cyano, eine gegebenenfalls substituierte Aminogruppe, gegebenenfalls substituiertes Aryl, Sulfo, Sulfamoyl, Alkylsulfonyl mit bis zu 6 Kohlenstoffatomen, bevorzugt mit bis zu 4 Kohlenstoffatomen, Hydroxy, Mercapto, Acyloxy, Acylthio mit jeweils bis zu 7 Kohlenstoffatomen, Carbamoyloxy, Carboxy, Alkoxycarbonyl mit bis zu 8 Kohlenstoffatomen, bevorzugt mit bis zu 6 Kohlenstoffatomen, Phenoxy, Phenylthio, Benzyloxy oder Benzylthio.

Eine gegebenenfalls substituierte Aminogruppe im Rahmen der oben angegebenen Definition steht im allgemeinen für einen Rest der Formel 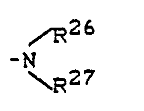 wobei
R²⁶, R²⁷ gleich oder verschieden sind und für Wasserstoff stehen, oder
   - für geradkettiges, verzweigtes oder cyclisches Alkyl mit bis zu 10 Kohlenstoffatomen, bevorzugt mit bis zu 6 Kohlenstoffatomen, stehen, oder
   - für C_{S}-C, o-Aryl, bevorzugt für Phenyl stehen, oder
   - für C₇-C₁₄-Aralkyl, bevorzugt C₇-C₁₀-Aralkyl, besonders bevorzugt für Benzyl stehen, oder
   - für Acyl mit bis zu 10 Kohlenstoffatomen, bevorzugt mit bis zu 8 Kohlenstoffatomen, besonders bevorzugt für Benzoyl oder Acetyl stehen.

Der Begriff Heterocyclyl, Heterocyclyloxy oder Heterocyclylthio steht im Rahmen der oben angegebenen Bedeutung für gesättigte oder ungesättigte, gegebenenfalls über Sauerstoff oder Schwefel gebundene Heterocyclen mit bis zu 3 Stickstoffatomen, einem Sauerstoffatom und/oder einem Schwefelatom, bevorzugt für Pyrrolyl, Pyrrolidinyl, Pyrazolyl, Imidazolyl, Pyridyl, Pyridazinyl, Pyrimidyl, Pyrazinyl, Chinolyl, Isochinolyl, Indolyl, Chinoxalyl, Chinazolyl, Piperidinyl, Morpholinyl, Piperazinyl, Thio morpholinyl, Furyl, Thienyl, Oxazolyl, Thiazolyl, Isoxazolyl, Thiadiazolyl, Triazolyl oder Tetrazolyl.

Sind diese Heterocyclen substituiert dann 1- bis 3-fach, bevorzugt 1- bis 2-fach gleich oder verschieden durch geradkettiges oder verzweigtes Alkyl, Alkylthio, Alkoxy mit jeweils bis zu 4 Kohlenstoffatomen, bevorzugt mit 1 oder 2 Kohlenstoffatomen, Halogen, bevorzugt Fluor, Chlor oder Brom, Nitro, Cyano, Hydroxy, Amino, Trifluormethyl, Trifluormethoxy oder Trifluormethylthio.

Carboxyschutzgruppe im Rahmen der oben angegebenen Definition steht für die in der β-Lactam-Chemie üblichen Carboxyschutzgruppe, Bevorzugt sind leicht abspaltbare Gruppen zu nennen wie zum Beispiel: Methyl, Ethyl, tert.Butyl, Decyl, 2-Chlorethyl, 2,2,2-Trichlorethyl, Cyanoethyl, Diphenylmethyl, Triphenylmethyl, Acetoxymethyl, Allyl, Benzyl, 4-Methoxyphenyl, 4-Nitrobenzyl, 2-Nitrobenzyl, 4-Methoxybenzyl, 2,4-Dimethoxybenzyl, Trimethylsilylethyl, Trimethylsilyl, tert.Butyl-dimethylsilyl, Acetonyl, 1-Phenoxyethyl oder 2-Methyl-2-propenyl.

Steht R³ für einen in vivo leicht abspaltbaren Esterrest so sind hiermit pharmazeutisch verträgliche Esterreste gemeint, die in vivo leicht zu freien Carboxylgruppen (R³ = H) hydrolysiert werden.

Solche Esterreste sind auf dem ß-Lactam-Gebiet gut bekannt. In den meisten Fällen bessern sie die Absorptionseigenschaften der β-Lactam-Verbindungen, Außerdem sollte der Rest R³ von solcher Art sein, daß er einer Verbindung der Formel (I) pharmazeutisch annehmbare Eigenschaften verleiht und bei Spaltung in vivo pharmazeutisch annehmbare Fragmente freisetzt.

Beispiele für solche Gruppen befinden sich in der DE-OS 2 517 316. Bevorzugte in vivo abspaltbare Estergruppen sind die der folgenden Formeln: 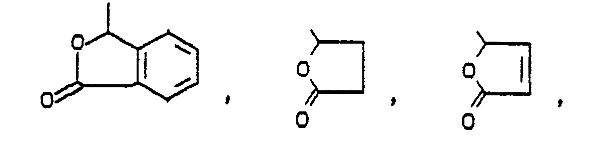 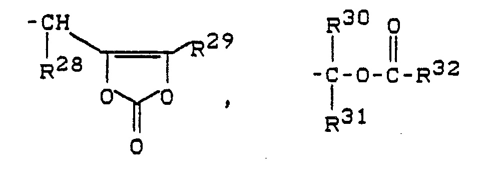 oder 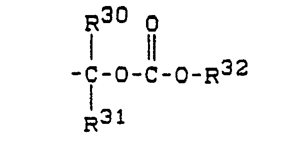 worin
R²⁸ und R²⁹ gleich oder verschieden sind und
   - für Wasserstoff, Phenyl oder
   - für Ci-C₄-Alkyl, bevorzugt für Methyl stehen,
   R³⁰ und R³' gleich oder verschieden sind und
   - für Wasserstoff oder
   - für C₁-C₄-Alkyl, bevorzugt Methyl stehen
und
R^{32 -} für C₁-C₆-Alkyl, bevorzugt für C₁-C₄-Alkyl steht.

Die erfindungsgemäßen Verbindungen der allgemeinen Formel (I) können je nach Bedeutung von R³ und T als freie Säuren, als Ester, als innere Salze 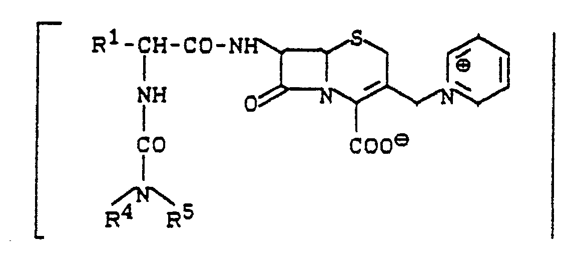 Beispiel für inneres Salz oder als nicht-toxische, physiologisch verträgliche Salze mit einem Kation 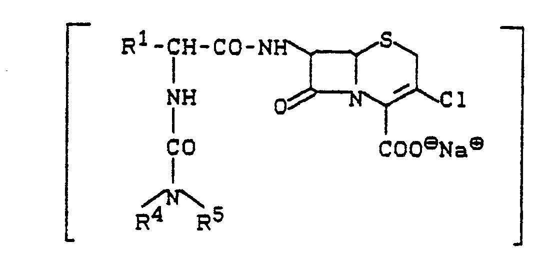 Beispiel für Salz mit Gegenkation oder wenn T ein positiv geladener Rest ist, als nicht-toxische, physiologisch verträgliche Salze mit einem Gegenanion 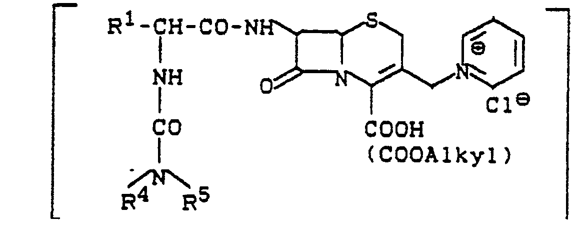 Beispiel für Salz mit Gegenanion vorliegen.

Als Gegenkationen sind bevorzugt Alkali- oder Erdalkalikationen wie beispielsweise Natrium-, Kalium-, Magnesium- oder Calciumionen zu nennen, oder Aluminium- oder Ammoniumionen, sowie nicht-toxische substituierte Ammoniumionen aus Aminen wie Diniedrigalkylamine, Triniedrigalkylamine, Prokain, Dibenzylamin, N,N'-Dibenzylethylendiamin, N-Benzyl-ß-phenyl-ethylamin, N-Methylmorpholin, 1-Ephenamin, Dihydroabiethylamin, N,N'-Bisdihydroabiethylethylendiamin, N-Niedrigalkylpiperidin oder andere Amine, die zur Bildung von Salzen von β-Lactamverbindungen verwendet werden können.'

Als Gegenanionen sind bevorzugt anorgänische oder organische Säurereste zu nennen, wie beispielsweise Chlorid, Bromid, lodid, Sulfat, Hydrogensulfat, Phosphat, Hydrogenphosphat, Carbonat, Hydrogencarbonat, oder Sulfonate wie Methylsulfonat, Ethylsulfonat, Toluolsulfonat, Benzolsulfonat, Naphthalindisulfonat, oder Carboxylate wie Acetat, Formiat, Oxalat, Tartrat, Citrat, Maleat, Fumarat, Benzoat, Succinat oder Lactat.

Wegen der Anwesenheit des mit bezeichneten asymmetrischen Kohlenstoffatoms (siehe Formel I), schließen die erfindungsgemäßn β-Lactam-Antibiotika der allgemeinen Formel (I) die D-, L- und D,L-Formen ein. Sowohl die Diastereomerengemische, als auch die D-Form und die L-Form der erfindungsgemäßen Verbindungen, können zur Behandlung bakterielle Infektionskrankheiten eingesetzt werden. Besonders bevorzugt ist die D-Form der erfindungsgemäßen Verbindungen.

Bevorzugt seien Verbindungen der allgemeinen Formel (1) genannt,
in welcher
R¹ - für eine Gruppe der Formel 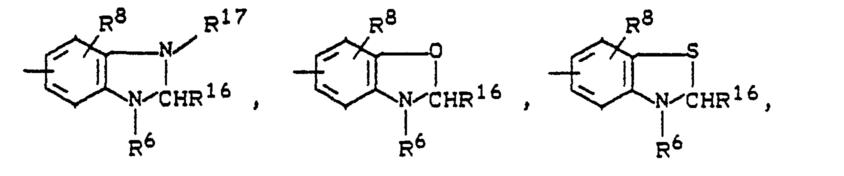 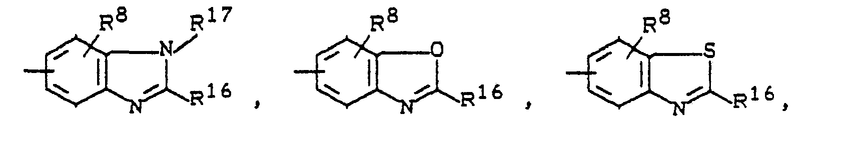 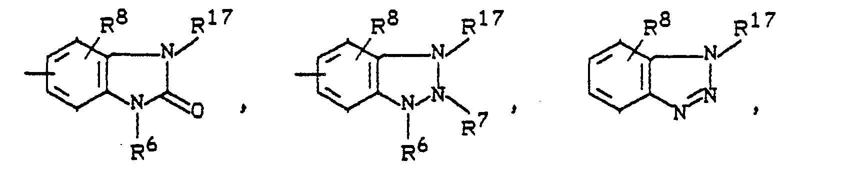 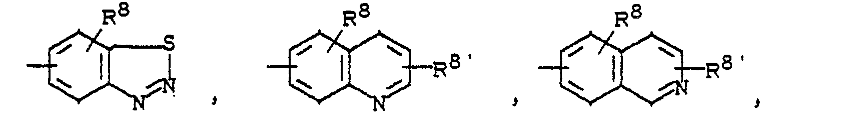 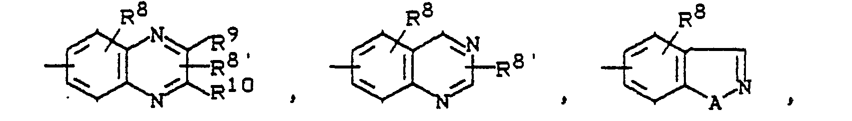 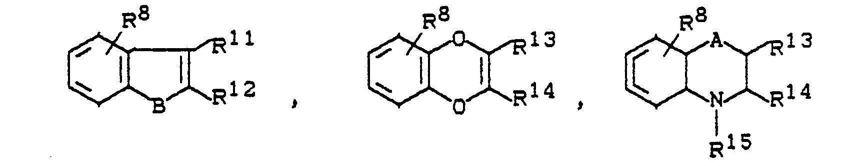 oder 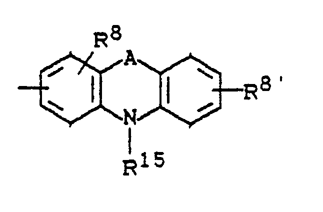 steht, worin
R^{6 -} für Wasserstoff steht, oder
   - für Hydroxy oder Amino steht, oder
   für geradkettiges, verzweigtes oder cyclisches gegebenenfalls durch ein oder mehrere Fluor, Chlor, Brom, gegebenenfalls substituiertes Amino, Hydroxy oder Phenyl substituiertes Alkyl oder Alkenyl mit jeweils bis zu 8 Kohlenstoffatomen steht, oder
   - für gegebenenfalls substituiertes Phenyl steht,
R^{7 -} für Wasserstoff steht, oder
   - für gegebenenfalls substituiertes Phenyl steht, oder
   - für geradkettiges, verzweigtes oder cyclisches Alkyl oder Alkenyl mit jeweils bis zu 8 Kohlenstoffatomen steht, das gegebenenfalls' substituiert ist, durch ein oder mehrere Fluor, Chlor, Brom, Alkoxy mit bis zu 4 Kohlenstoffatomen, Hydroxy, Carboxy, Phenyl, Sulfo oder eine gegebenenfalls substituierte Aminogruppe, R⁸, R⁸' gleich oder verschieden sind und
   - für Wasserstoff stehen, oder
   - für geradkettiges oder verzweigtes Alkyl, Alkoxy oder Alkylthio mit jeweils bis zu 6 Kohlenstoffatomen stehen, oder
   - für Trifluormethyl oder Trifluormethoxy stehen, oder
   - für Hydroxy, Mercapto, Nitro, Cyano, Fluor, Chlor oder Brom stehen, oder
   - für eine gegebenenfalls substituierte Aminogruppe stehen.
R⁹, R¹⁰ gleich oder verschieden sind und für Wasserstoff stehen, oder
   - für Phenyl stehen, das gegebenenfalls ein- bis zweifach gleich oder verschieden substituiert ist durch Fluor, Chlor, Brom, Alkyl, Alkoxy, Alkylthio mit jeweils bis zu 4 Kohlenstoffatomen, Halogenalkyl, Halogenalkoxy, Halogenalkylthio mit jeweils bis zu 3 Kohlenstoffatomen und mit einem bis drei Fluor, durch Nitro, Cyano, Amino oder Dimethylamino, oder
   - für eine gegebenenfalls substituierte Aminogruppe mit der oben angegebenen Bedeutung stehen, oder
   - für Hydroxy oder Alkoxy mit bis zu 6 Kohlenstoffatomen stehen, oder
   - für Benzyloxy oder Alkanoyloxy mit bis zu 4 Kohlenstoffatomen stehen, oder
   - für geradketttiges, verzweigtes oder cyclisches Alkyl mit bis zu 8 Kohlenstoffatomen stehen, das gegebenenfalls substituiert ist durch ein oder mehrere Fluor, Chlor, Brom, Alkoxy, Alkyl thio mit jeweils bis zu 4 Kohlenstoffatomen, Halogenalkoxy, Halogenalkylthio mit jeweils bis zu 4 Kohlenstoffatomen und ein bis drei Fluor, Nitro, Cyano, eine gegebenenfalls substituierte Aminogruppe mit der oben angegebenen Bedeutung, Phenyl, Sulfo, Sulfamoyl, Alkylsulfonyl mit bis zu 2 Kohlenstoffatomen, Hydroxy, Mercapto, Benzoyloxy, Alkanoyloxy mit bis zu 4 Kohlenstoffatomen, Carbamoyloxy oder Alkoxycarbonyl mit bis zu 4 Kohlenstoffatomen,
R¹¹, R¹² gleich oder verschieden sind und
   - für Wasserstoff stehen, oder
   - für Phenyl stehen, das gegebenenfalls ein- bis zweifach gleich oder verschieden substituiert ist durch Fluor, Chlor, Brom, Alkyl, Alkoxy oder Alkylthio mit jeweils bis zu 4 Kohlenstoffatomen, Halogenalkyl, Halogenalkoxy, Halogenalkylthio mit jeweils bis zu 3 Kohlenstoffatomen und mit ein bis drei Fluor, durch Nitro, Cyano, Dimethylamino oder Amino, oder
   - für Hydroxy stehen, oder
   - für Pyridyl, Thienyl, Furyl oder Pyrimidyl stehen, oder
   - für eine gegebenenfalls substituierte Aminogruppe mit der oben angegebenen Bedeutung stehen, oder
   - für geradkettiges, verzweigtes oder cyclisches Alkoxy mit bis zu 6 Kohlenstoffatomen stehen, oder
   - für Benzoyloxy, Alkanoyloxy mit bis zu 4 Kohlenstoffatomen stehen, oder
   - für Benzoyl oder Acetyl stehen, oder
   - für Alkoxycarbonyl mit bis zu 6 Kohlenstoffatomen stehen, oder
   - für geradkettiges, verzweigtes oder cyclisches Alkyl mit bix zu 8 Kohlenstoffatomen stehen, das gegebenenfalls substituiert ist durch ein bis drei Fluor, Chlor, Brom, Alkoxy, Alkylthio mit jeweils bis zu 4 Kohlenstoffatomen, Halogenalkoxy, Halogenalkylthio mit jeweils bis zu 4 Kohlenstoffatomen und mit einem bis drei Fluor, Nitro, Cyano, eine gegebenenfalls substituierte Aminogruppe mit der oben angegebenen Bedeutung, Phenyl, Sulfo, Sulfamoyl, Alkylsulfonyl mit bis zu 2 Kohlenstoffatomen, Hydroxy, Mercapto, Benzyloxy, Alkanoyloxy mit 'bis zu 4 Kohlenstoffatomen, Carbamoyloxy, Alkoxycarbonyl mit bis zu 4 Kohlenstoffatomen, Phenyloxy, Phenylthio, Benzyloxy oder Benzylthio,
oder
R¹¹ und R'² zusammen für eine Gruppjerung der Formel 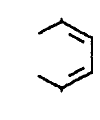 stehen,
R¹³, R'⁴ gleich oder verschieden sind und - für Wasserstoff stehen, oder
   - für geradkettiges, verzweigtes oder cyclisches Alkyl mit bis zu 8 Kohlenstoffatomen stehen, das gegebenenfalls substituiert ist durch ein oder mehrere Fluor, Chlor, Brom, Alkoxy, Alkylthio mit jeweils bis zu 4 Kohfenstoffatomen, Halogenalkoxy, Halogenalkylthio mit jeweils bis zu 4 Kohlenstoffatomen und mit einem bis drei Fluor, Nitro, Cyano, eine gegebenenfalls substituierte Aminogruppe mit der oben angegebenen Bedeutung, Phenyl, Sulfo, Sulfamoyl, Alkylsulfonyl mit bis zu 2 Kohlenstoffatomen, Hydroxy, Mercapto, Benzoyloxy, Alkanoyloxy mit bis zu 4 Kohlenstoffatomen, Carbamoyloxy oder Alkoxycarbonyl mit bis zu 4 Kohlenstoffatomen, oder - für Phenyl stehen, das gegebenenfalls ein- bis zweifach gleich oder verschieden substituiert ist durch Fluor, Chlor, Brom, Alkyl, Alkoxy oder Alkylthio mit jeweils bis zu 4 Kohlenstoffatomen, Halogenalkyl, Halogenalkoxy oder Halogenalkylthio mit jeweils bis zu 3 Kohlenstoffatomen und einem bis drei Fluor, Nitro, Cyano, Amino oder Dimethylamino, oder
   - für Alkoxycarbonyl mit bis zu 6 Kohlenstoffatomen stehen,
A - für 0, S oder -NR'⁸ steht,
B - für 0 oder -NR'^{s} steht,
R¹⁵ für Wasserstoff, oder
   - für Phenyl, oder
   - für geradkettiges, verzweigtes oder cyclisches Alkyl mit bis zu 6 Kohlenstoffatomen steht,
   R¹⁶ die gleiche Bedeutung hat wie R⁷ und außerdem - für Fluor, Chlor, Brom steht, oder
   - für Alkoxy, Alkylthio oder Alkylsulfonyl mit jeweils bis zu 6 Kohlenstoffatomen steht, oder - für eine gegebenenfalls substituierte Aminogruppe steht, oder
   - für Phosphono, Sulfo, Sulfamoyl, Hydroxy, Mercapto, Phenylthio oder Phenyloxy steht, oder - für Guanidino, Hydrazino oder Hydroxylamino steht, oder
   - für gegebenenfalls substituiertes Heterocyclyl, Heterocyclyloxy oder Heterocyclylthio steht,
R¹⁷ die gleiche Bedeutung hat wie R'⁶, jedoch nicht mit R⁷ eine Doppelbindung vervollständigt,
oder
R16 und R¹⁷ gemeinsam für eine gegebenenfalls durch Schwefel unterbrochene C₂-C₄-Methylenkette stehen,
und
R¹⁸ die gleiche Bedeutung hat wie R¹⁵ und mit diesem gleich oder verschieden sein kann,
R^{2 -} für Wasserstoff, oder
   - für Methoxy oder Methylthio steht, oder
   - für Amino, Alkylamino oder Dialkylamino mit jeweils bis zu 3 Kohlenstoffatomen je Alkylgruppe, Phenylamino, Benzylamino oder Acetylamino steht, oder
   - für Formamidino steht,
R^{3 -} für Wasserstoff steht, oder
   - für Methyl, Ethyl, tert.Butyl, 2-Chlorethyl, 2,2,2-Trichlorethyl, Cyanoethyl, Diphenylmethyl, Triphenylmethyl, Acetoxymethyl, Allyl, Benzyl, 4-Methoxybenzyl, 2,4-Dimethoxybenzyl, 1-Phenoxyethyl, 2-Methyl-2- propenyl, 4-Nitrobenzyl, 2-Nitrobenzyl, Trimethylsilylethyl oder tert.Butyl-dimethylsilylethyl steht, oder
   - für einen Rest der Formal 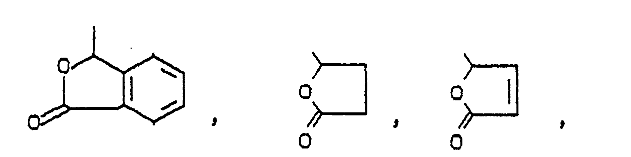 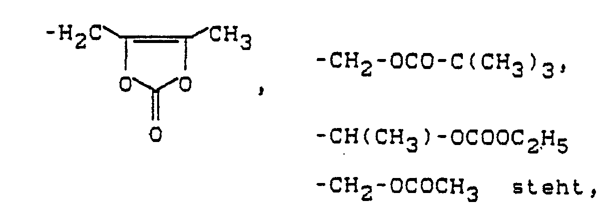 oderX - für eine Gruppe der Formel 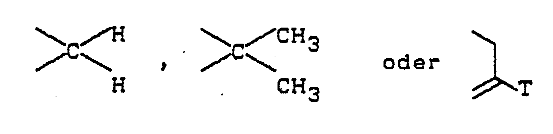 steht, oder die Gruppierung 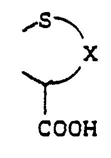 für die Gruppierung 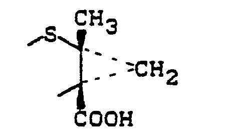 steht, worin
T - Wasserstoff oder Fluor, Chlor oder Brom bedeutet, oder
   - geradkettiges, verzweigtes oder cyclisches Alkoxy oder Alkylthio mit jeweils bis zu 3 Kohlenstoffatomen bedeutet, oder
   - geradkettiges oder verzweigtes Alkyl oder Alkenyl mit jeweils bis zu 5 Kohlenstoffatomen bedeutet, das gegebenenfalls substituiert ist durch ein oder mehrere Fluor, Chlor, Brom, Alkoxy oder Alkylthio mit jeweils bis zu 3 Kohlenstoffatomen, Aminocarbonyloxy, Acetyloxy, Benzoyloxy oder durch einen Rest der Formel 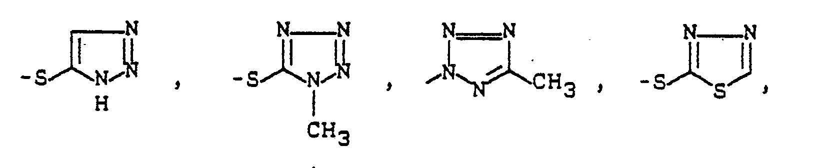 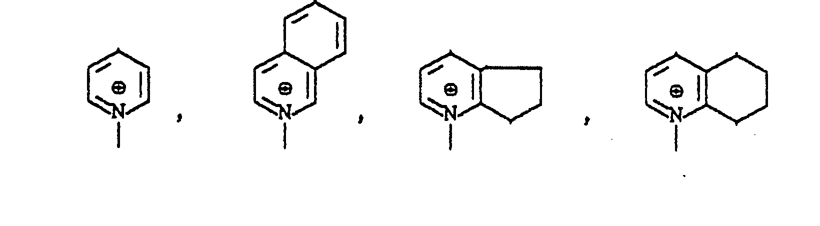 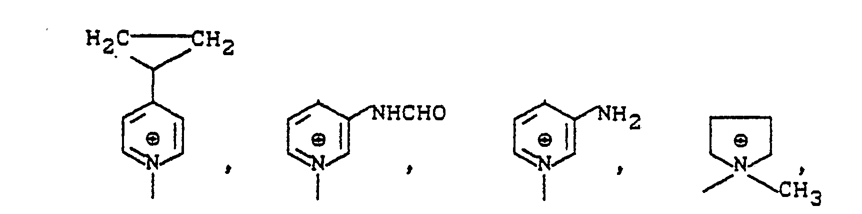 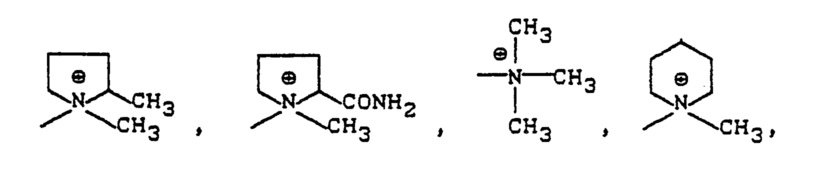 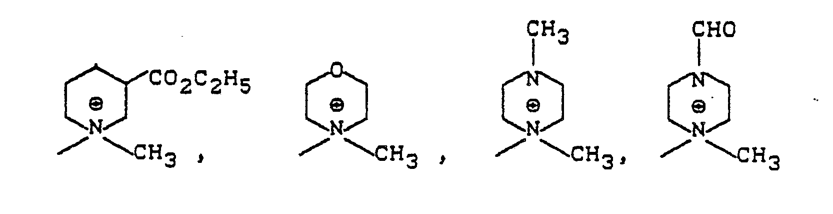 oder 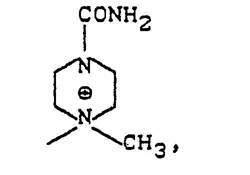
R^{4 -} für Wasserstoff steht, oder
   - für geradkettiges oder verzweigtes Alkyl mit bis zu 4 Kohlenstoffatomen steht, oder
   - für Phenyl steht,
   R^{S -} für Wasserstoff steht, oder
   - für geradkettiges oder verzweigtes Alkyl mit bis zu 4 Kohlenstoffatomen steht, oder
   - für einen 5- bis 6-gliedrigen heterocyclischen Ring steht, der als Heteroatome ein Stickstoff-, Sauerstoff-oder Schwefelatom enthalten kann und der bis zu 2-fach gleich oder verschieden substituiert sein kann durch Alkyl oder Alkenyl mit bis zu 4 Kohlenstoffatomen, durch Cyclopropyl, Cyclopentyl oder Cyclohexyl, durch Alkoxy, Alkenyloxy, Alkylsulfonyl oder Alkenylsulfonyl mit jeweils bis zu 4 Kohlenstoffatomen, Phenyl, Phenyloxy, Phenylsulfonyl oder durch Amino, Alkylamino oder Dialkylamino mit jeweils bis zu 3 Kohlenstoffatomen je Alkylgruppe, durch Oxo, Alkylimino mit bis zu 3 Kohlenstoffatomen oder Phenylimino,
oder
R⁴ und R⁵ zusammen mit dem Stickstoffatom einen Ring der Formel 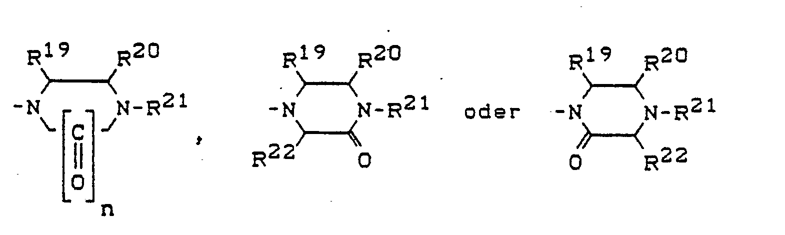 bilden, worin
R'⁹, R²⁰ gleich oder verschieden sind und Wasserstoff, Methyl, Ethyl, Methoxy, Phenyl, Fluor oder Chlor bedeuten,
R²¹ Wasserstoff, oder
   - geradkettiges oder verzweigtes Alkyl mit bis zu 6 Kohlenstoffatomen bedeutet, oder
   - geradkettiges oder verzweigtes Alkenyl mit bis zu 6 Kohlenstoffatomen bedeutet, oder
   - Cycloalkyl mit 3 bis 6 Kohlenstoffatomen bedeutet, wobei bei den genannten Resten eine Methylengruppe durch CO, SO₂, 0 oder S ersetzt werden kann, oder
   - Phenyl oder Benzyl bedeutet, oder
   - einen heterocyclischen Rest der Reihe Thienyl, Furyl, Pyridyl, Pyrimidyl, Oxazolyl, Thiazolyl, Oxadiazolyl, Thiadiazolyl, Benzoxazolyl, Benzthiazolyl, Benzdiazolyl, Benzthiadiazolyl bedeutet, wobei die genannten heterocyclischen Reste bis zu 2-fach gleich oder verschieden substituiert sein können durch geradkettiges oder verzweigtes Alkyl, Alkoxy, Alkylthio oder Alkylsulfonyl mit jeweils bis zu 6 Kohlenstoffatomen, durch Trifluormethyl, Trifluormethoxy, Fluor, Chlor, Brom, Cyano, Phenyl oder Benzyl, oder
   - eine Gruppe der Formel 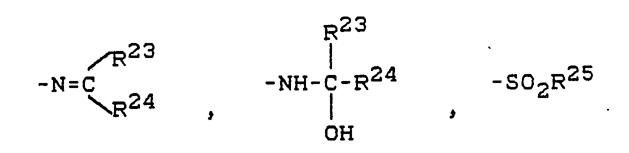 oder 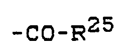 bedeutet, worin
R²²⁻ Wasserstoff, oder geradkettiges oder verzweigtes Alkyl mit bis zu 4 Kohlenstoffatomen bedeutet,
_{R}²3, _{R}²⁴ gleich oder verschieden sind und Wasser stoff, oder
   - Alkoxycarbonyl mit bis zu 4 Kohlenstoffatomen bedeuten, oder
   - geradkettiges oder verzweigtes Alkyl oder Alkenyl mit bis zu 6 Kohlenstoffatomen bedeuten, oder
   - Cycloalkyl mit 3 bis 6 Kohlenstoffatomen bedeuten, oder
   - Phenyl oder Benzyl bedeuten, oder
   - einen heterocyclischen Rest der Reihe Furyl, Pyridyl, Pyrimidyl, Thienyl, Oxazolyl oder Thiazolyl bedeuten, die gegebenenfalls bis zu 2-fach gleich oder verschieden substituiert sein können durch Alkyl oder Alkoxy mit jeweils bis zu 4 Kohlenstoffatomen, Fluor, Chlor, Brom, Nitro, Cyano, Trifluormethyl oder Trifluormethoxy,
oder
R²³, R24 gemeinsam mit dem Kohlenstoffatom einen 5-oder 6-gliedrigen heterocyclischen Ring bilden, der als Heteroatome bis zu zwei Stickstoffatome, ein Sauerstoffatom und/oder ein Schwefelatom enthalten kann, der gesättigt oder ungesättigt sein kann und der bis zu 2-fach gleich oder verschieden substituiert sein kann durch Alkyl oder Alkoxy mit bis zu 6 Kohlenstoffatomen, Cyclopropyl, Cyclopentyl oder Cyclohexyl, oder durch Fluor, Chlor, Brom, Cyano, Nitro, Trifluormethyl oder Trifluormethoxy
R²⁵ für Hydroxy oder Alkoxy mit bis zu 4 Kohlenstoffatomen steht, oder
   - für Alkyl oder Alkenyl mit bis zu 6 Kohlenstoffatomen steht, oder
   - für Cycloalkyl mit 3 bis 6 Kohlenstoffatomen steht, oder
   - für gegebenenfalls substituiertes Phenyl oder Benzyl steht, oder ,
   - für gegebenenfalls substituiertes Amino steht, oder
   - für einen heterocyclischen Rest der Reihe Furyl, Pyridyl, Pyrimidyl, Thiazolyl, Oxazolyl, Thiadiazolyl oder Oxadiazolyl steht, wobei die genannten Heterocyclen bis zu 2-fach gleich oder verschieden substituiert sein können durch Alkyl oder Alkoxy mit bis zu 4 Kohlenstoffatomen, Fluor, Chlor, Brom, Nitro, Cyano, Trifluormethyl oder Trifluormethoxy,

und deren Salze.

Besonders bevorzugt seien Verbindungen der allgemeinen Formel (I) genannt,
in welcher
R^{I -} für eine Gruppe der Formel 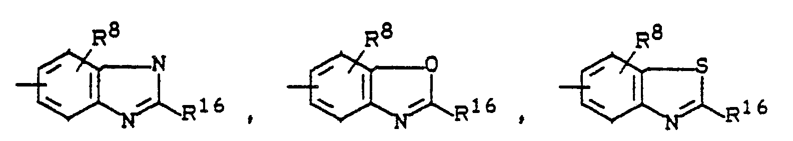 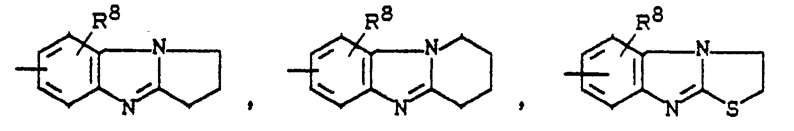 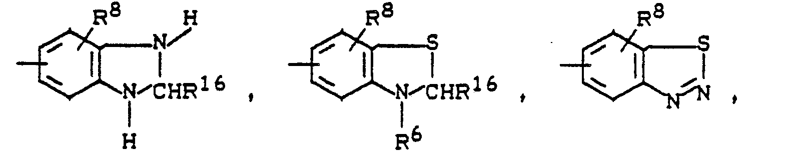 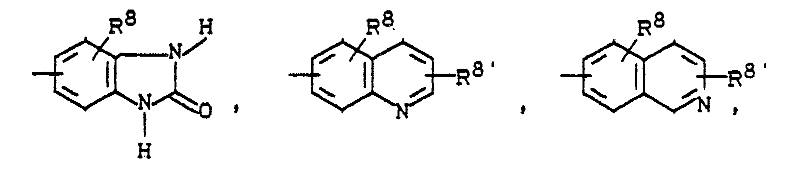 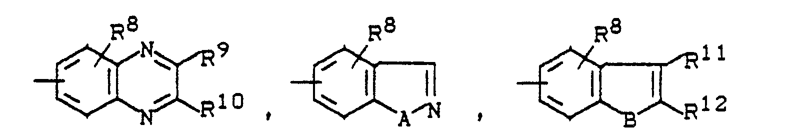 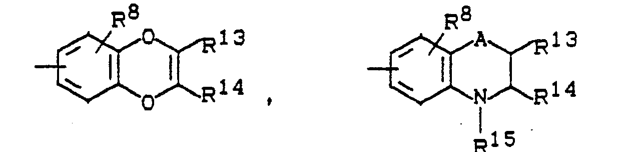 oder 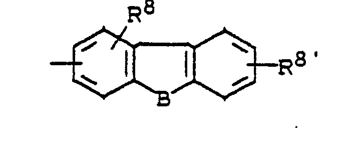 steht, worin
R⁶ - für Wasserstoff steht, oder
   - für geradkettiges, verzweigtes oder cyclisches, gegebenenfalls durch Fluor, Amino, Hydroxy oder Phenyl substituiertes Alkyl oder Alkenyl mit bis zu 6 Kohlenstoffatomen steht, oder
   - für gegebenenfalls substituiertes Phenyl steht,
   R⁸, R^{8'} gleich oder verschieden sind und
   - für Wasserstoff stehen, oder
   - für geradkettiges oder verzweigtes Alkyl, Alkoxy oder Alkylthio mit jeweils bis zu 4 Kohlenstoffatomen stehen, oder
   - für Trifluormethyl oder Trifluormethoxy stehen, oder
   - für Hydroxy, Nitro, Cyano, Fluor oder Chlor stehen, oder
   - für Amino, Methylamino, Dimethylamino, Phenylamino oder Acetylamino stehen,
R⁹, R¹⁰ gleich oder verschieden sind und für Wasserstoff stehen, oder
   - für Phenyl stehen, das gegebenenfalls substituiert ist durch Chlor, Fluor, Alkyl mit bis zu 4 Kohlenstoffatomen, Methoxy, Methylthio, Trifluormethyl, Trifluormethoxy, Trifluormethylthio, Nitro, Cyano, Amino oder Dimethylamino, oder
   für Amino, Methylamino, Dimethylamino, Phenylamino oder Acetylamino stehen, oder
   - für Hydroxy stehen, oder
   - für Alkoxy mit bis zu 4 Kohlenstoffatomen ste hen, oder
   - für Benzoyloxy oder Acetyloxy stehen, oder
   - für geradkettiges, verzweigtes oder cyclisches Alkyl oder Alkenyl mit jeweils bis zu 6 Kohlenstoffatomen stehen, das substituiert sein kann, durch Fluor, Chlor, Methoxy, Methylthio, Trifluormethoxy oder Cyano,
R¹¹, R¹² gleich oder verschieden sind und
   - für Wasserstoff stehen, oder
   - für Phenyl stehen, das gegebenenfalls substituiert ist durch Fluor, Chlor, Alkyl mit bis zu 4 Kohlenstoffatomen, Methoxy, Methylthio, Trifluormethyl, Trifluormethoxy, Trifluormethylthio, Nitro, Cyano, Amino oder Dimethylamino, oder
   - für Pyridyl, Thienyl, Furyl, Pyrimidyl oder Hydroxy stehen, oder
   - für Amino, Methylamino, Dimethylamino, Phenylamino oder Acetylamino stehen, oder
   - für Alkoxy mit bis zu 4 Kohlenstoffatomen stehen, oder
   - für Benzoyloxy oder Acetyloxy stehen, oder
   - für Benzoyl oder Acetyl stehen, oder
   - für Alkoxycarbonyl mit bis zu 4 Kohlenstoffatomen stehen, oder
   - für geradkettiges verzweigtes oder cyclisches Alkyl oder Alkenyl mit bis zu 6 Kohlenstoffatomen stehen, das gegebenenfalls substituiert ist durch Fluor, Chlor, Methoxy, Methylthio, Trifluormethoxy, Cyano, Phenyloxy oder Benzyloxy,
R¹³, R'⁴ gleich oder verschieden sind und
   - für Wasserstoff stehen, oder
   - für geradkettiges, verzweigtes oder cyclisches Alkyl oder Alkenyl mit bis zu 6 Kohlenstoffatomen stehen, das gegebenenfalls substituiert ist durch Fluor, Chlor, Methoxy, Methylthio, Trifluormethoxy oder Cyano, oder
   - für Phenyl stehen, das gegebenenfalls substituiert ist durch Fluor, Chlor, Alkyl mit bis zu 4 Kohlenstoffatomen, Methoxy, Methylthio, Trifluormethyl, Trifluormethoxy, Trifluormethylthio, Nitro, Cyano, Amino oder Dimethylamino, oder
   - für Alkoxycarbonyl mit bis zu 4 Kohlenstoffatomen stehen,
A - für O, S oder -NR¹⁸ steht,
B - für 0 oder -NR¹⁵ steht,
R¹⁵ für Wasserstoff steht, oder
   - für Phenyl steht, oder
   - für geradkettiges oder verzweigtes Alkyl mit bis zu 4 Kohlenstoffatomen steht,
R'⁶ für Wasserstoff steht, oder
   - für geradkettiges oder verzweigtes Alkyl oder Alkenyl mit jeweils bis zu 4 Kohlenstoffatomen steht, das gegebenenfalls substituiert ist durch Fluor, Chlor, Alkoxy mit bis zu 4 Kohlenstoffatomen, Hydroxy, Carboxy, Phenyl, Sul fo, Amino, Alkylamino, Dialkylamino met jeweils bis zu 3 Kohlenstoffatomen je Alkylgruppe, Phenylamino, Benzylamino oder durch Acetylamino, oder
   - für Fluor, Chlor oder Brom steht, oder
   - für Alkoxy oder Alkylthio mit jeweils bis zu 4 Kohlenstoffatomen steht, oder
   - für Phenyl steht, oder
   - für Amino, Alkylamino, Dialkylamino mit jeweils bis zu 3 Kohlenstoffatomen je Alkylgruppe, Phenylamino, Benzylamino oder Acetylamino steht, oder
   - für Alkylsulfonyl mit bis zu 4 Kohlenstoffatomen steht, oder
   - für Sulfo oder Sulfamoyl steht, oder
   - für Hydroxy, Mercapto, Phenyloxy oder Phenylthio steht, oder
   - für Guanidino, Hydrazino oder Hydroxylamino steht, oder
   - für Pyrrolyl, Pyrrolidinyl, Pyrazolyl, Imidazolyl, Pyridyl, Chinolyl, Isochinolyl, Furyl, Thienyl, Morpholinyl, Piperidinyl, Piperazinyl oder Pyrimidyl steht, das substituiert sein kann durch Fluor, Chlor, Methyl, Nitro, Cyano, Hydroxy, Trifluormethyl, Methoxy oder Amino, oder
   - für Pyridylthio oder Pyridyloxy steht,
R¹⁷ - die gleiche Bedeutung hat wie R¹⁶ und mit diesem gleich oder verschieden sein kann und
R'⁸⁻ die gleiche Bedeutung hat wie R¹⁵ und mit diesem gleich oder verschieden ist,
R^{2 -} für Wasserstoff steht, oder
   - für Methoxy oder Methylthio steht, oder
   - für Amino, Methylamino, Dimethylamino, Phenylamino, Benzylamino oder Acetylamino steht, oder
   - für Formamidino steht,
R^{3 -} für Wasserstoff steht, oder
   - für Methyl, Ethyl, tert.Butyl, 2,2,2-Trichlorethyl, Allyl, Acetoxymethyl, 4-Nitrobenzyl, 2-Nitrobenzyl, 4-Methoxybenzyl, Benzyl oder Trimethylsilylethyl steht, oder
   - für einen Rest der Formel 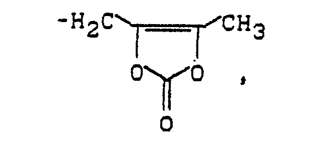 -CH(CH₃)-OCOOC₂H₅ oder -CH₂-OCO-C(CH₃)₃ steht, X - für eine Gruppe der Formel 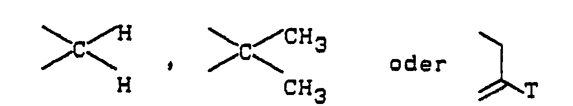 steht, oder die Gruppierung 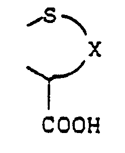 für die Gruppierung 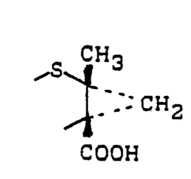 steht, worin
T - Wasserstoff, Chlor, Fluor, Methyl, Methoxy, Methylthio, Trifluormethyl, Methoxymethyl, Vinyl, Carbamoyloxymethyl oder Acetyloxymethyl bedeutet, oder
   - eine Gruppe der Formel 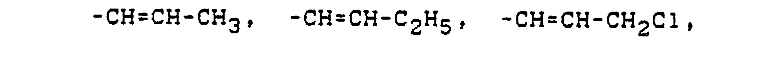 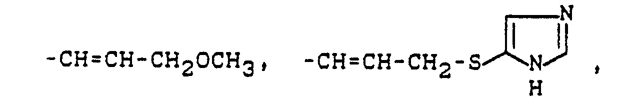 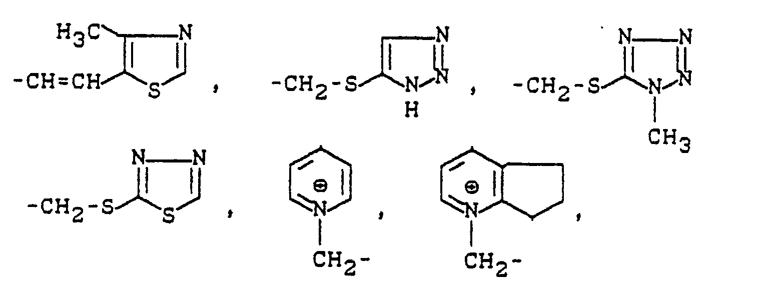 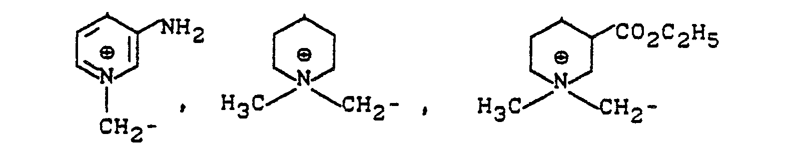 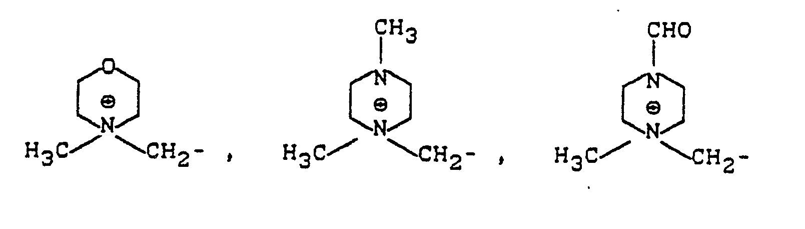 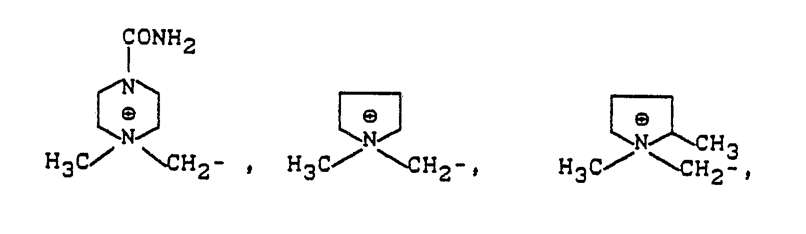 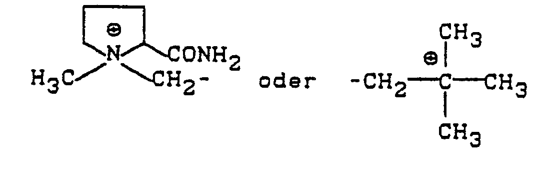 bedeutet, und
R⁴ und R⁵ zusammen mit Stickstoffatom einen Ring der Formel 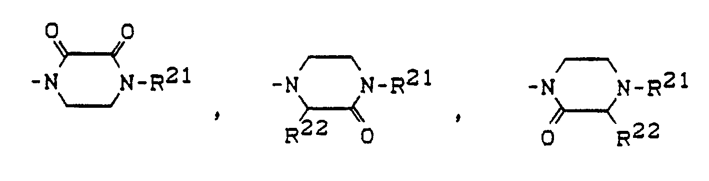 oder 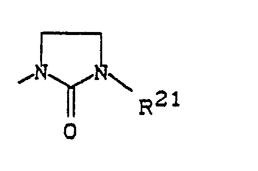 bilden, worin
R²' Wasserstoff oder Alkyl mit bis zu 4 Kohlenstoffatomen bedeutet, oder
   - Cyclopropyl, Cyclopentyl oder Cyclohexyl bedeutet, oder
   - Phenyl bedeutet, oder
   - einen heterocyclischen Rest der Reihe Furyl, Pyridyl, Pyrimidyl, Thiazolyl, Benzothiazolyl, Thiadiazolyl oder Benzothiadiazolyl bedeutet, wobei die genannten Heterocyclen substituiert sein können durch geradkettiges oder verzweigtes Alkyl, Alkenyl, Alkoxy, Alkylthio oder Alkylsulfonyl mit jeweils bis zu 4 Kohlenstoffatomen, durch Trifluormethyl, Fluor oder Chlor, oder
   - eine Gruppe der Formel 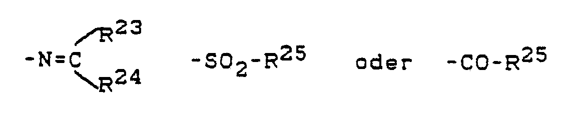 bedeutet,
R²² Wasserstoff oder Methyl bedeutet,
R²³, R²⁴ gleich oder verschieden sind und Wasserstoff bedeuten, oder
   - geradkettiges oder verzweigtes Alkyl oder Alkenyl mit jeweils bis zu 4 Kohlenstoffatomen bedeuten, oder
   - Cyclopropyl, Cyclopentyl oder Cyclohexyl bedeuten, oder
   - Phenyl oder Benzyl bedeuten, oder
   - einen heterocyclischen Ring der Reihe Furyl, Pyridyl oder Pyrimidyl bedeuten,
oder
R²³ und R24 gemeinsam mit dem Kohlenstoffatom einen Ring der Reihe Furyl, Pyridyl, Pyrimidyl, Oxazolyl, Thiazolyl, Oxazadiazolyl oder Thiadiazolyl bilden,
und
R?⁵⁻ Methoxy oder Ethoxy bedeutet, oder
   - geradkettiges oder verzweigtes Alkyl oder Alkenyl mit jeweils bis zu 4 Kohlenstoffatomen bedeutet, oder
   - Cyclopropyl, Cyclopentyl oder Cyclohexyl bedeutet, oder
   - Phenyl oder Benzyl bedeutet, oder
   - Amino, Alkylamino oder Dialkylamino mit jeweils bis zu 3 Kohlenstoffatomen je Alkylgruppe bedeutet, oder
   - einen heterocyclischen Ring der Reihe Furyl, Pyridyl, Pyrimidyl oder Chinolyl bedeutet, wobei die heterocyclischen Ring substituiert sein können durch Methyl, Ethyl, Propyl, Isopropyl, Methoxy, Fluor, Chlor oder Trifluormethyl

und deren Salze.

Darüberhinaus wurde ein Verfahren zur Herstellung der erfindungsgemäßen heteroanellierten Phenylglycin-ß-Lactam-Antibiotika der allgemeinen Formel (I) gefunden, das dadurch gekennzeichnet ist, daß man

Carbonsäuren der allgemeinen Formel (II) 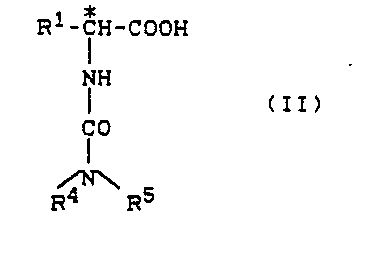 in welcher
R¹, R⁴ und R⁵ die oben angegebene Bedeutung haben,
nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester, beispielsweise mit Dicyclohexylcarbodiimid (DCC), gegebenenfalls in Anwesenheit von N-Hydroxybenztriazol,
mit den ß-Lactamaminen der allgemeinen Formel (III), 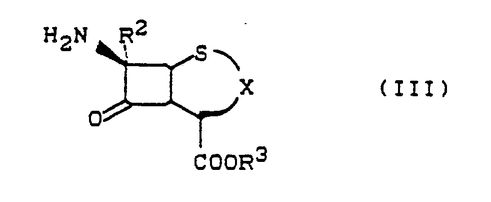 in welcher
R², R³ und X die oben angegebene Bedeutung haben,
umsetzt,
dann gegebenenfalls Schutzgruppen abspaltet und die gewünschten Salze oder aus den Salzen die freien Säuren herstellt.

Das erfindungsgemäße Verfahren kann durch das folgende Formelschemata erläutert werden:

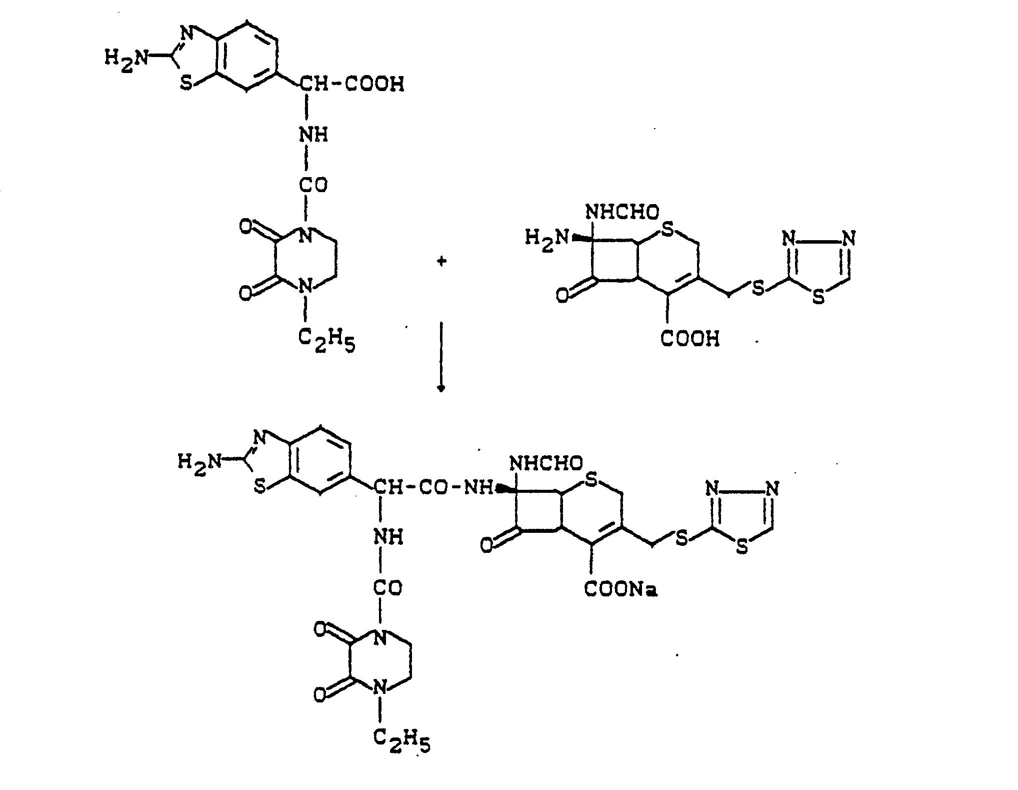

Bei der Durchführung des Verfahrens hat es sich als vorteilhaft erwiesen, die Carbonsäure zu aktivieren und dann mit den ß-Lactamaminen, die als Salze mit Amin und Lösung gebracht werden, zu kuppeln. Besonders vorteilhaft ist die Aktivierung mit Sulfonsäurederivaten der allgemeinen Formel (IV) oder mit Chlorameisensäureestern, bevorzugt Chlorameisensäureethylester, zu Anhydriden der allgemeinen Formel (Va, b), wie im folgenden Reaktionsschema verdeutlicht wird.

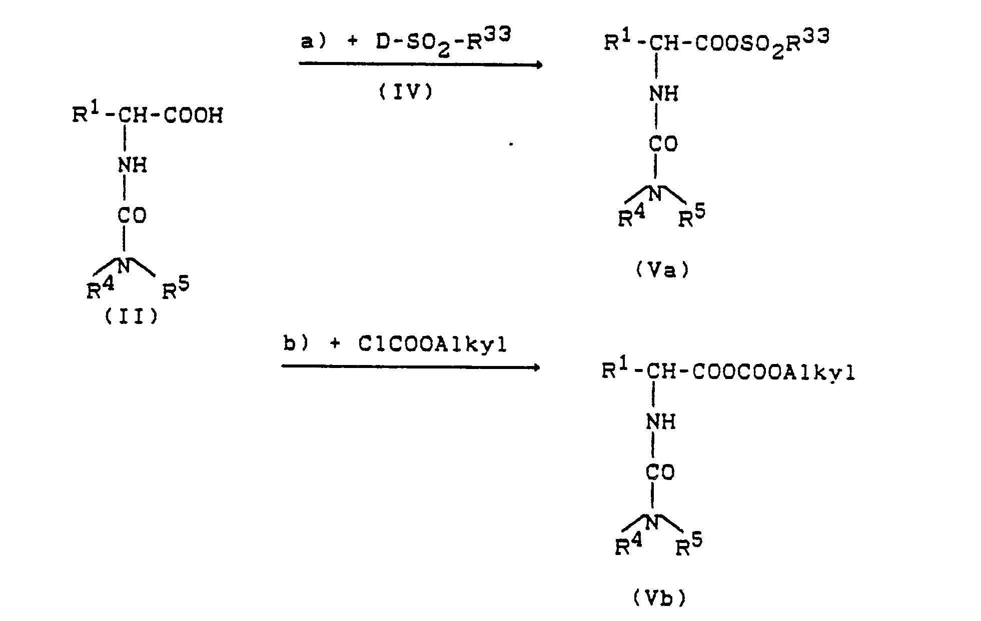

Hierbei steht in der Formel (IV) bzw. (Va) D - für den Rest
R³³-SO₂-O- oder Halogen
und
R³³⁻ für Alkyl mit bis zu 10 Kohlenstoffatomen, das gegebenenfalls substituiert ist durch Fluor, Chlor, Cyano, Phenyl, Alkoxycarbonyl oder Alkoxy mit jeweils bis zu 4 Kohlenstoffatomen, oder
- für Phenyl, das gegebenenfalls substituiert ist durch Fluor, Chlor, Brom, Cyano, Alkyl, Alkoxy, Alkylthio, Alkoxycarbonyl oder Alkylcarbonyl mit jeweils bis zu 4 Kohlenstoffatomen, Nitro, Trifluormethyl oder Phenyl Wenn R³³ substituiert ist, sind bevorzugt ein bis drei Substituenten, besonders bevorzugt die oben genannten vorhanden.

Ganz besonders bevorzugt stellt R³³ einen Methyl- oder p-Tolylrest dar.

Die gemischten Anhydride der allgemeinen Formel (Va, b) werden hergestellt, indem man die Carbonsäuren der allgemeinen Formel (11) und 1- bis 1,4-Äquivalente eines Amins in einem Lösemittel löst und mit 1- bis 1,2-Äquivalenten eines Sulfonsäurederivates der Formel (IV) oder eines Chlorameisensäureesters reagieren läßt.

Als Lösemittel eignen sich alle Solventien, die sich unter den Reaktionsbedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlorkohlenstoff, oder Amide wie Dimethylformamid oder Hexamethylphosphorsäurtriamid, oder Acetonitril, oder Aceton. Ebenso ist es möglich, Gemische der genannten Lösemittel einzusetzen.

Als Amine eignen sich tertiäre Amine wie beispielsweise Triethylamin, Ethyldiisopropylamin oder Tributylamin, aber auch sterisch gehinderte sekundäre Amine wie beispielsweise Diisopropylamin. Ebenso können Gemische der genannten Amine eingesetzt werden.

Die Umsetzung kann bei Temperaturen zwischen -80 C und Raumtemperatur durchgeführt werden. Vorteilhaft wird die Aktivierung mit Methansulfonsäurechlorid in Dimethylformamid bei -40 C bis -60 C innerhalb von 0,2 bis 24 Stunden, vorzugsweise 0,25 bis 5 Stunden durchgeführt.

Zum Lösen der β-Lactamamine der allgemeinen Formel (III) können die bei der Herstellung der Verbindung der Formel (V) genannten Lösemittel oder Wasser, sowie also Base die dort genannten Amine verwendet werden.

Besonders vorteilhaft ist auch eine Aktivierung der Carbonsäure der allgemeinen Formel (II) durch Überführen in einen aktivierten Ester mit beispielsweise Dicyclohexylcarbodiimid, gegebenfalls in Anwesenheit von N-Hydroxysuccinimid oder 1-Hydroxybenztriazol.

Als Lösemittel eignen sich hierbei alle Solventien, die sich auch für die Herstellung von Anhydriden der allgemeinen Formel (V) eignen und dort bereits aufgeführt sind. Die Umsetzungen können bei Temperaturen zwischen -30 C und +100°C durchgeführt werden. Vorteilhaft wird mit 1-Hydroxybenztriazol und Dicyclohexylcarbodiimid in Dimethylformamid bei Raumtemperatur 2 bis 6 Stunden aktiviert. Dann vom ausgefallenen Dicyclohexylharnstoff abgesaugt und mit dem ß-Lactamamin der allgemeinen Formel (III) in Form einer Lösung ihres Aminsalzes, innerhalb von 2 bis 24 Stunden umgesetzt. Zum Lösen der β-Lactamamine der allgemeinen Formel (III) können die bei der Herstellung der Verbindung der Formel (V) genannten Lösemittel sowie als Base die dort genannten Amine verwendet werden.

Die als Ausgangsverbindungen eingesetzten Carbonsäuren der allgemeinen Formel (II) sind bekannt oder können nach bekannten Methoden hergestellt werden [DE-OS 35 09 618; DE-OS 35 08 258].

Die als Ausgangsstoffe eingesetzten β-Lactamamine der allgemeinen Formel (III) sind bekannt oder können nach bekannten Methoden hergestellt werden [US-Patent 3 925 372; DE-OS 26 06 196; US-Patent 3 994 884; Britisches Patent 1 546 622; P.H. Miltner et al., J. Chem. Soc. Chem. Commun. 1984, 1335; DE-OS 35 09 618; DE-OS 35 08 258].

Darüberhinaus können die Verbindungen der allgemeinen Formel (I) nach einer weiteren Verfahrensvariante hergestellt werden, indem man Verbindungen der allgemeinen Formel (VI) 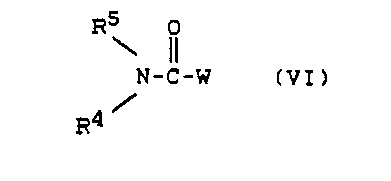 in welcher
R⁴ und R⁵ die oben angegebene Bedeutung haben,
und
W - für Halogen, Azid oder eine nucleofuge Abgangsgruppe steht,
in Gegenwart eines Lösemittels und gegebenenfalls eines Säurebindemittels bei Temperaturen von etwa -20 C bis etwa +50° C mit Verbindungen der allgemeinen Formel (VII) 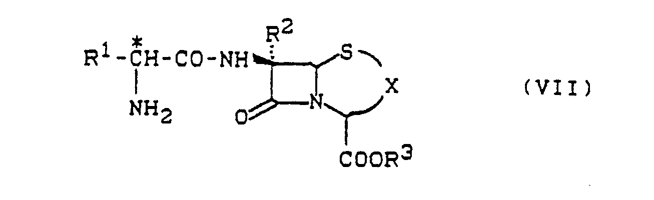 in welcher
R¹, R², R³ und X die oben angegebene Bedeutung haben,
umsetzt,
die erhaltenen ß-Lactam-Antibiotika gegebenenfalls nach Abspaltung von Schutzgruppen, gegebenenfalls in ihre nicht-toxische pharmazeutisch verträglichen Salze überführt, oder aus den gegebenenfalls erhaltenen Salze die freien Säuren herstellt.

Die erfindungsgemäße Verfahrensvariante kann durch das folgende Schema erläutert werden:

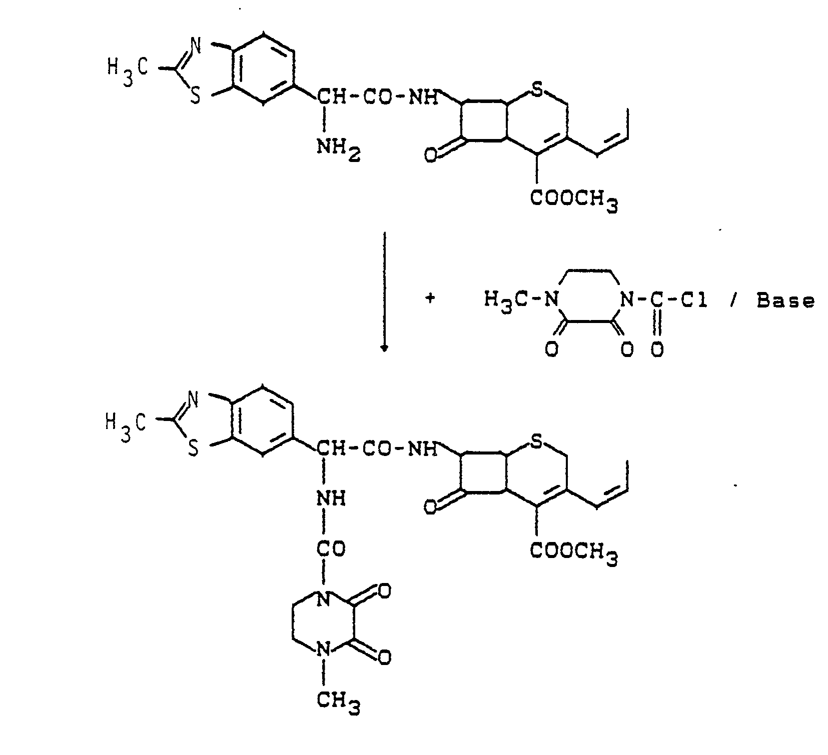

Steht W für Halogen, dann bevorzugt für Chlor oder Brom, besonders bevorzugt für Chlor.

Unter nucleofugen Abgangsgruppen in der Definition von W sind alle üblicherweise in der organischen Chemie verwendeten nucleofugen Gruppen und vor allem solche zu verstehen, welche in der Angewandten Chemie 81, 543 (1969) beschrieben sind.

Die als Ausgangsstoffe eingesetzten Verbindungen der allgemeinen Formel (VII) sind bekannt oder können nach bekannten Methoden hergestellt werden [DE-OS 35 09 618; DE-OS 35 08 258].

Die als Ausgangsstoffe eingesetzten Verbindungen der allgemeinen Formel (VI) sind bekannt oder können nach bekannten Methoden hergestellt werden [DE-OS 25 12 998, US-Patent 4 087 424].

Als neue erfindungsgemäße Wirkstoffe aus der Reihe der Penicilline seien im einzelnen, neben den experimentellen Beispielen folgende Verbindungen genannt:

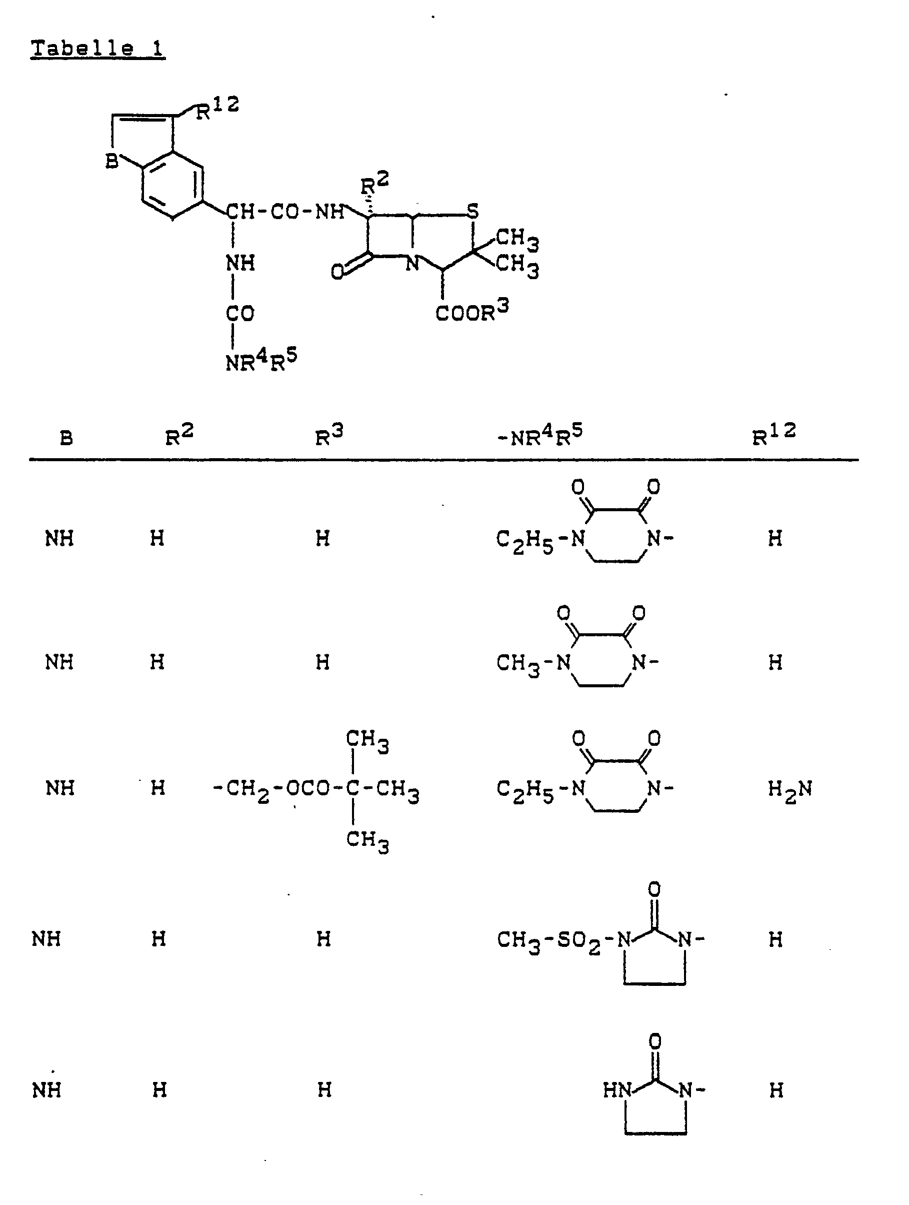

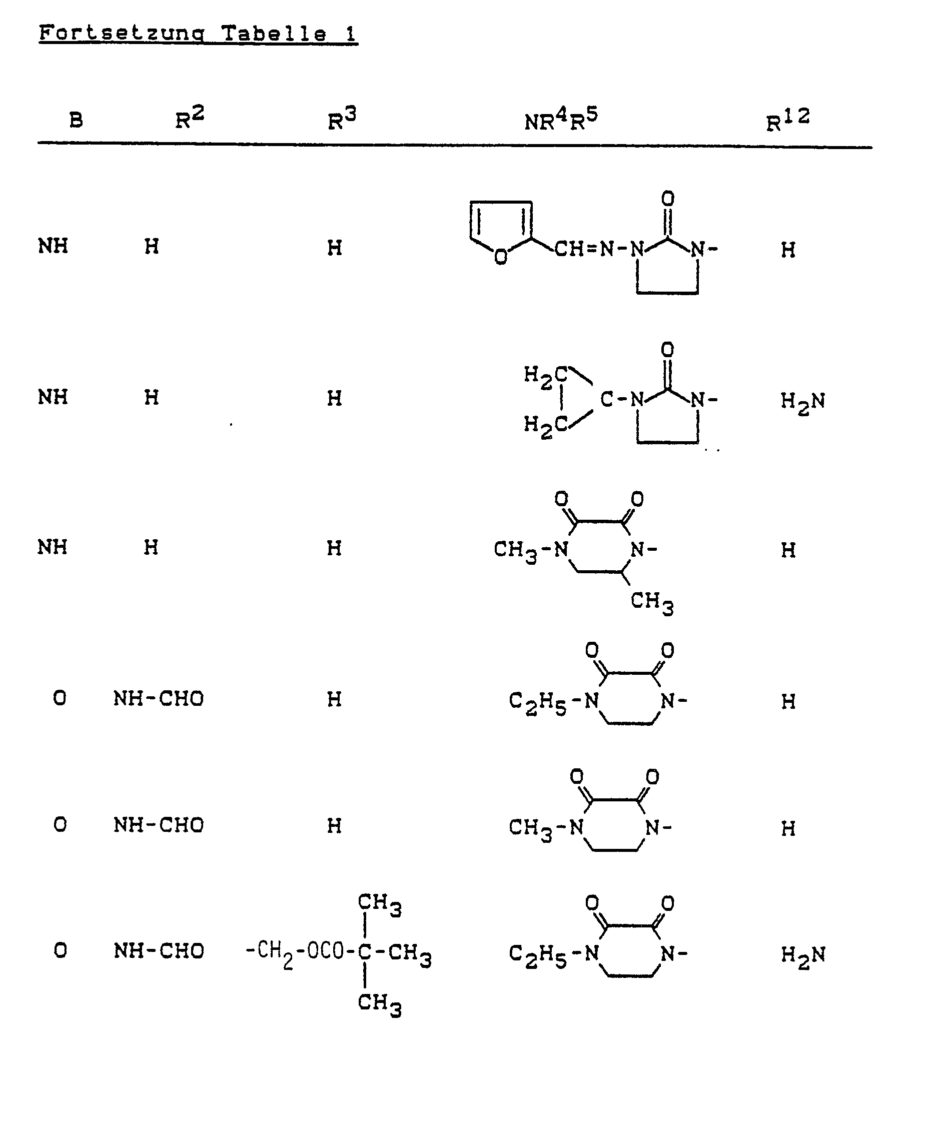

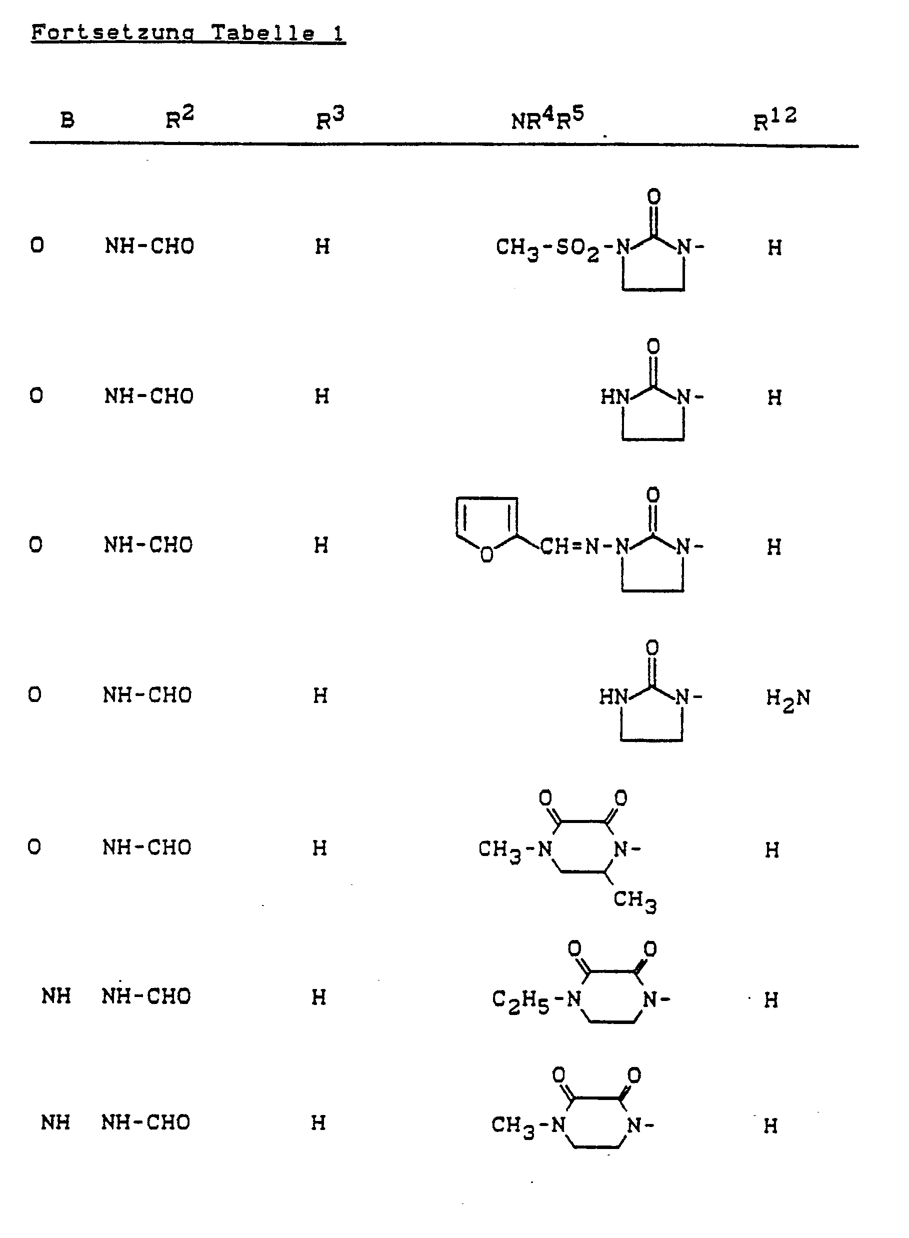

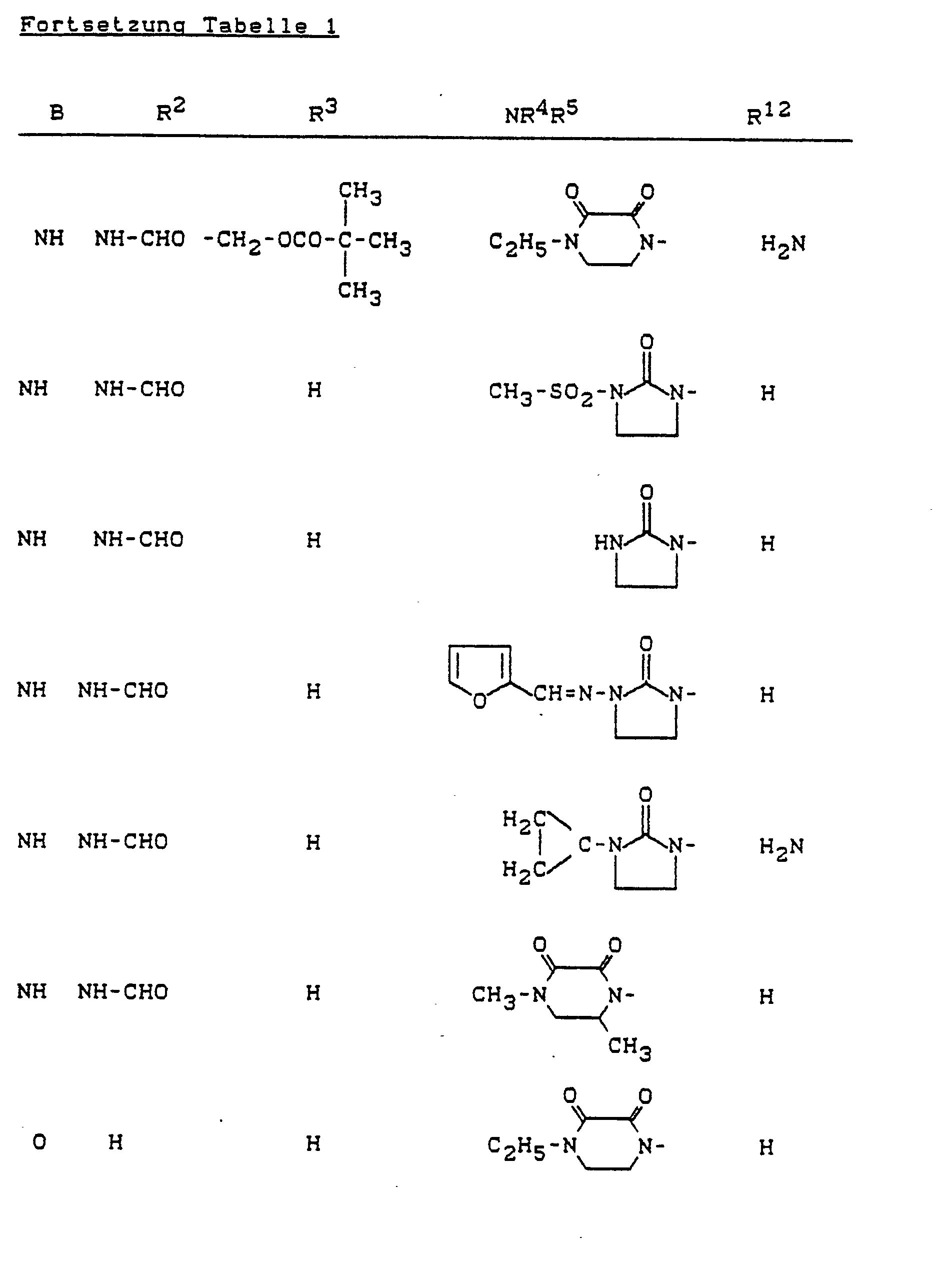

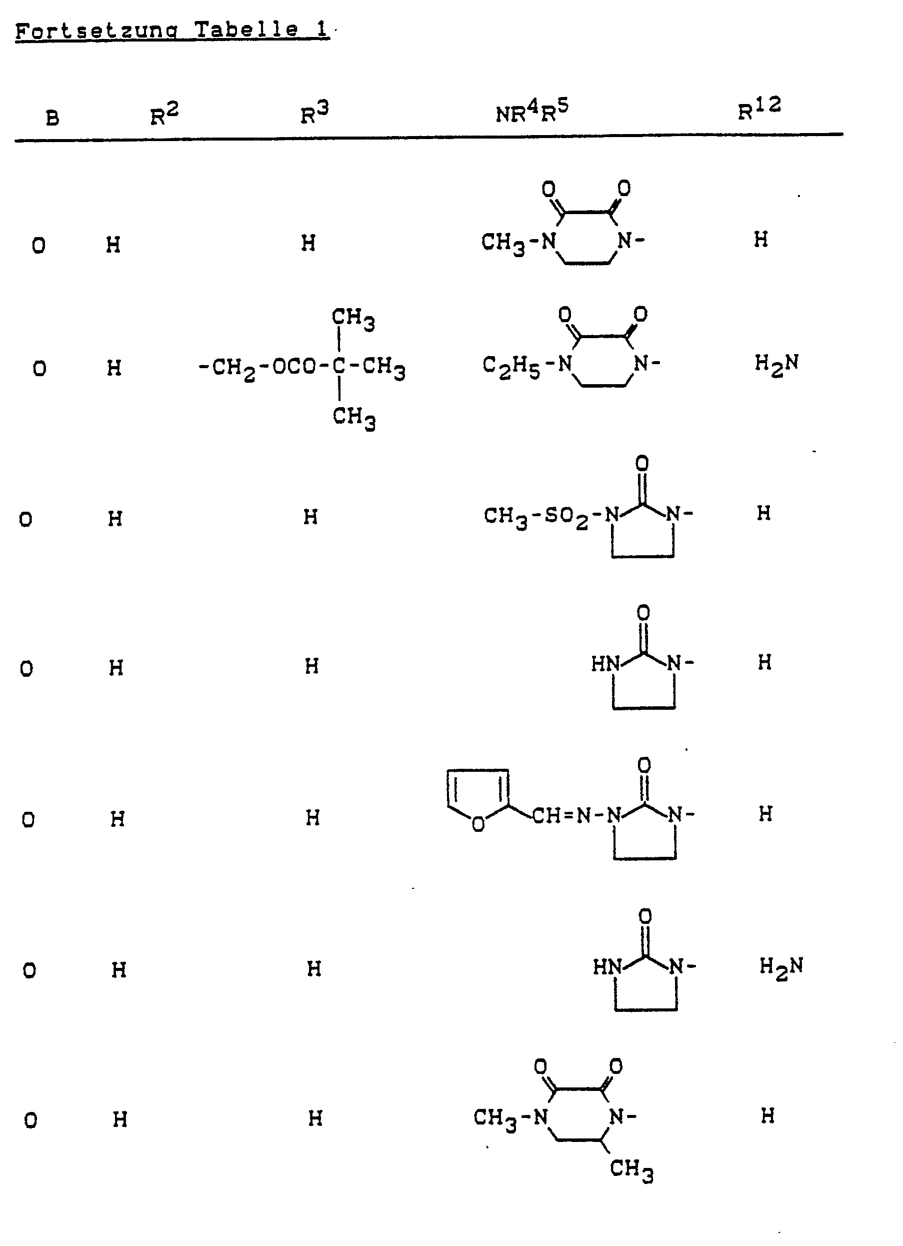

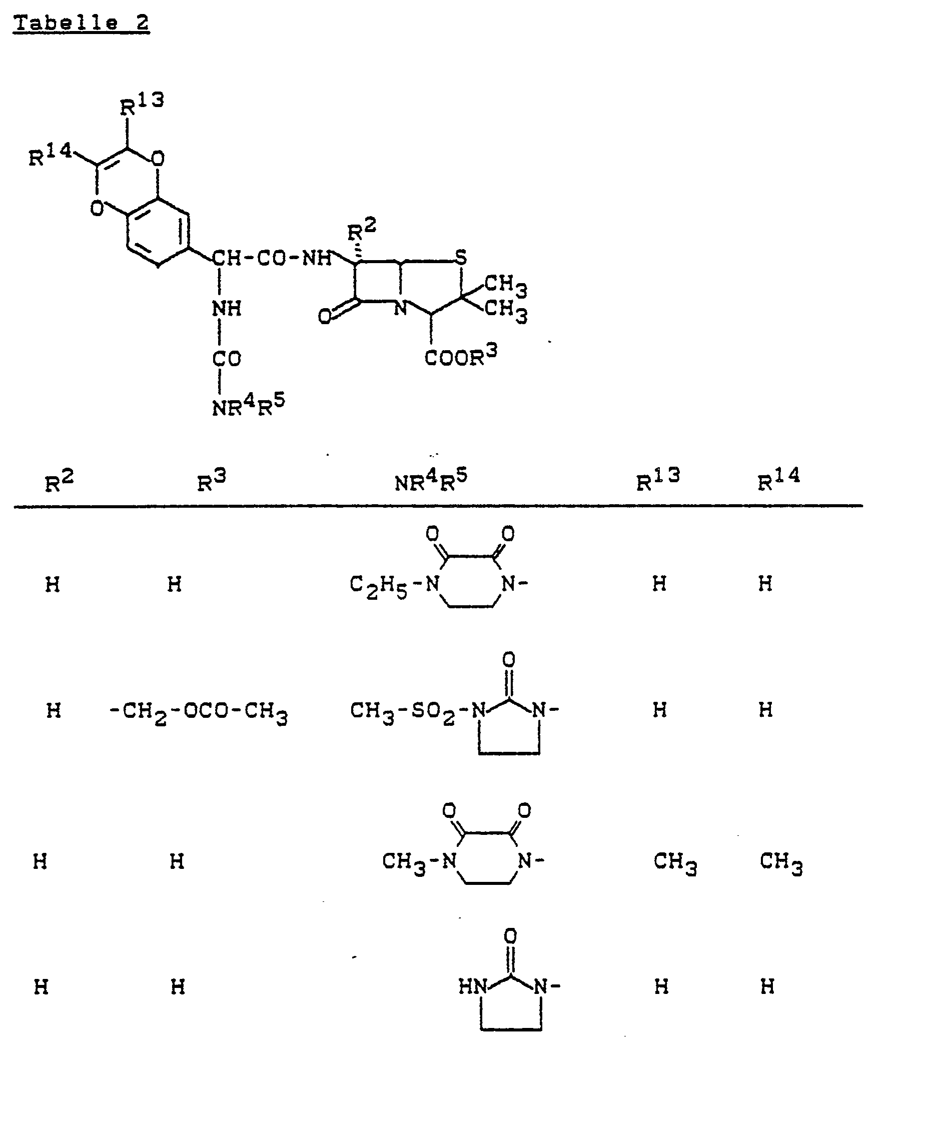

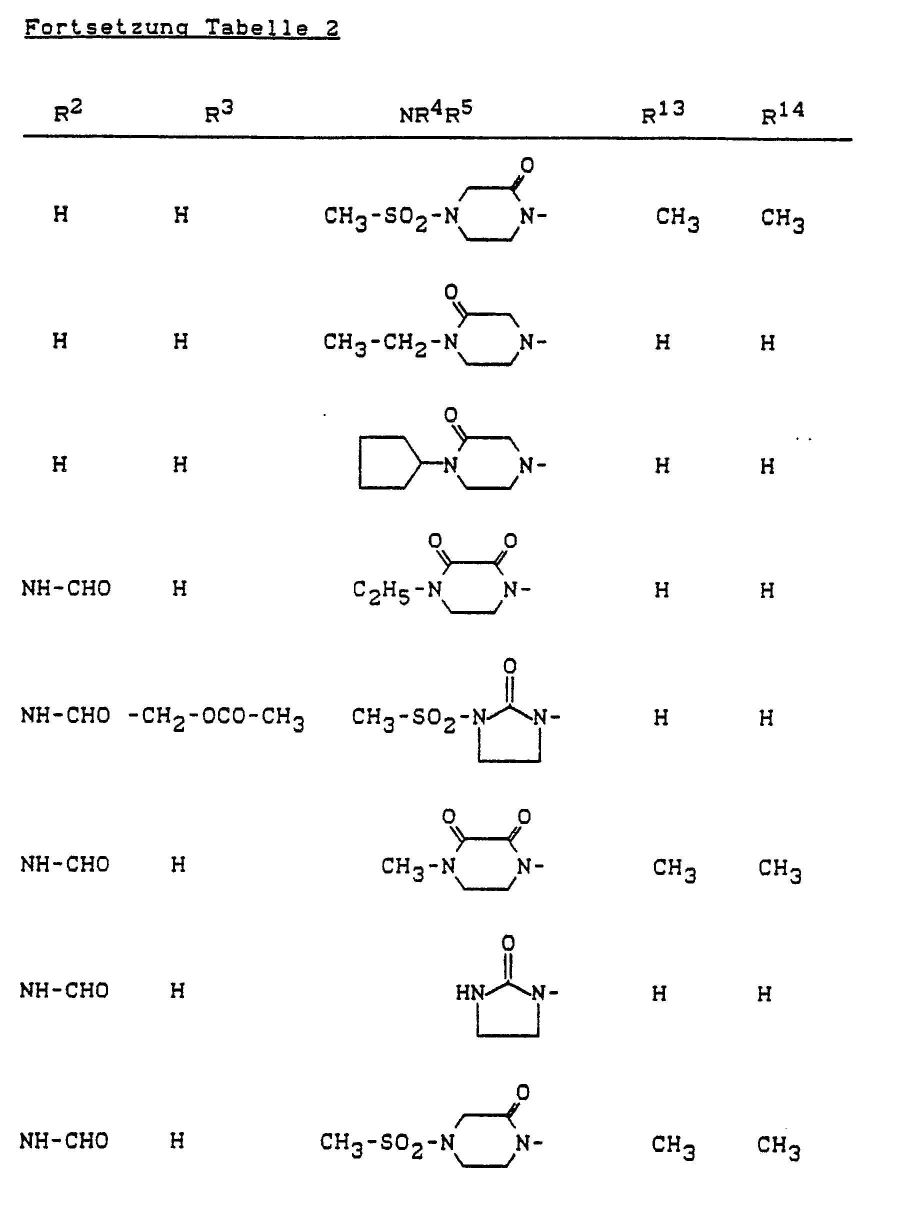

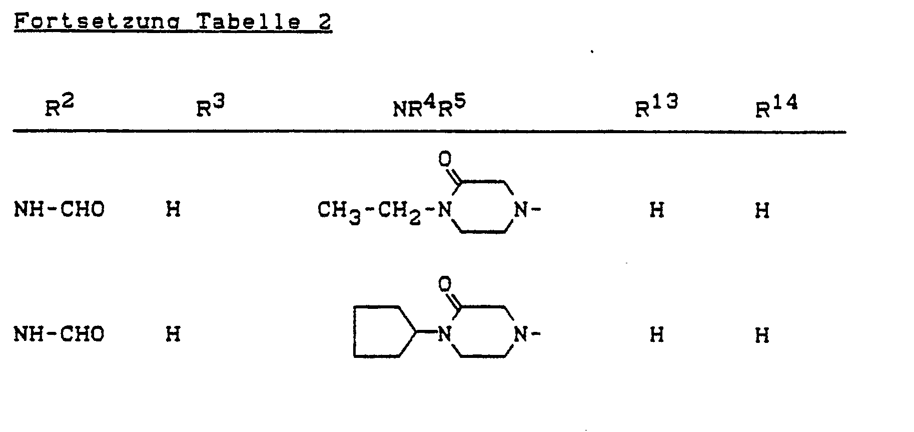

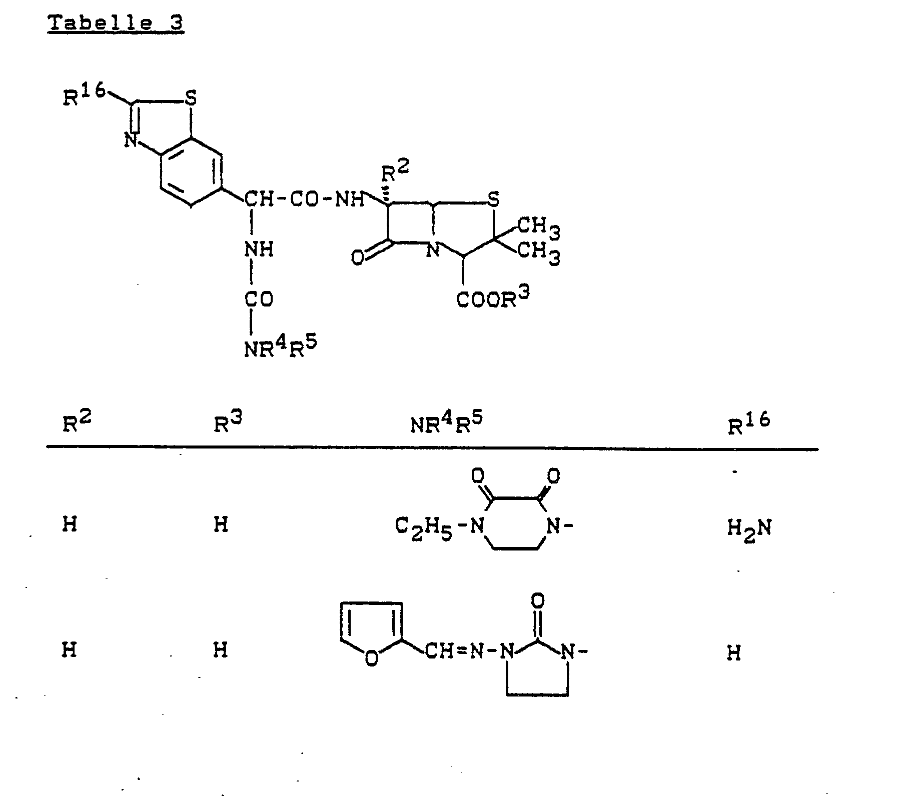

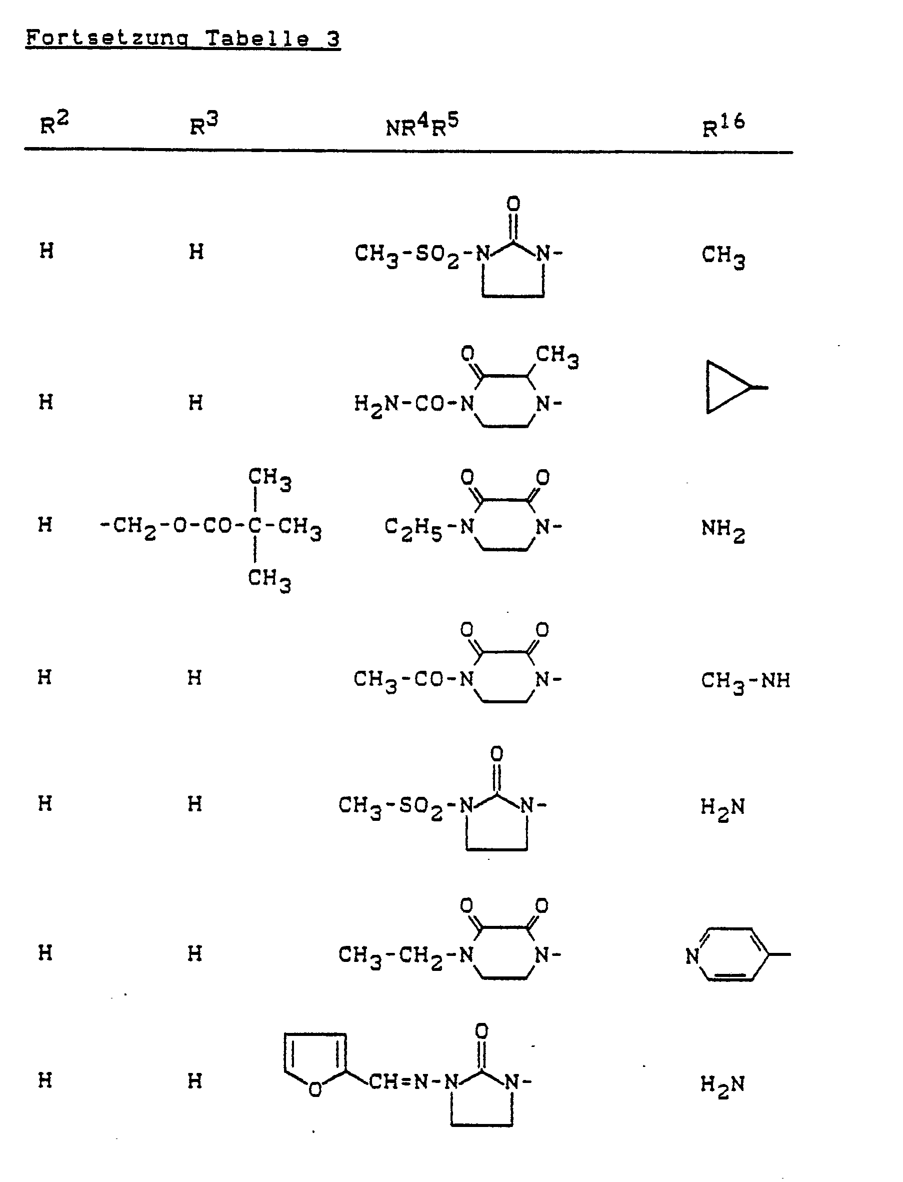

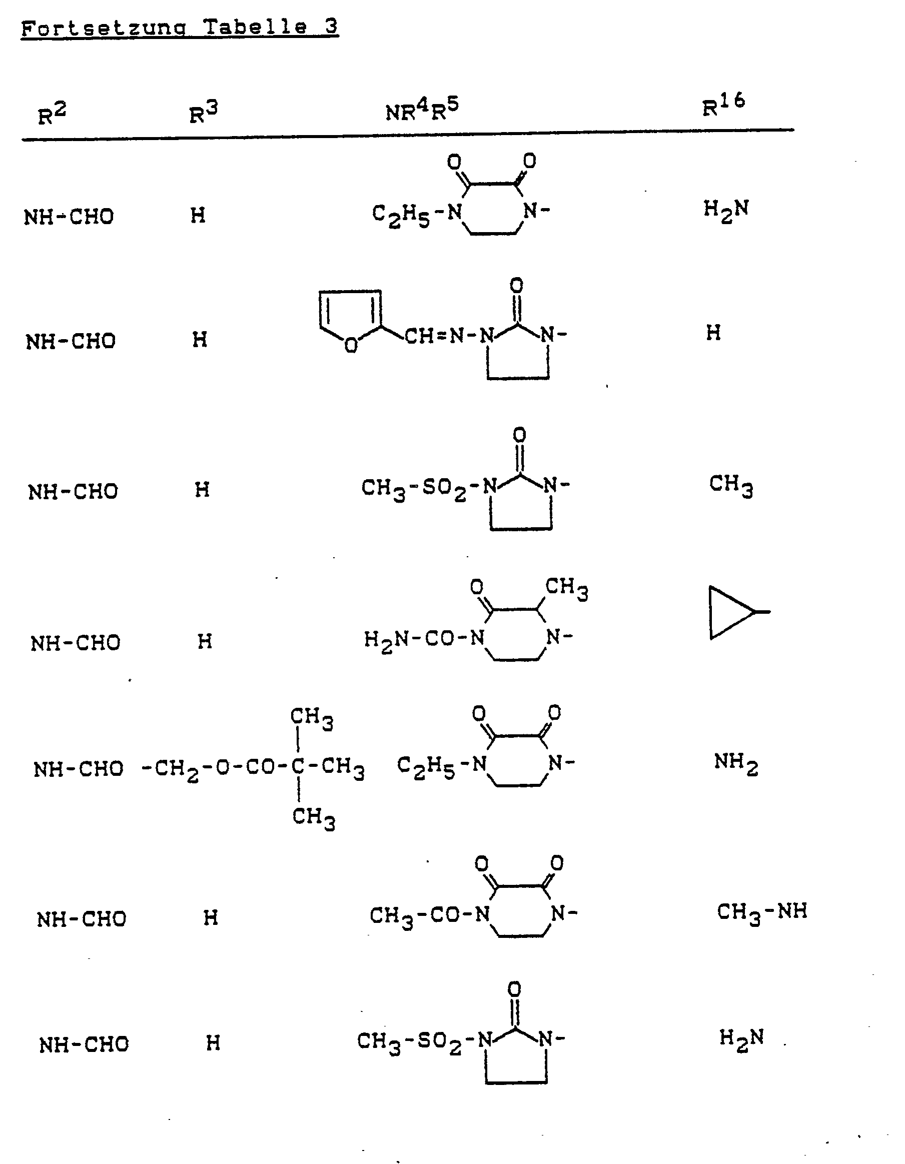

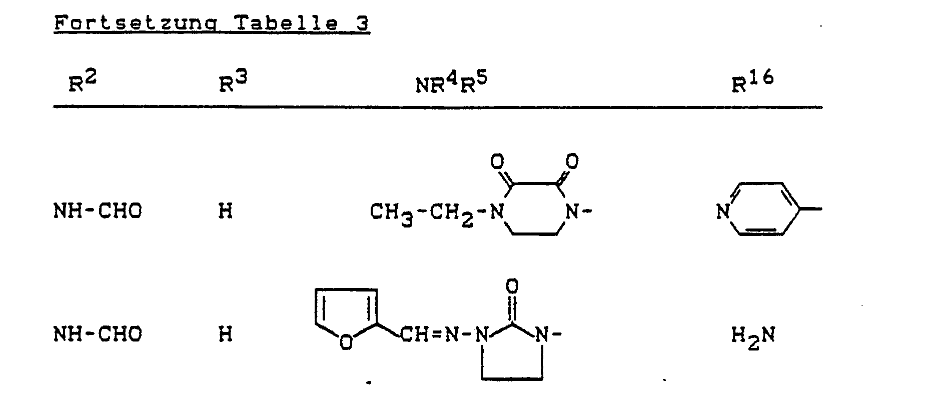

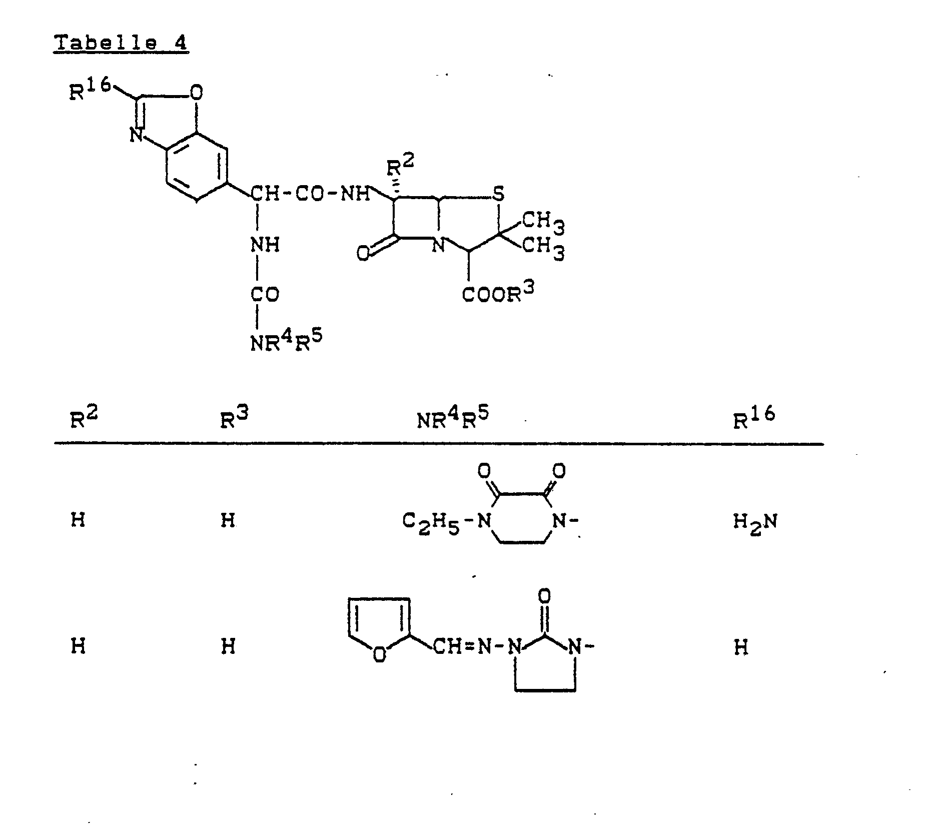

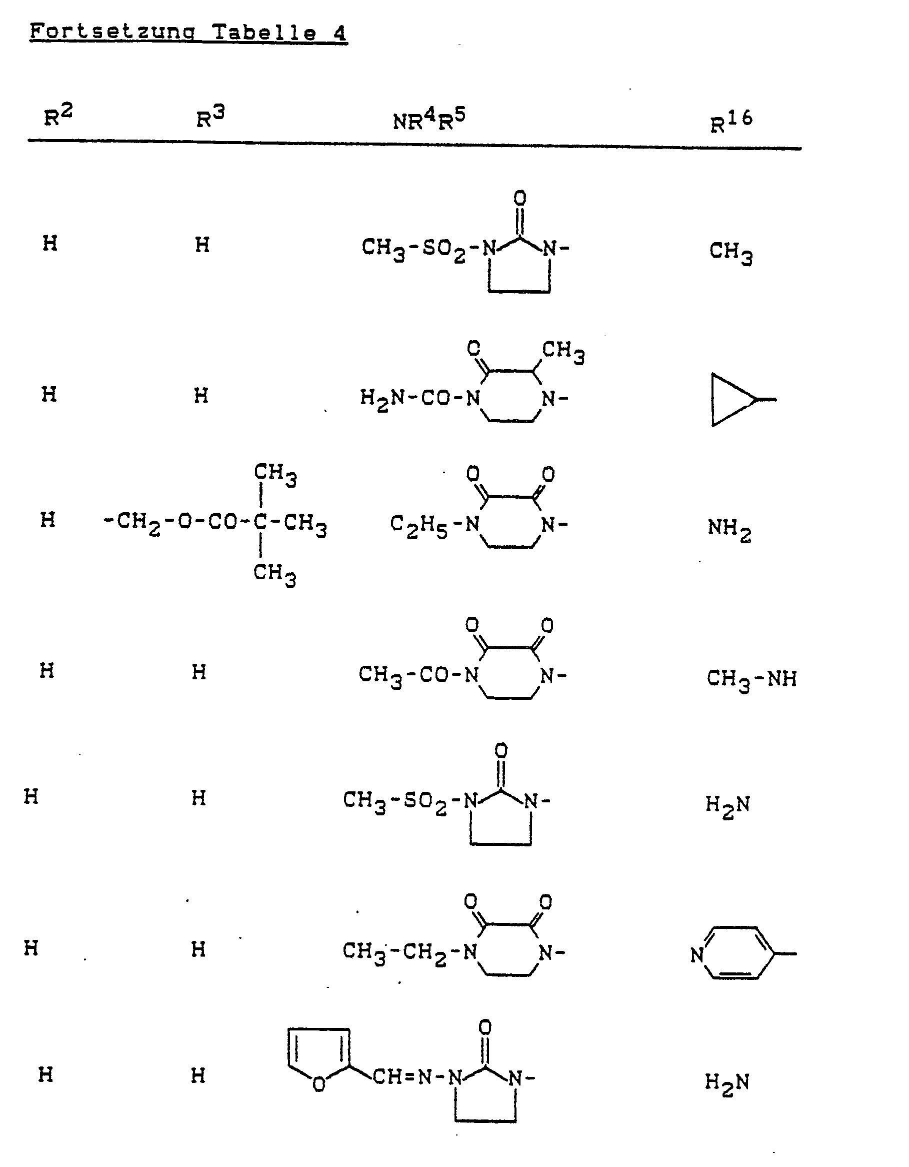

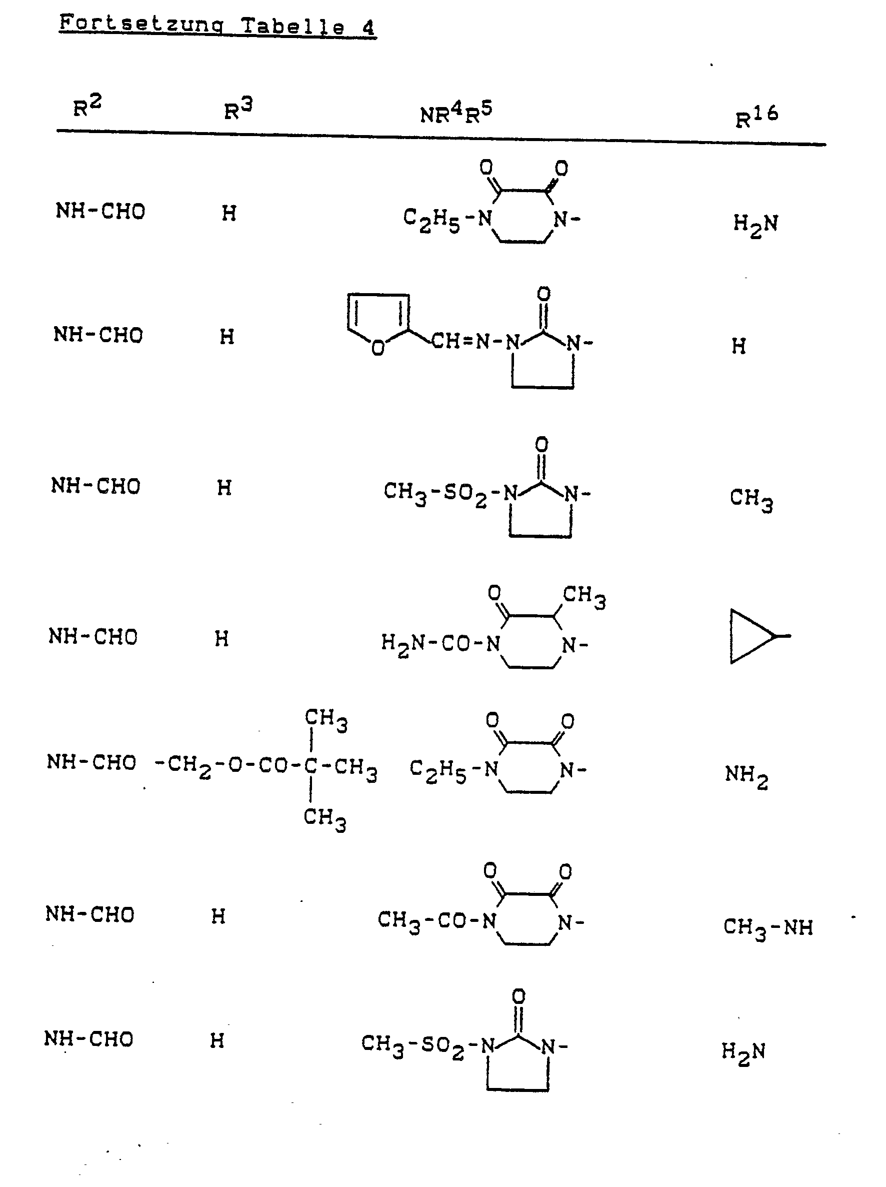

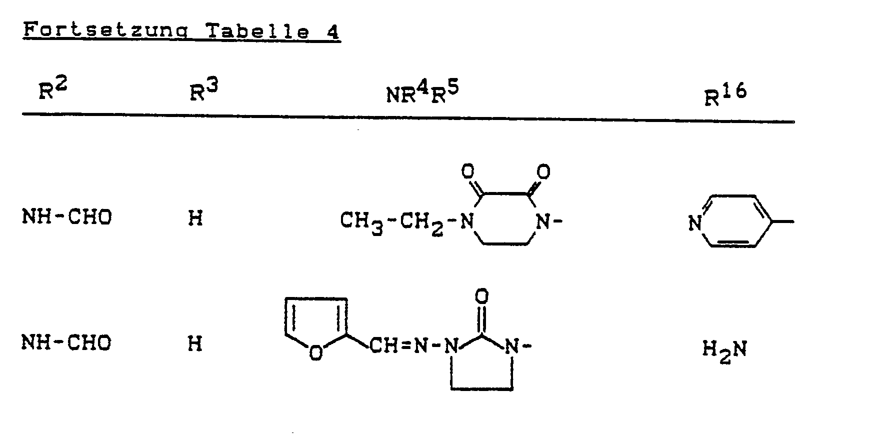

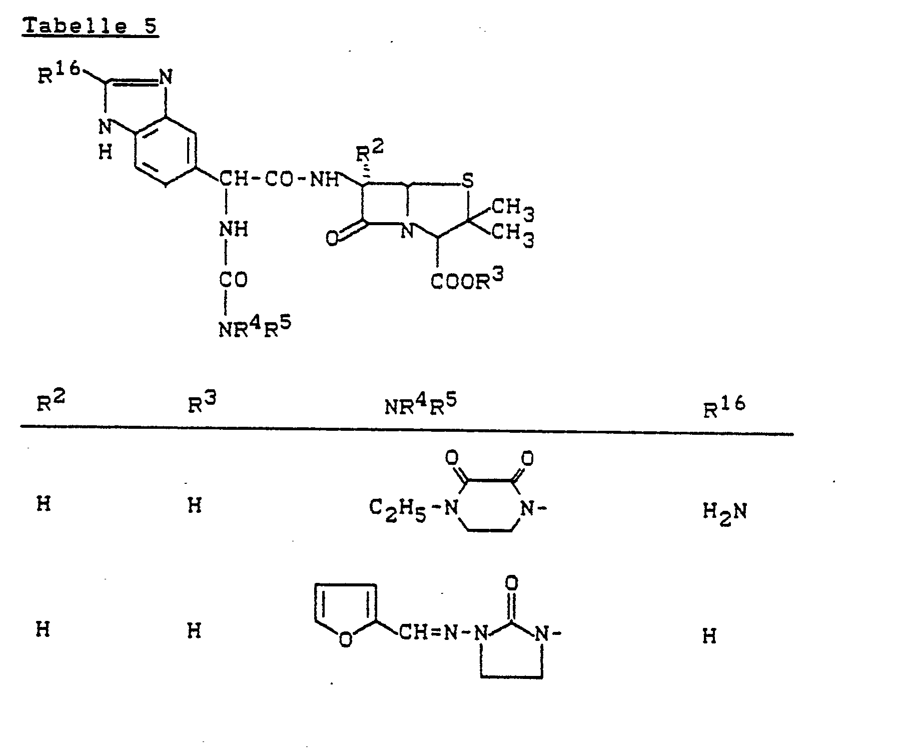

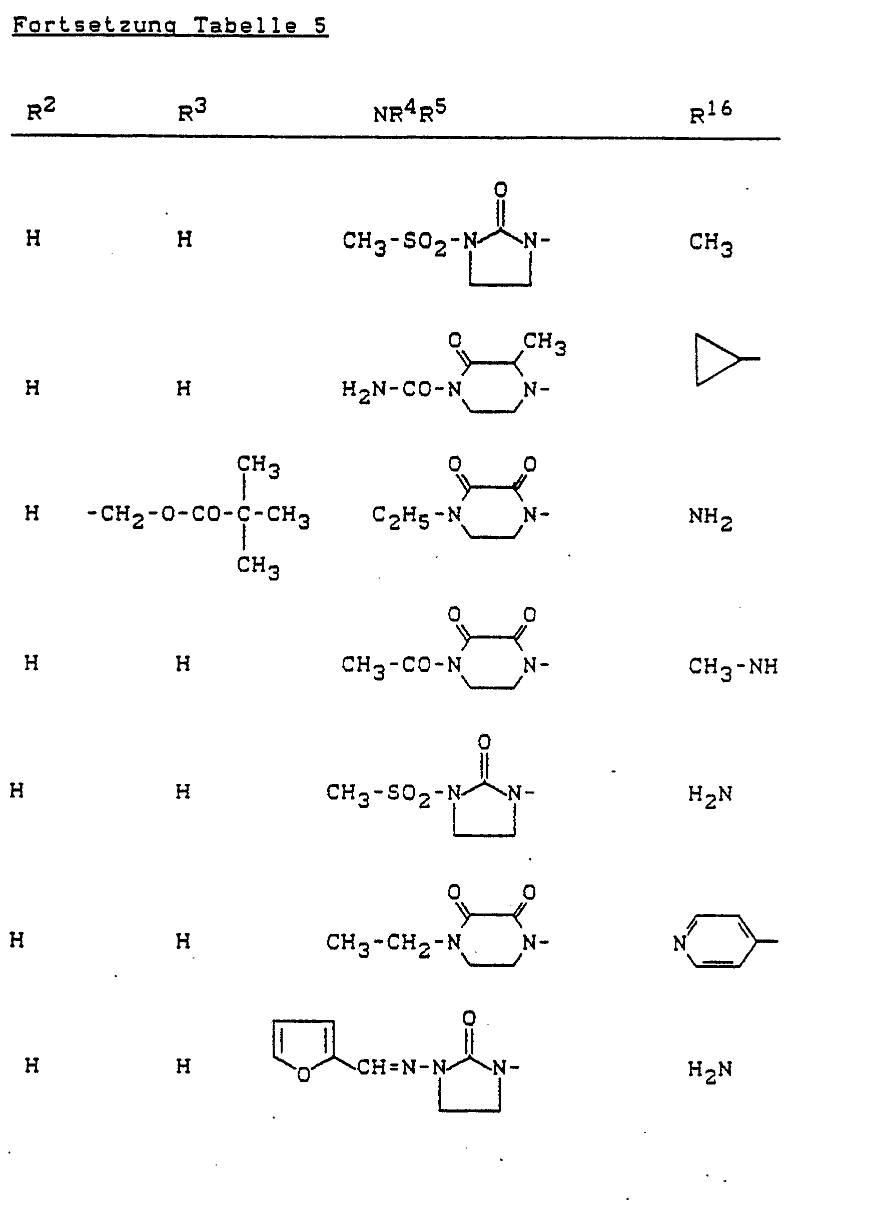

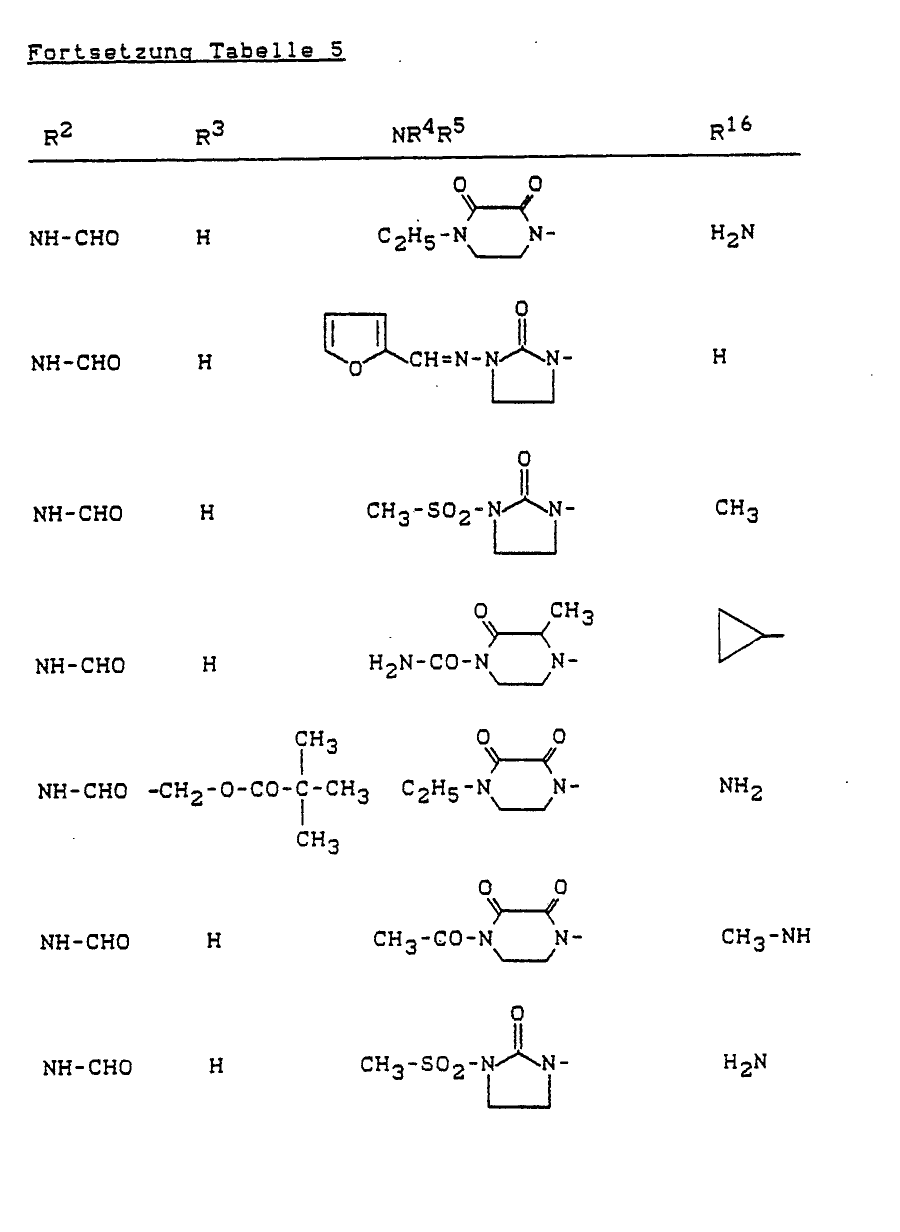

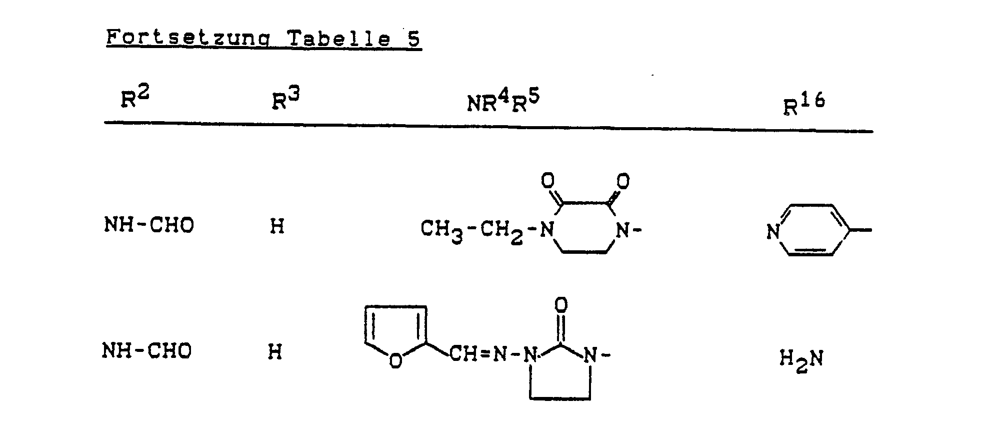

Als neue Wirkstoffe aus der Reihe der Cephalosporine seien im einzelnen neben den experimentellen Beispielen folgende Verbindungen genannt:

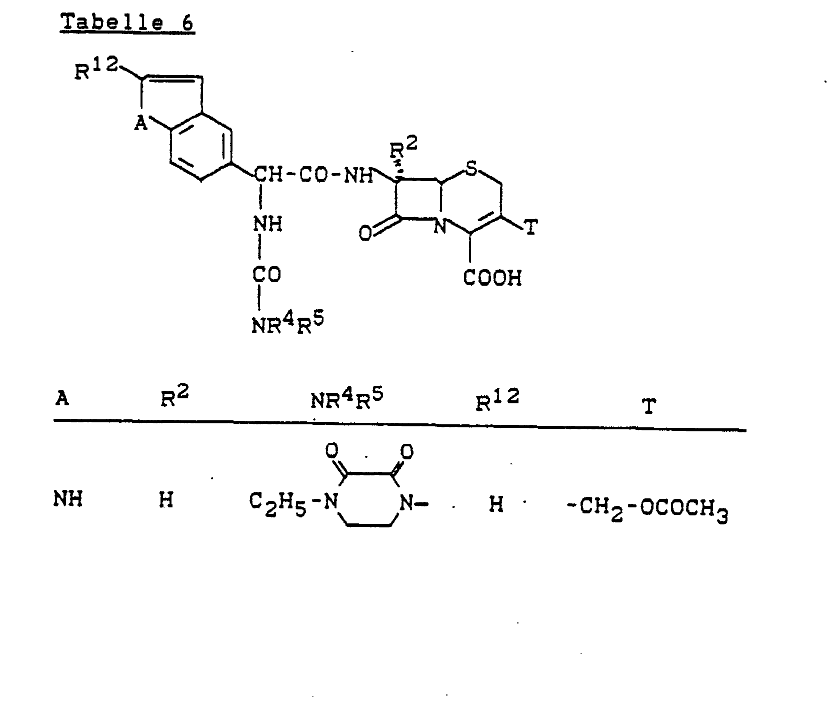

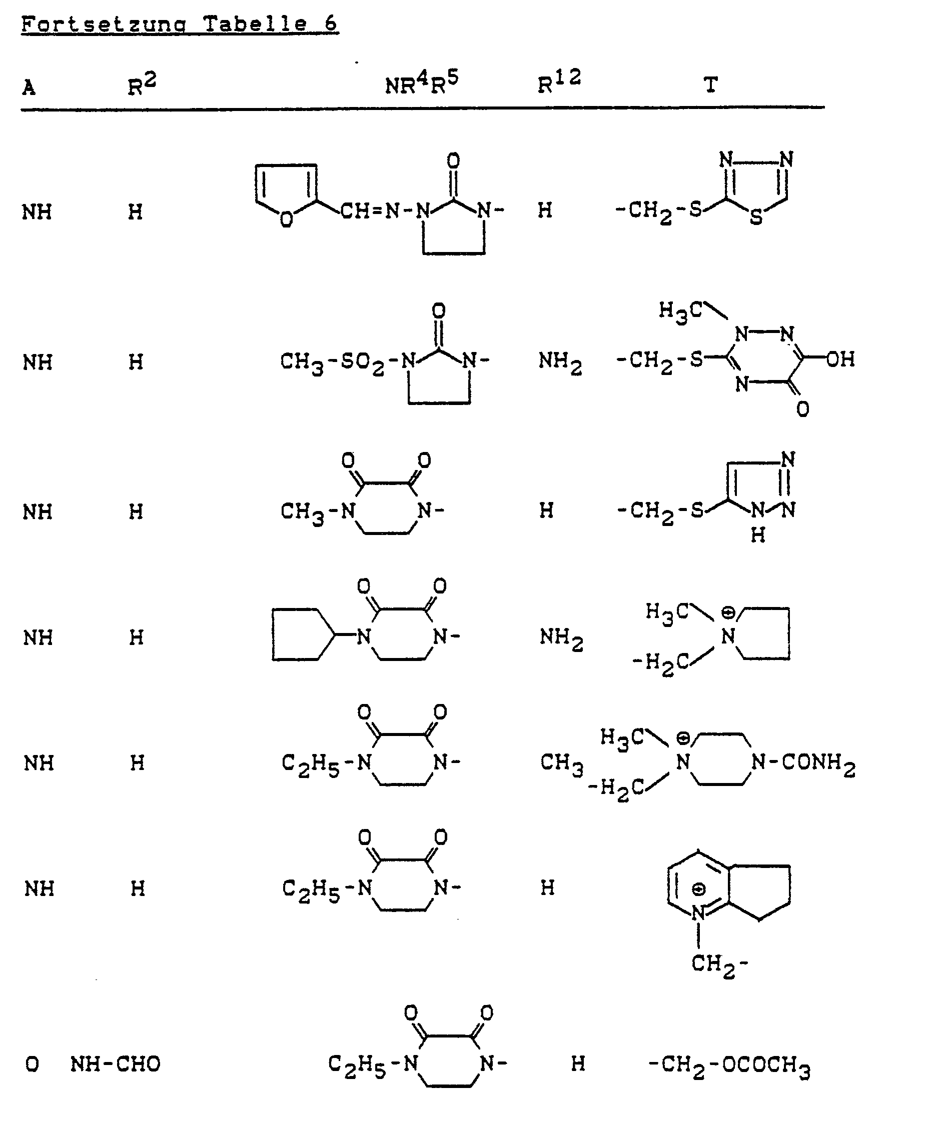

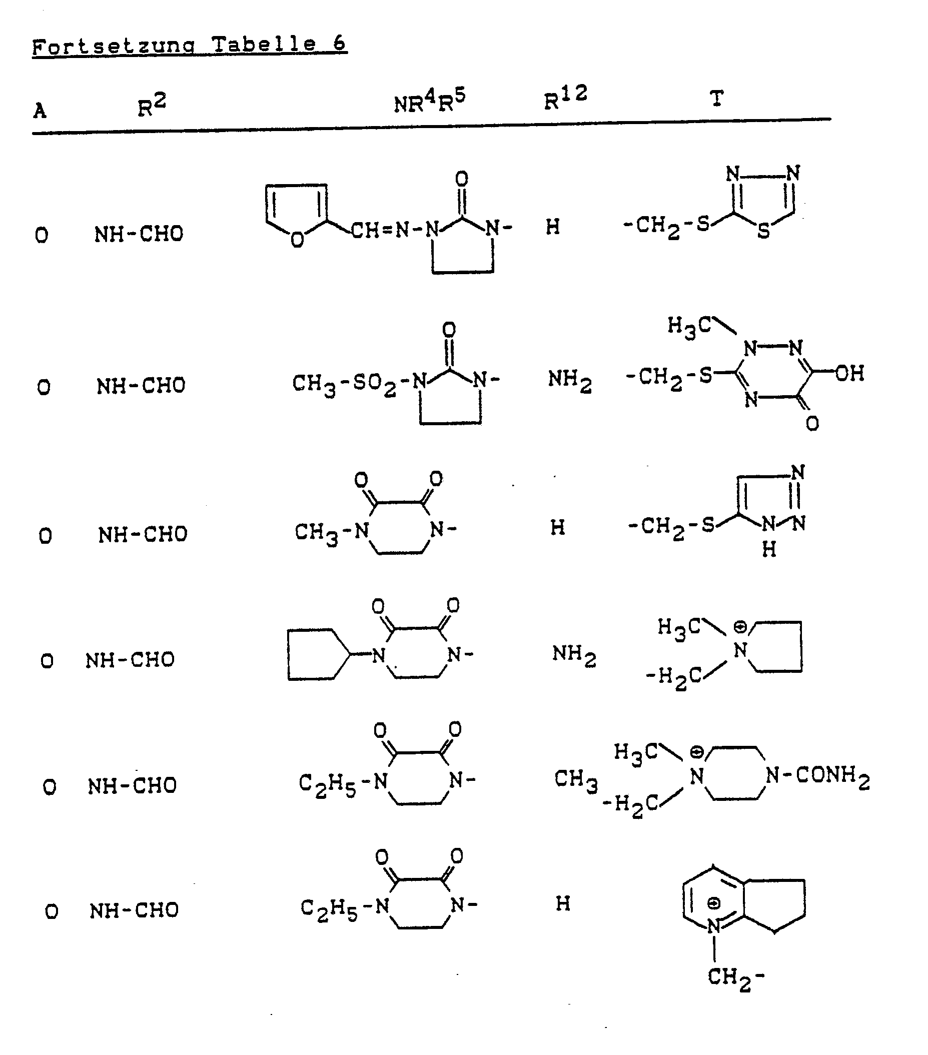

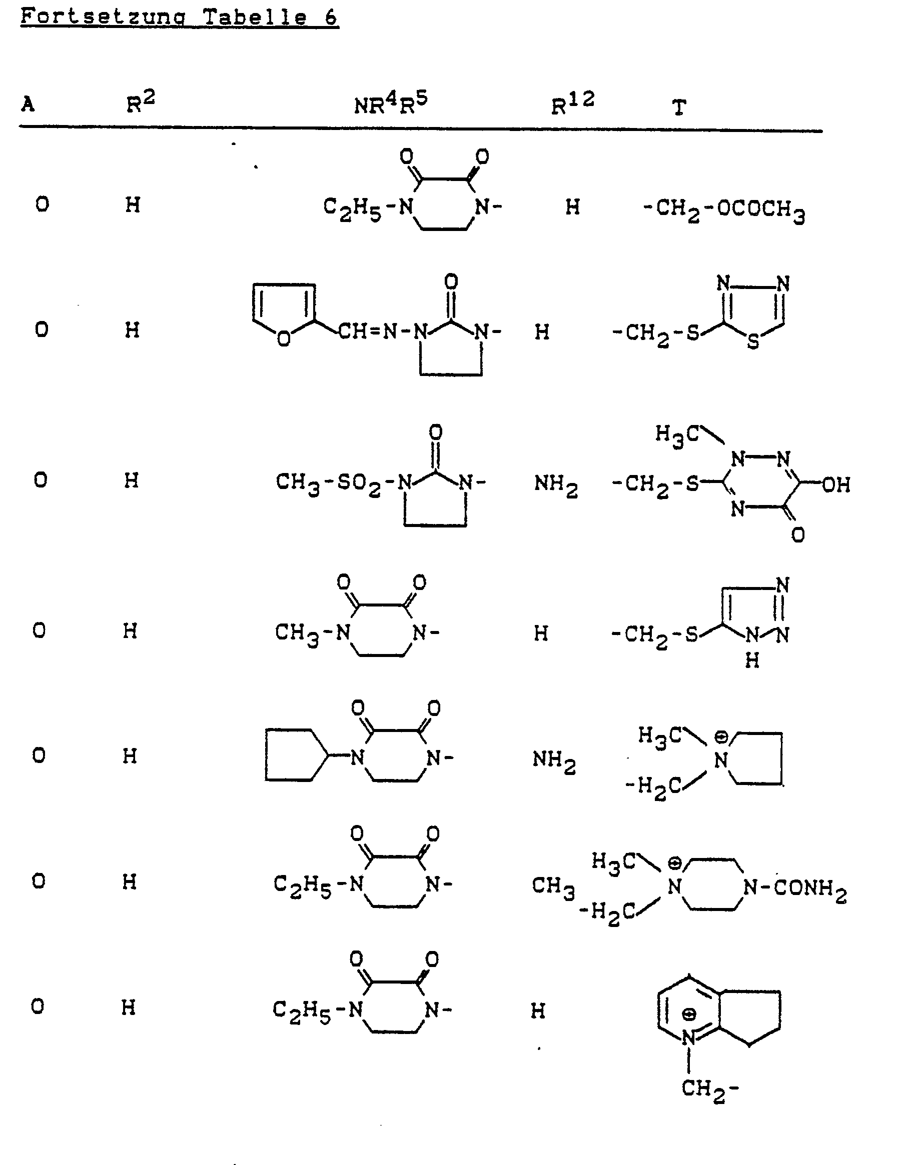

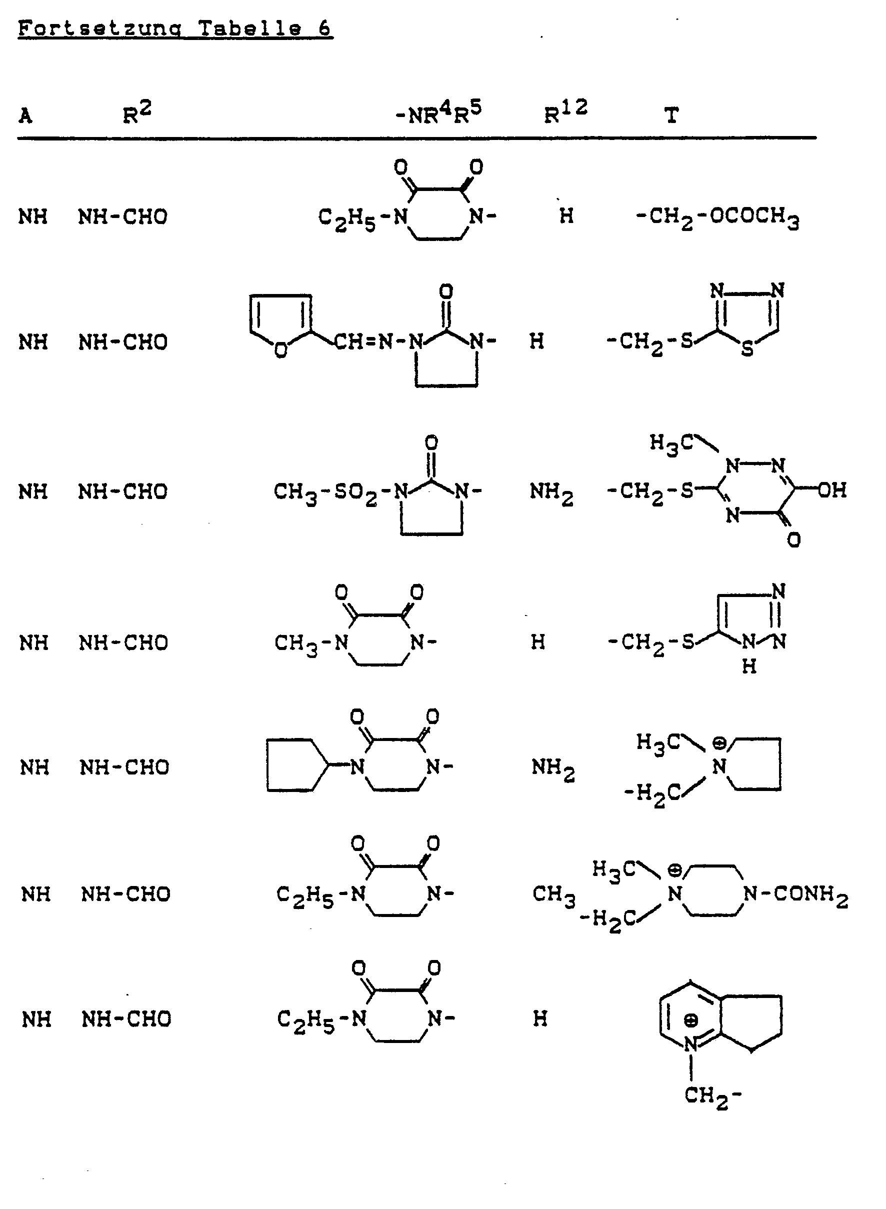

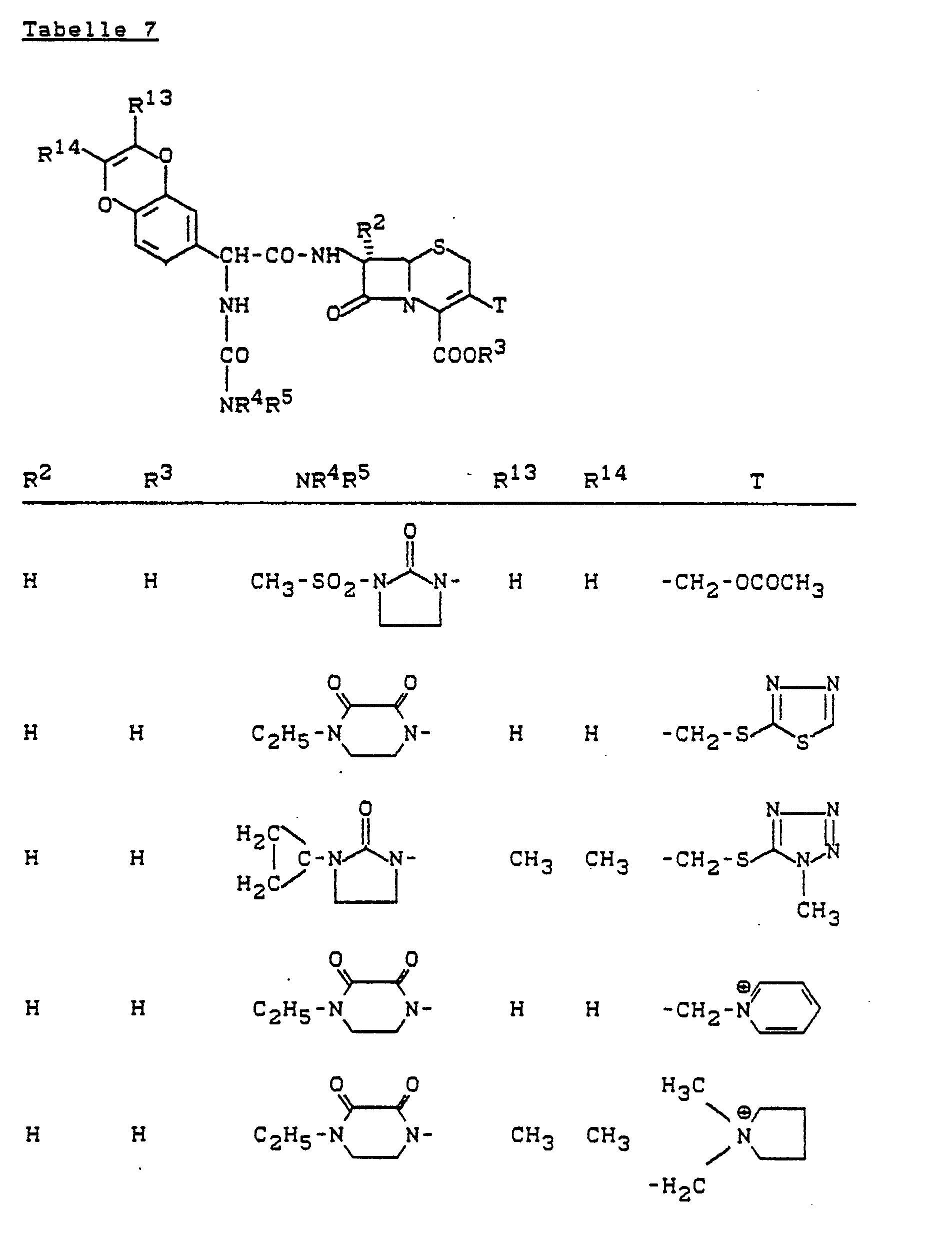

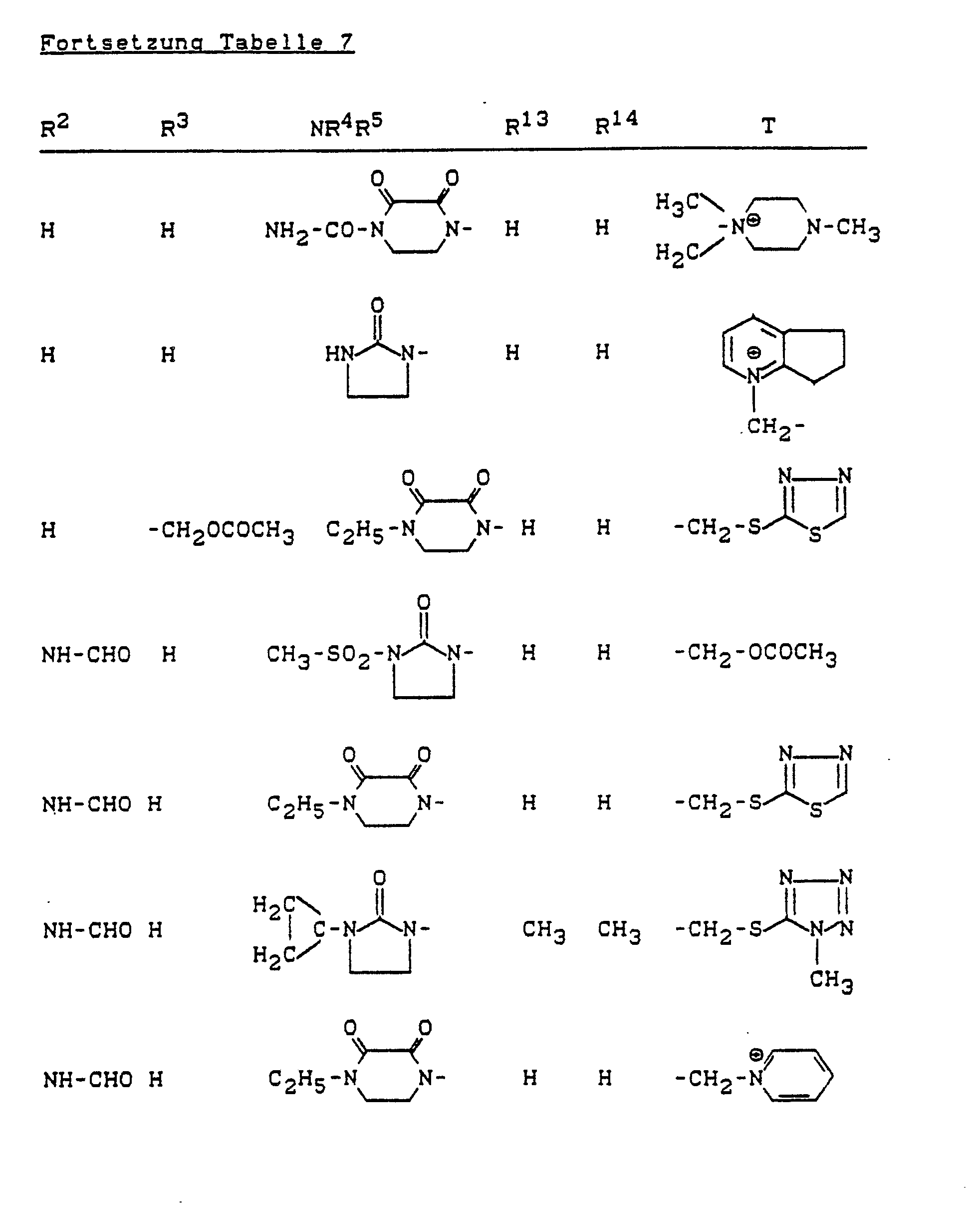

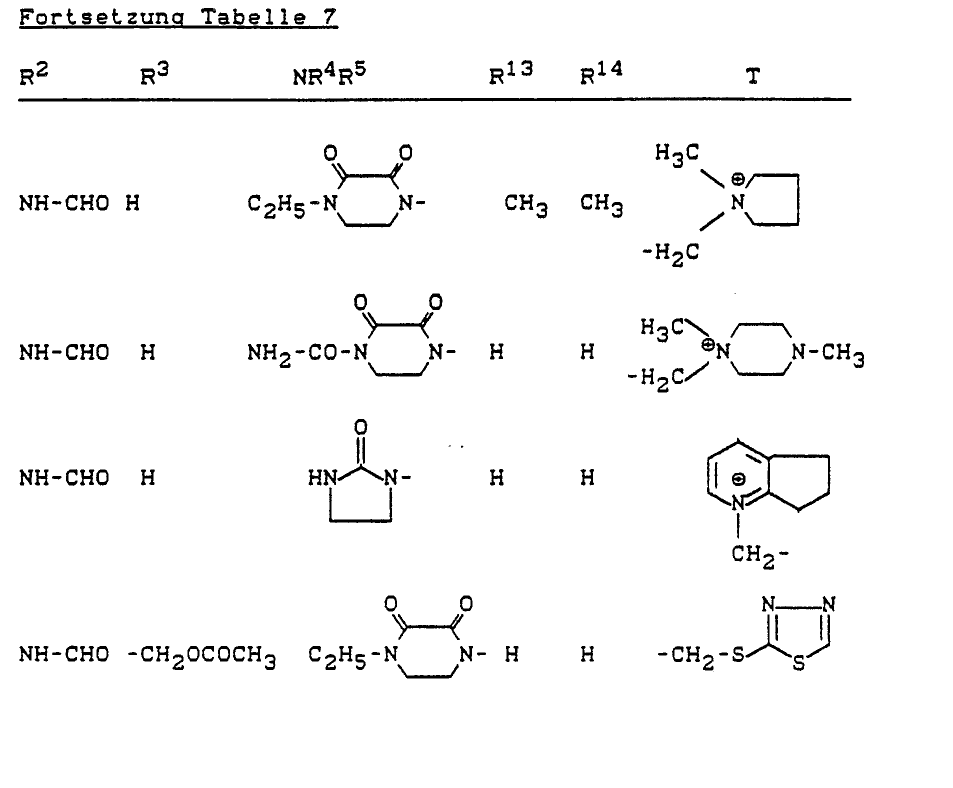

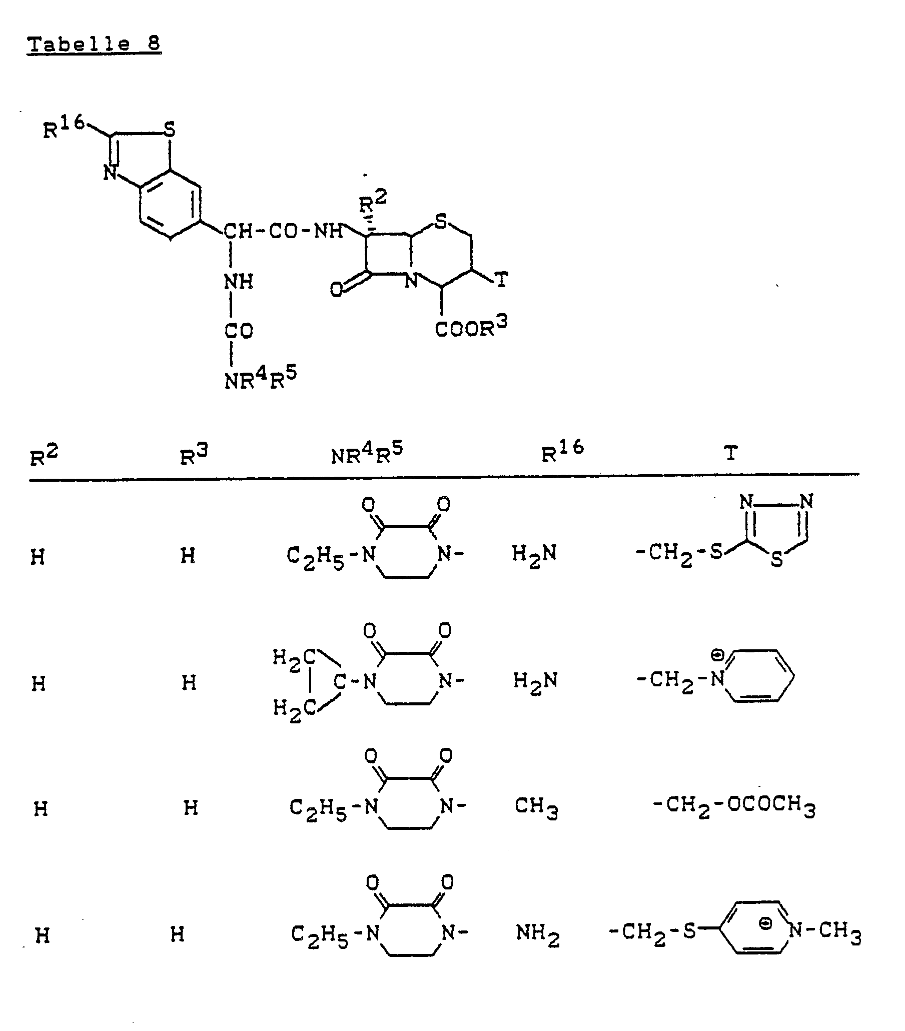

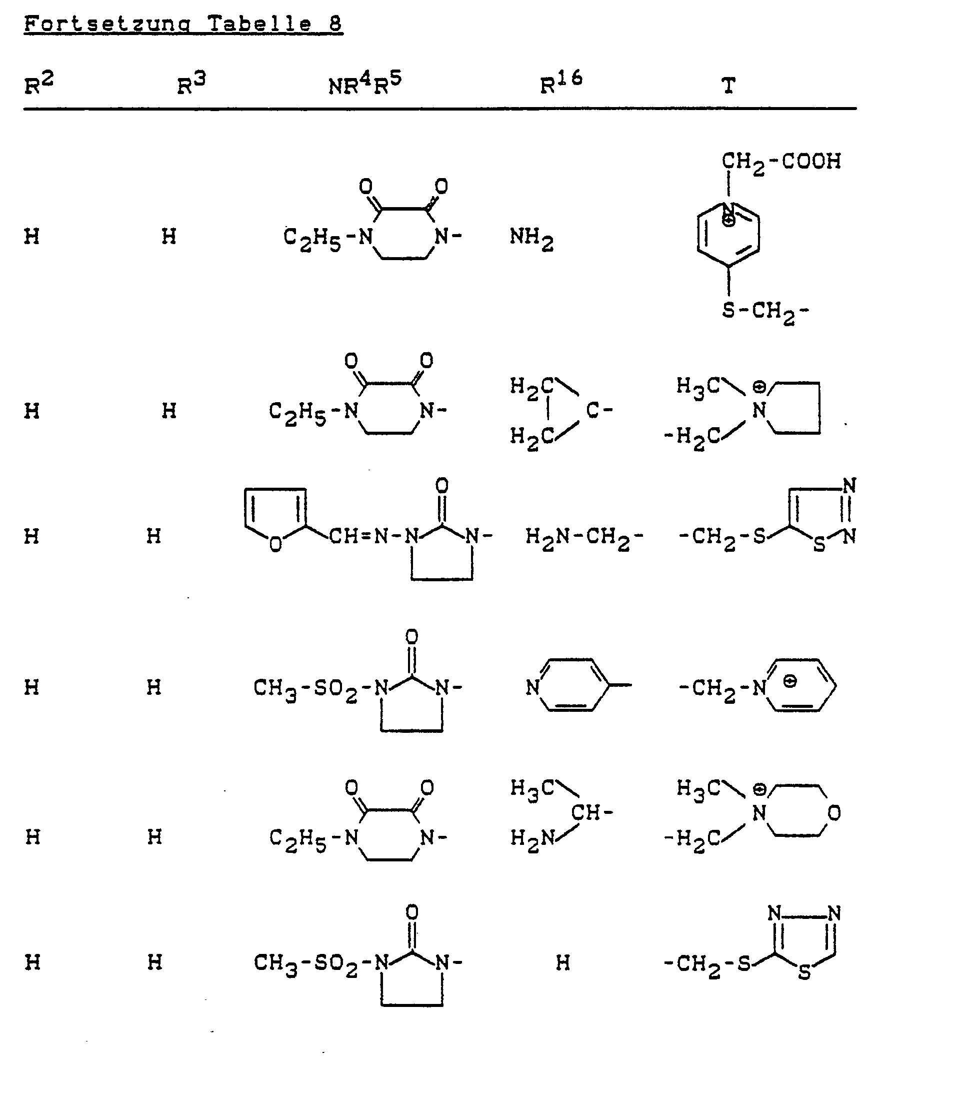

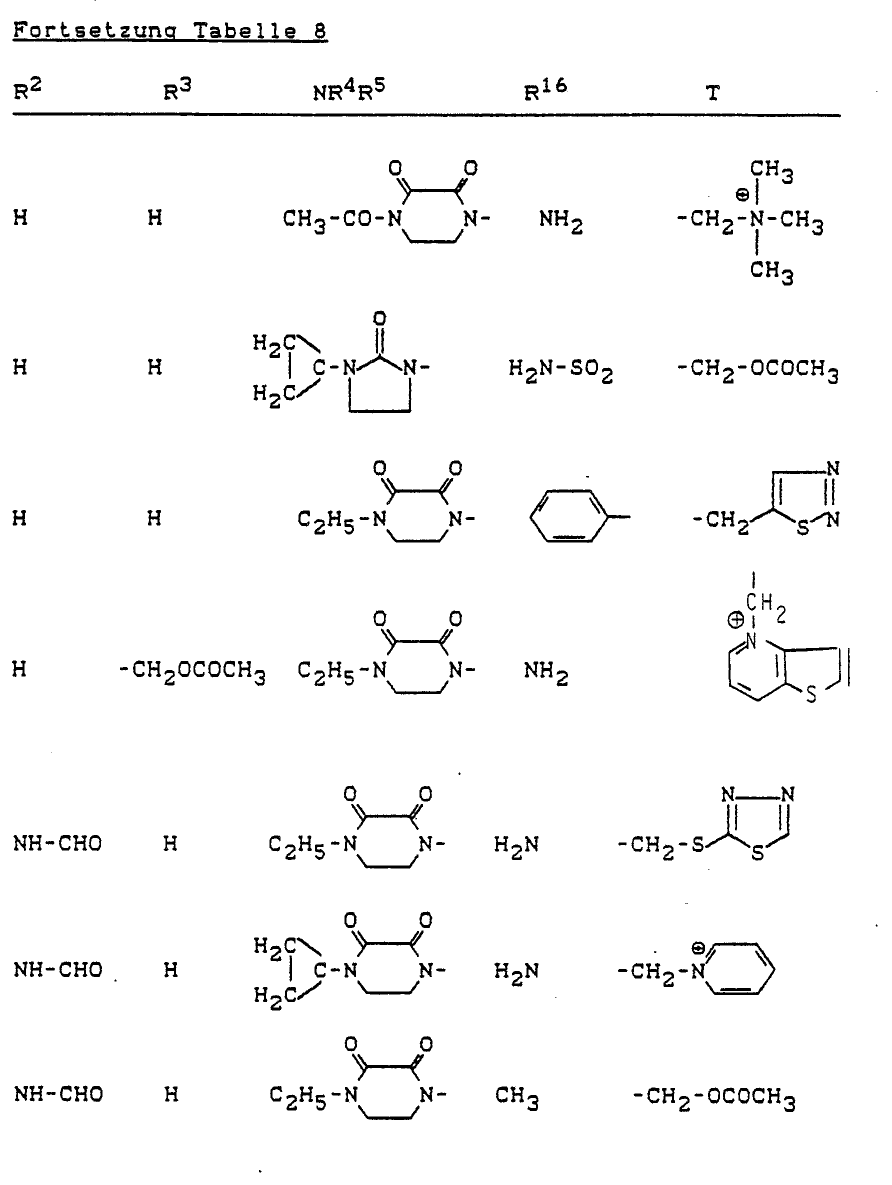

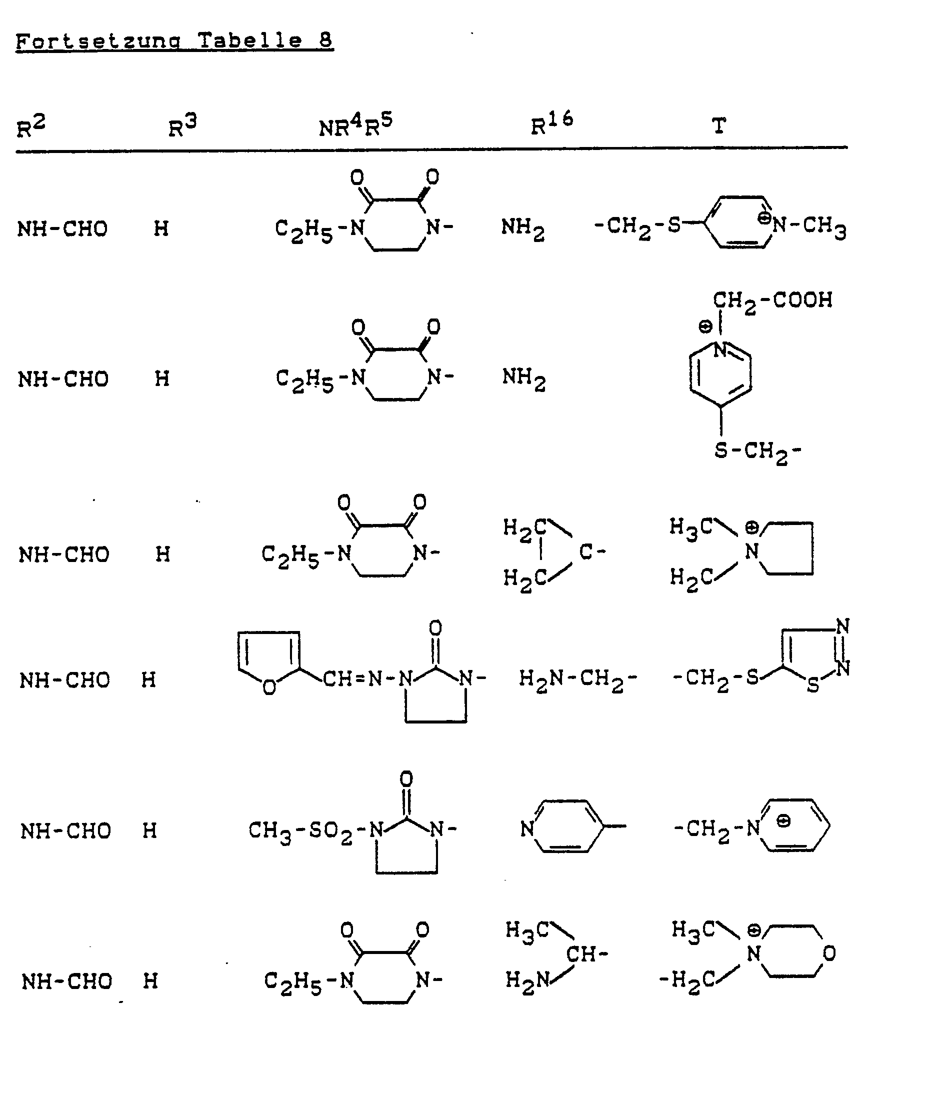

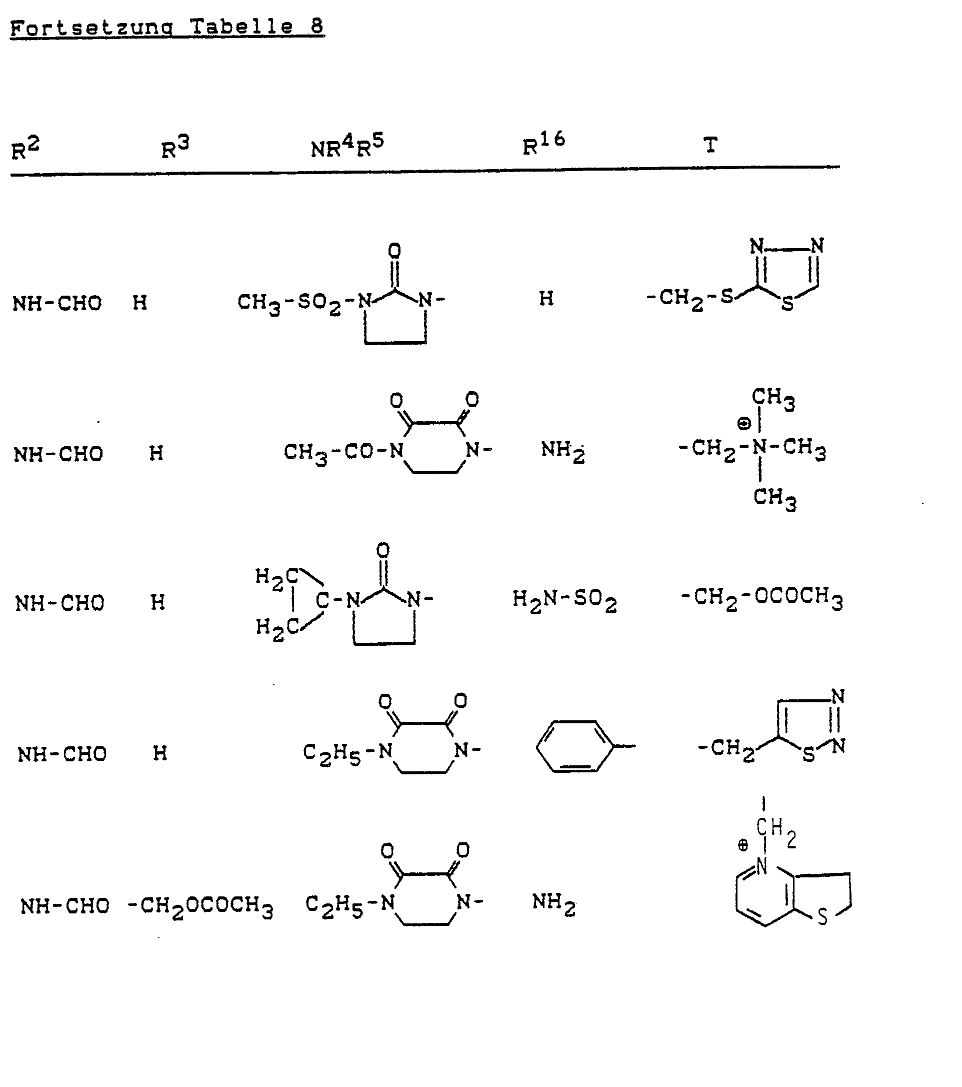

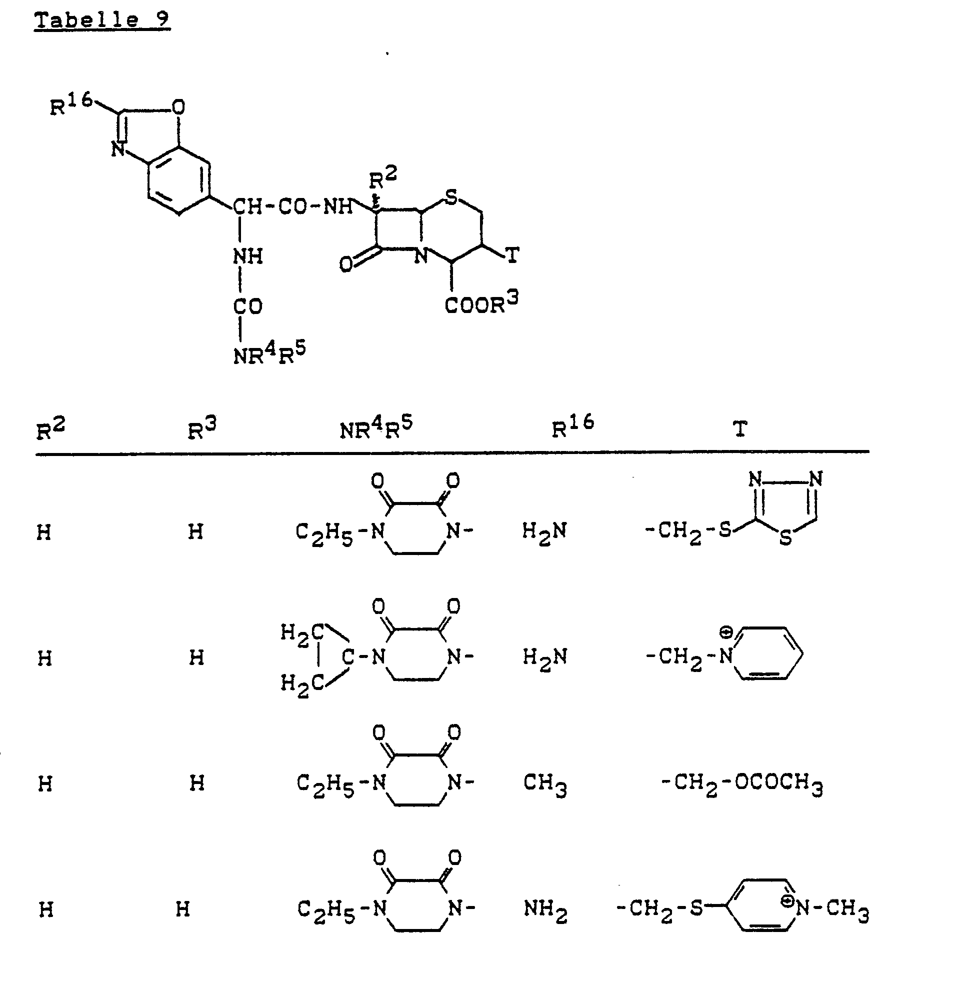

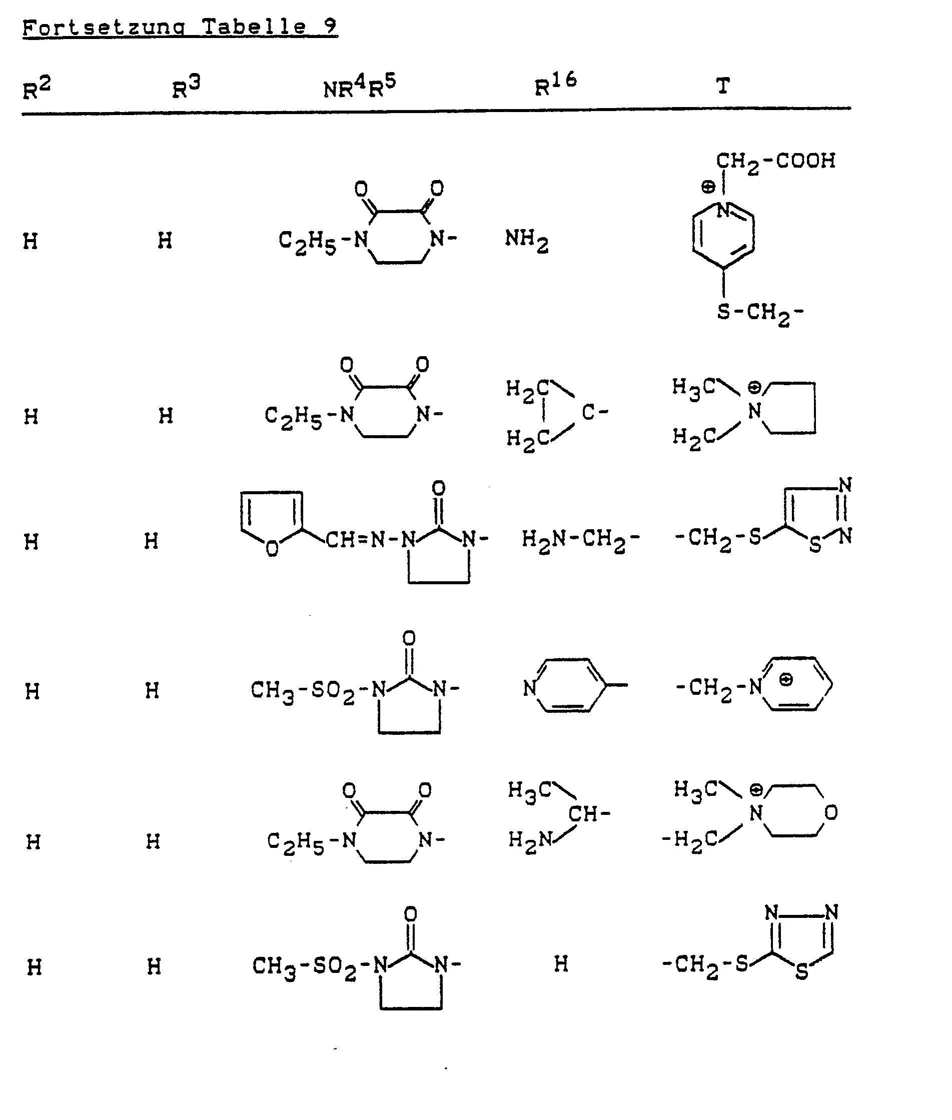

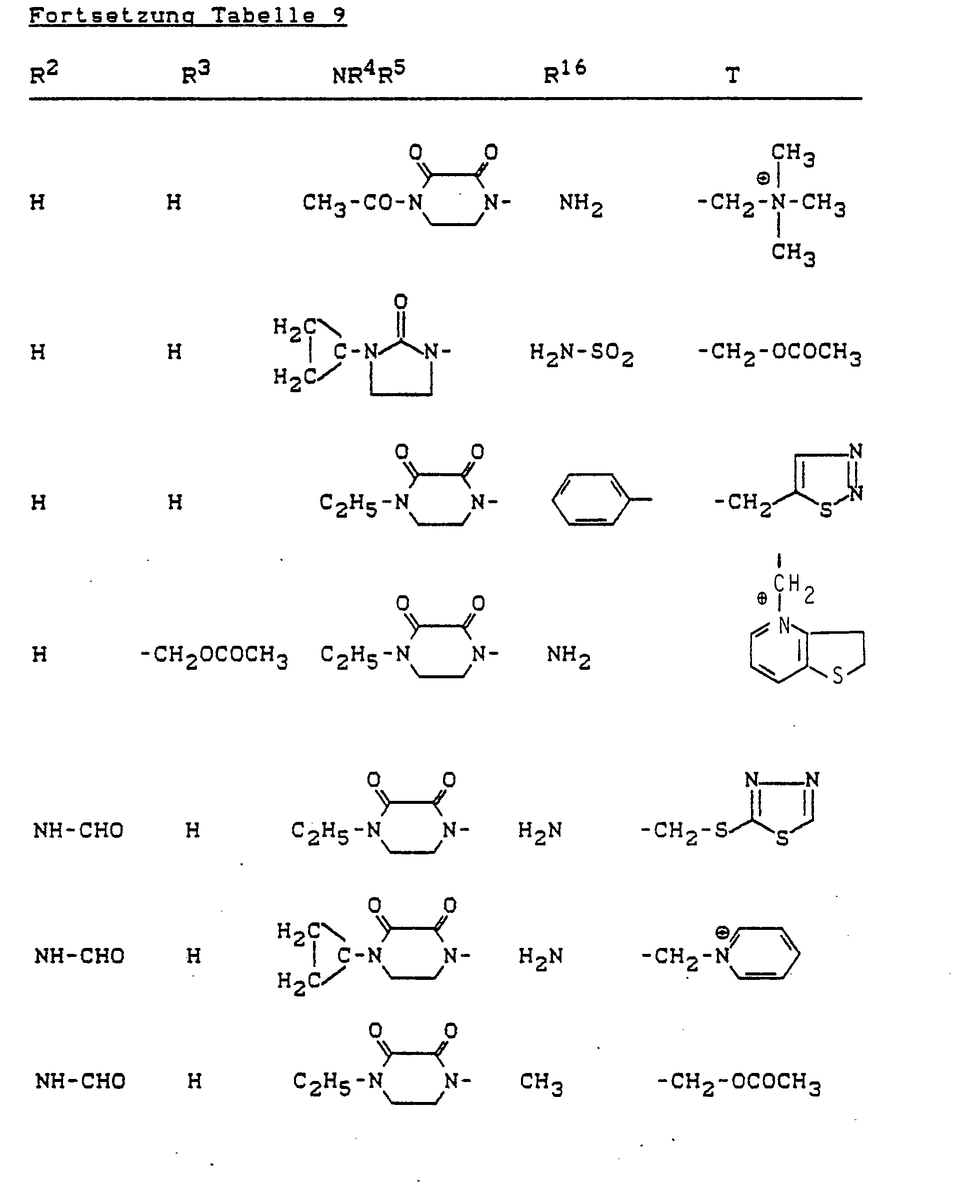

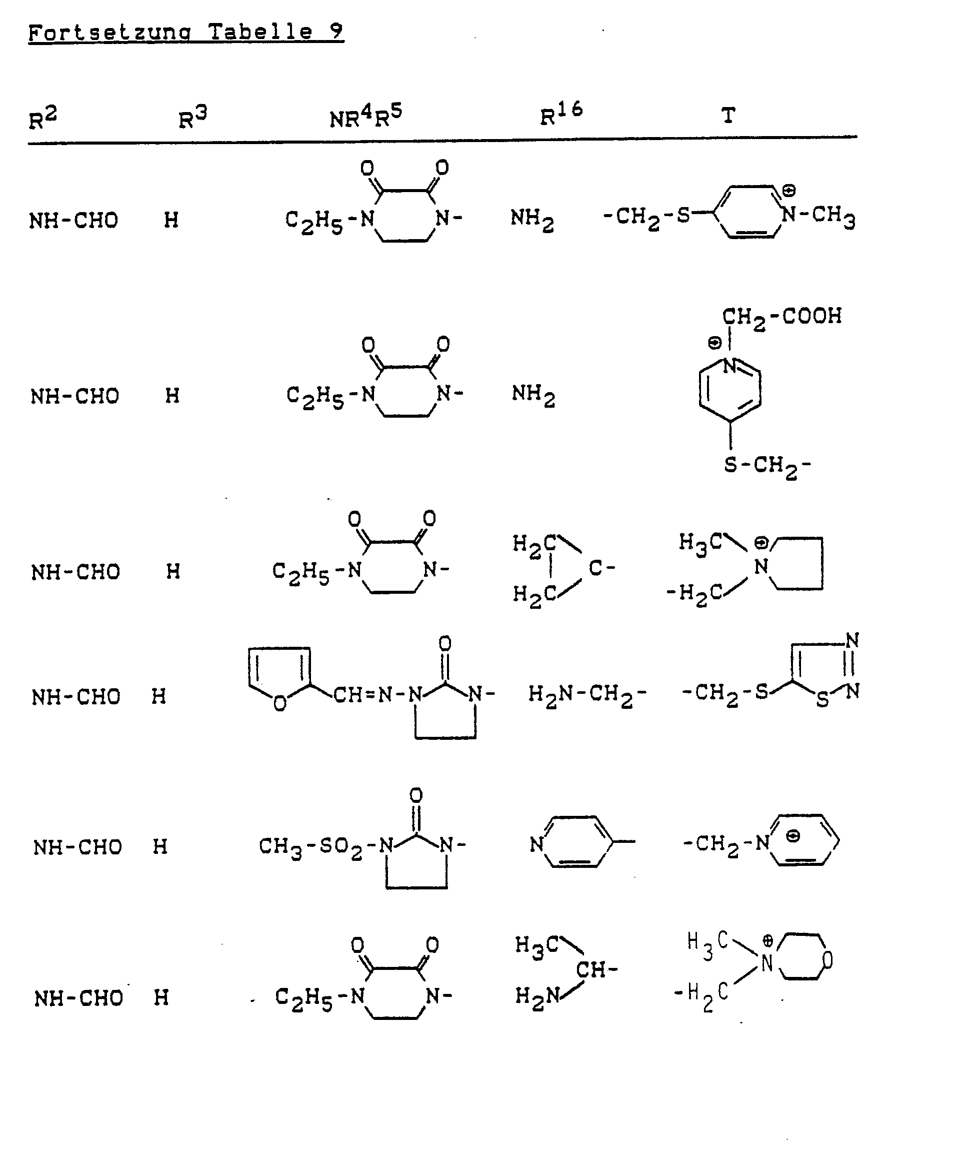

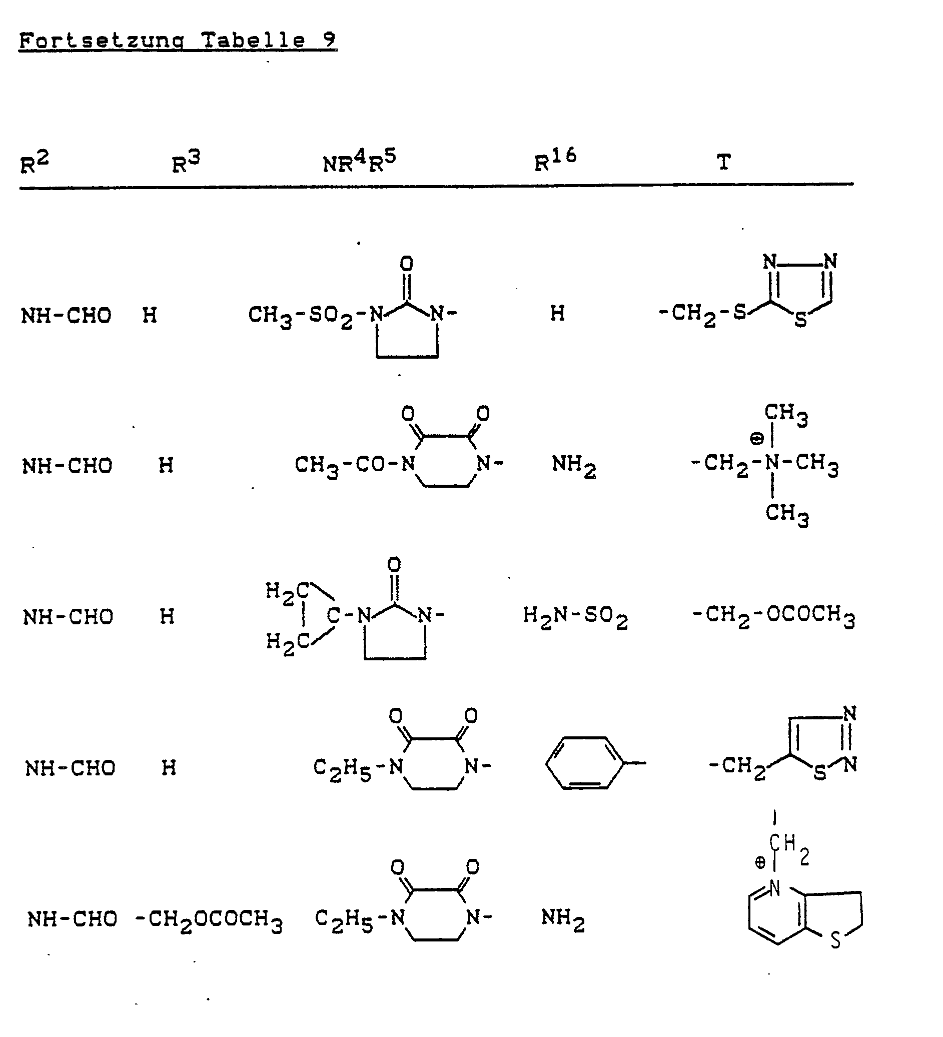

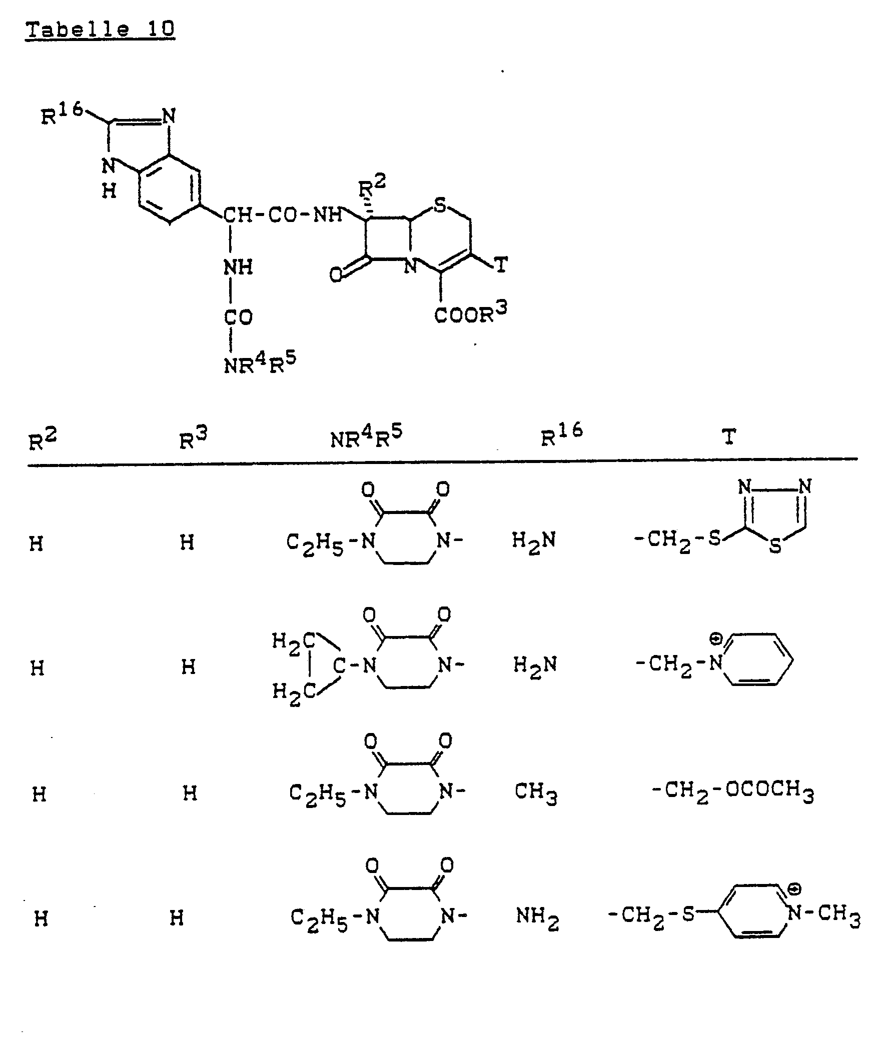

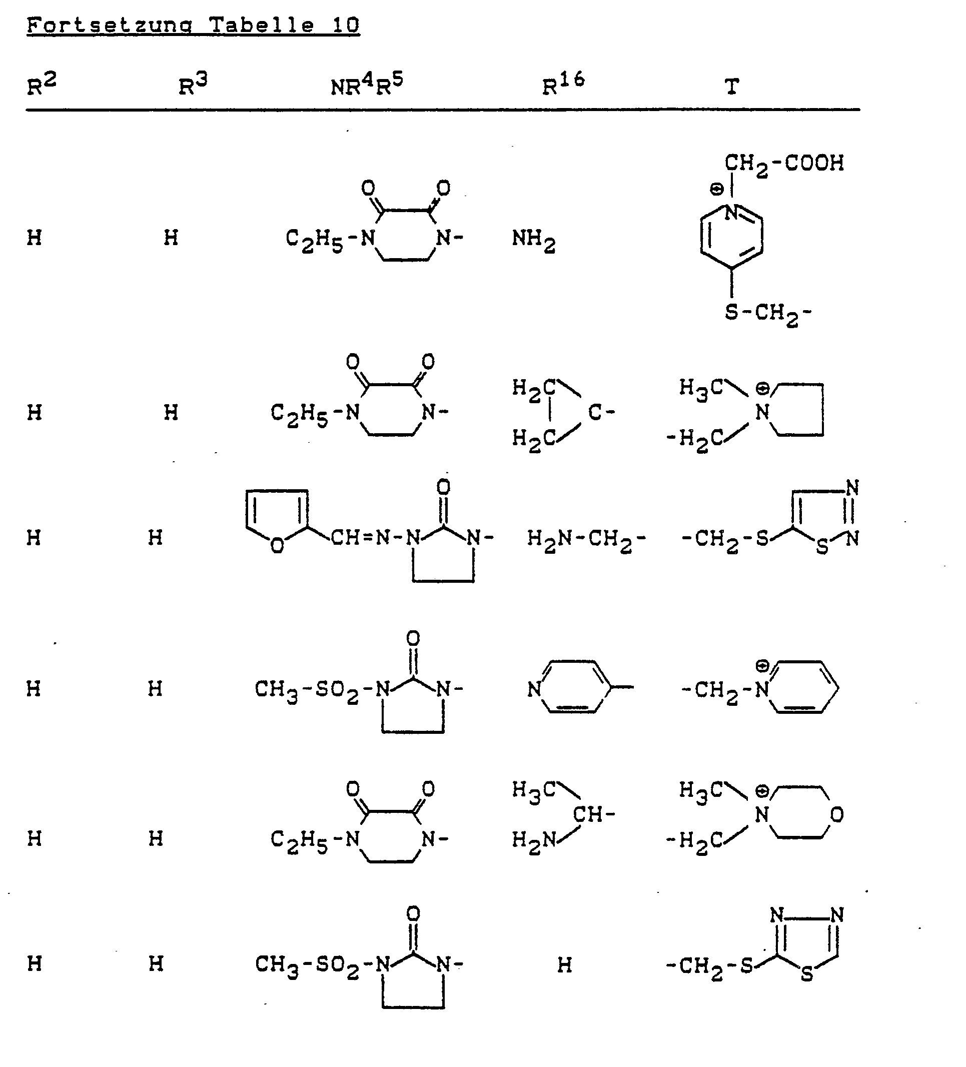

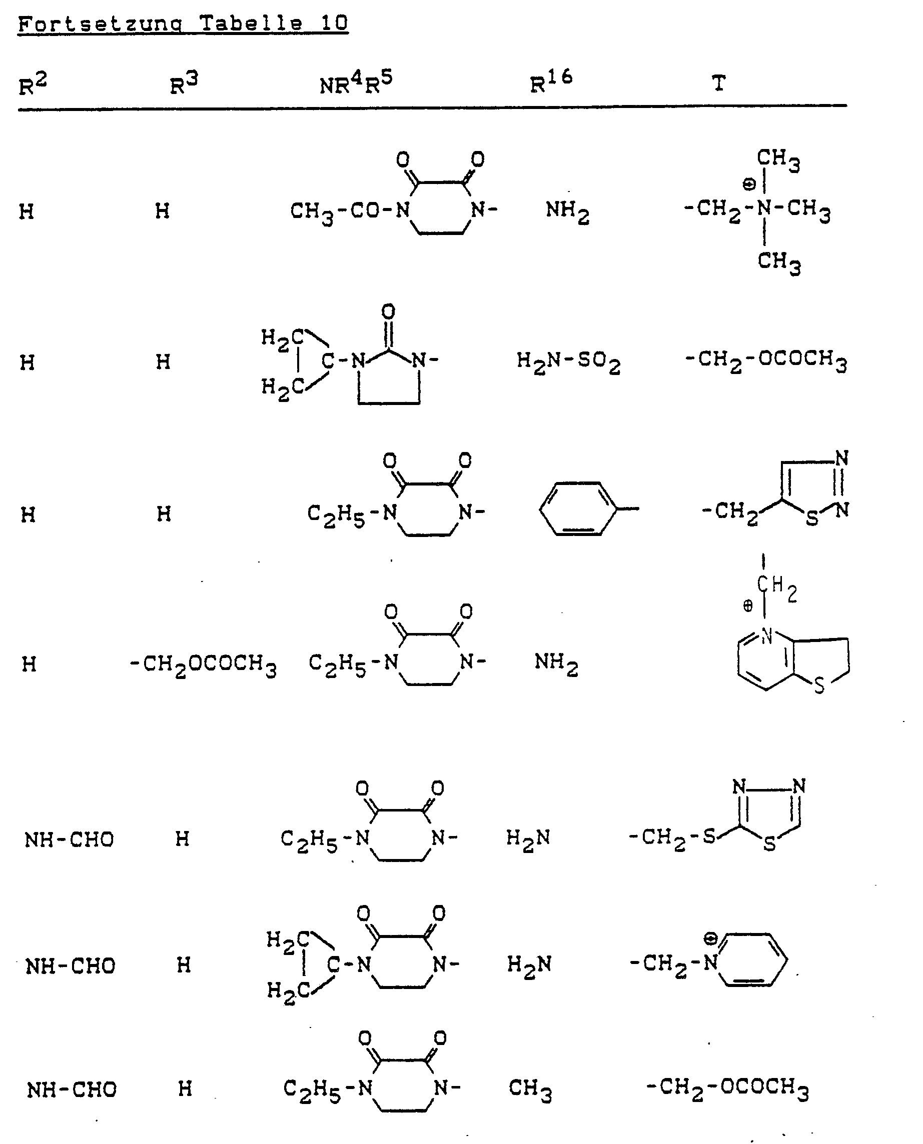

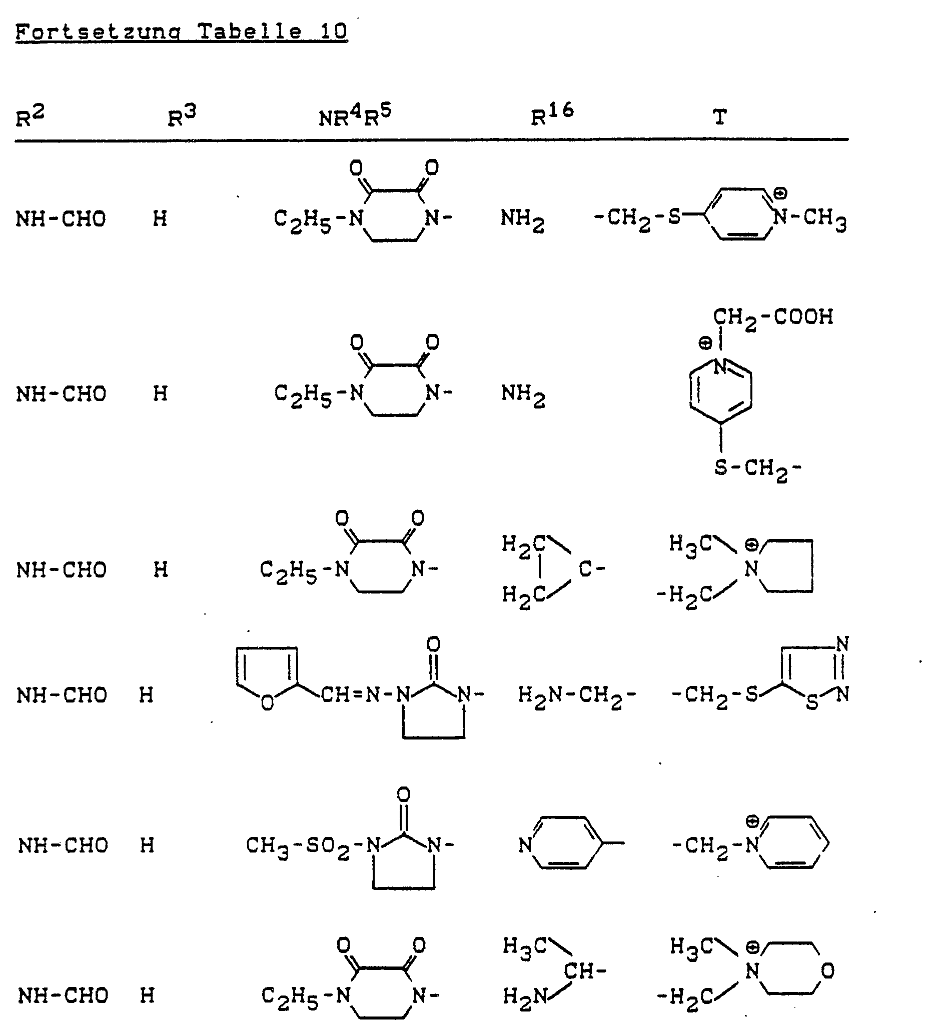

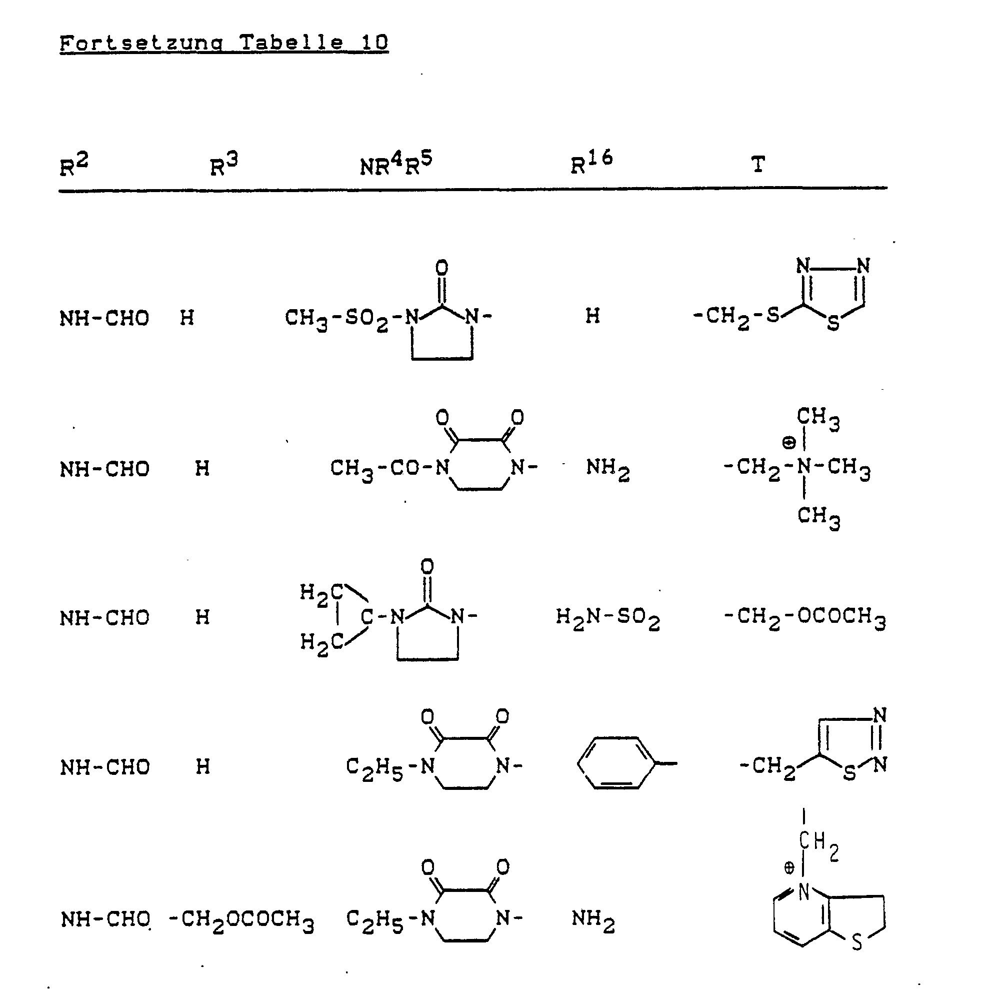

Als neue Wirkstoffe seien im einzelnen folgende Verbindungen in Form ihrer Natriumsalze genannt:
D-6-[α-(4-Ethyl-2,3-dioxo-1-piperazino-carbonylamino)-α-(indol-5-yl)glycyl]penicillansäure
D-6-[α-(Imidazolidin-2-on-1-yl)carbonylamino-α-(indol-5-yl)glycylamido]penicillansäure
D-6-[α-(4-Ethyl-2,3-dioxo-1-piperazino-carbonylamino)-α-(benzofur-5-yl)glycylamido]penicillansäure
6α-Formamido-6β-{D-α-[(4-ethyl-2,3-dioxo-piperazin-1-yl)-carbonylamino]-α-(benzofur-5-yl)-glycylamido}penicillansäure
D-6-[α-(2-Oxo-3-furylidenamino-imidazoliden-1-yl-carbonylamino)-α-(benzofur-5-yl)-glycylamido]-penicillansäure
D-6-[α-(4-Methyl-2,3-dioxo-1-piperazino-carbonylamino)-α-(1,4-benzodioxin-6-yl)glycylamido]penicillansäure
6α-Formamido-6β-{D-α-[4-ethyl-2,3-dioxo-piperazin-1-yl)carbonylamino]-α-(1,4-benzodioxin-6-yl)-glycylamidol-penicillansäure
D-6-[α-(4-Ethyl-2,3-dioxo-1-piperazino-carbonylamino)-α-(2-methylbenzothiazol-6-yl)glycylamido]-penicillansäure
6α-Formamido-6β-{D-α-[(4-ethyl-2,3-dioxo-piperazin-1-yl)carbonylamino]-α-(2-methylbenzothiazol-6-yl)-glycylamido}penicillansäure
6α-Formamido-6β-{D-α-[(4-ethyl-2,3-dioxopiperazin-1-yl)carbonylamino]-α-(2-aminobenzothiazol-6-yl)-glycylamidol-penicillansäure
6α-Formamido-6β-{D-α-[(3-methylsulfonyl-imidazolidin-2-on-1-yl)carbonylamino]-α-(2-aminobenzothiazol-6-yl)glycylamidolpenicillansäure
D-6-[α-(4-Ethyl-2,3-dioxo-1-piperazino-carbonylamino)-α-(2-cyclopropylbenzothiazol-6-yl)glycylamido]-penicillansäure
D-6-[α-(4-Ethyl-2,3-dioxo-1-piperazino-carbonylamino)-α-(2-aminobenzoxazol-5-yl)glycylamido]-penicillansäure
D-6-[α-(4-Ethyl-2,3-dioxo-1-piperazino-carbonylamino)-α-(2-aminobenzoxazol-6-yl)glycylamido]-penicillansäure
6α-Formamido-6β-{D-α-[(3-methylsulfonyl-imidazolidin-2-on-1-yl)carbonylamino]-α-(2-aminobenzoxazol-6-yl)glycylamido}penicillansäure
6α-Formamido-6β-{D-α-[(4-ethyl-2,3-dioxopiperazin-1-yl)carbonylamino]-α-(2-aminobenzoxozol-6-yl)-glycylamido}penicillansäure
D-6-[α-(4-Ethyl-2,3-dioxo-1-piperazino-carbonylamino)-α-(2-amino-1 H-benzimidazol-5-yl)glycylamido]-penicillansäure
6α-Formamido-6β-{D-α-[4-ethyl-2,3-dioxopiperazin-1-yl)carbonylamino]-α-(2-amino-1 H-benzimidazol-5-yl)-glycylamido]penicillansäure
D-6-[α-(3-Methylsulfonyl-imidazolidin-2-on-1-yl-carbonylamino)-α-(2-methyl-1 H-benzimidazol-5-yl)-glycylamido]penicillansäure
6α-Formamido-6β-{D-α-[(3-ethyl-2,3-dioxopiperazin-yl)carbonylamino]-α-(2-methyl-1 H-benzimidazol-5-yl)-glycylamidolpenicillansäure
D-6-[α-(4-ethyl-2,3-dioxo-1-piperazino-carbonylamino)-α-(2-cyclopropyl-1 H-benzimidazol-5-yl)glycyiamido]-penicillansäure
D-7-[α-(4-Ethyl-2,3-dioxo-1-piperazino-carbonylamino)-α-(indol-5-yl)glycylamido]-3-acetoxymethyl-3-cephem-4-carbonsäure
7β-{D-α-[(4-Ethyl-2,3-dioxopiperazin-1-yl)carbonylamino]-α-(indol-5-yl)glycylamido)-7α-formamido-3-(pyridiniomethyl)-3-cephem-4-carbonsäure
7β-{D-α-[(4-Ethyl-2,3-dioxopiperazin-1-yl)carbonylamino]-α-(indol-5-yl)glycylamido}-7-α-formamido-3-[(1,3,4-thiadiazol-2-yl)thiomethyl]-3-cephem-4-carbonsäure
D-7-[α-(4-Ethyl-2,3-dioxo-1-piperazino-carbonylamino)-α-(benzofur-5-yl)glycylamido)-3-(pyridiniomethyl)-3-cephem-4-carbonsäure
7β-{D-α-(4-Ethyl-2,3-dioxo-1-piperazino-carbonylamino-α-(benzofur-5-yl)glycylamido)-7α-formamido-3-(1-methyl-1 H-tetrazol-5-yl-thiomethyl)-3-cephem-4-carbonsäure
D-7-[α-(2-Oxo-3-furylidenamino-imidazolidin-1-yl-carbonylamino)-α-(benzofur-5-yl)glycylamido]-3-[(1,3,4-thiadiazol-2-yl)thiomethyl]-3-cephem-4-carbonsäure
7β-[D-α-(4-Ethyl-2,3-dioxo-1-piperazino-carbonylamino)-α-(benzofur-5-yl)glycylamido]-7α-formamido-3-[-(1,3,4-thiadiazol-2-yl)thiomethyl]-3-cephem-4-carbonsäure
D-7-[α-(4-Ethyl-2,3-dioxo-1-piperazino-carbonylamino)-α-(1,4-benzodioxin-6-yl)glycylamido]-3-(pyridiniomethyl)-3-cephem-4-carbonsäure
7β-[D-α-(Ethyl-2,3-dioxo-1-piperazino-carbonylamino)-α-(1,4-benzodioxin-6-yl)glycylamido]-7α-formamido-3-(1-methyl-1 H-tetrazol-5-yl-thiomethyl)-3-cephem-4-carbonsäure
D-7-[α-(4-Ethyl-2,3-dioxo-1-piperazino-carbonylamino)-α-(2-methylbenzothiazol-6-yl)glycylamido]-3-(pyridiniomethyi)-3-cephem-4-carbonsäure
7β-[D-α-(4-Ethyl-2,3-dioxo-1-piperazino-carbonylamino)-α-(2-cyclopropylbenzothiazol-6-yl)glycylamido]-7α-formamido-3-[(1,3,4-thiadiazol-2-yl)thiomethyl]-3-cephem-4-carbonsäure
7β-[D-α-(3-Cyclopropyl-imidazolidin-2-on-1-yl-carbonylamino)-α-(2-aminobenzothiazol-6-yl)glycylamido]-7α-formamido-3-[(1,3,4-thiadiazol-2-yl)thiomethyl]-3-cephem-4-carbonsäure
D-7-[α-(4-Ethyl-2,3-dioxo-1-piperazino-carbonylamino)-α-(2-aminobenzoxazol-5-yl)glycylamido]-3-(pyridiniomethy!)-3-cephem-4-carbonsäure
D-7-[α-(4-Ethyl-2,3-dioxo-1-piperazino-carbonylamino)-α-(2-aminobenzoxazol-5-yl)glycylamido]-3-[(1-methyl-1-pyrrolidinium)methyl]-3-cephem-4-carbonsäure
D-7-[α-(4-Ethyl-2,3-dioxo-1-piperazino-carbonylamino)-α-(2-amino-1 H-benzimidazol-5-yl)glycylamido]-3-acetoxymethyl-3-cephem-4-carbonsäure
7β-[D-α-(4-Ethyl-2,3-dioxopiperazin-1-yl)carbonylamino)-α-(2-amino-1 H-benzimidazol-5-yl)glycylamido]-7α-formamido-3-(pyridiniomethyl)-3-cephem-4-carbonsäure
7β-{D-α-[(4-Ethyl-2,3-dioxopiperazin-1-yl)carbonylamino]-α-(2-methyl-1 H-benzimidazol-5-yl)-glycylamido}-7α-formamido-3-(1-methyl-1 H-tetrazol-5-yl-thiomethyl)-3-cephem-4-carbonsäure
D-7-[α-(4-Ethyl-2,3-dioxo-1-piperazino-carbonylamino)-α-(2-amino-1 H-benzimidazol-5-yl)glycylamido]-7α-formamido-3-[(2,3-cyclopenteno-1-pyridinio)methyl]-3-cephem-4-carbonsäure
D-7-[α-(2-Oxo-3-furylidenamino-imidazolidin-1-yl-carbonylamido)-α-(2-amino-1 H-benzimidazol-5-yl)-glycylamido]-7α-formamido-3-[(1,3,4-thiadiazol-2-yl)thiomethyl]-3-cephem-4-carbonsäure

Die erfindunsgemäßen Verbindungen der allgemeinen Formel I weisen bei geringer Toxizität ein breites antibakterielles Spektrum gegen gram-positive und gram-negative Keime auf. Diese Eigenschaften ermöglichen ihre Verwendung als chemotherapeutische Wirkstoffe in der Human-und Tiermedizin.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs, pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps, maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis. Insbesondere wirken die erfindungsgemäßen Stoffe gegen Staphylokokken, Streptokokken,Enterokokken und Haemophilus influenzae. Bei parenteraler oder insbesondere oraler Verabreichung sind die neuen Verbindungen gegen Mikroorganismen wie Staphylokokken, Streptokokken, Enterobakteriaceen, Escherichia coli, Klebsiella, Salmonella, Shigella. und Proteus sehr gut wirksam.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankunger der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Metritis-Mastitis-Agalaktiae-Syndrom, Mastitis:
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonie, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis; Geflügel (Huhn, Pute, Wachtel, Taube, Ziervögel und andere): Mycoplasmose, E. coli-Infektionen, chronische Luftwegserkrankungen, Salmonellose, Pateurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

In der folgenden Tabelle sind die minimalen Hemmkonzentrationen (MHK-Werte, ug/M1) für die Beispiele 1, 3 und 4 im Vergleich mit Piperacillin angegeben. Die MHK-Werte werden durch den Agarverdünnungstest mit Hilfe eines Multipoint-Inokulators bestimmt, wobei die Ablesung nach 18- bis 24- stündiger Bebrütung bei 37° C erfolgt. Als Wuchsmedium wird Isosensitest-Agar verwendet.

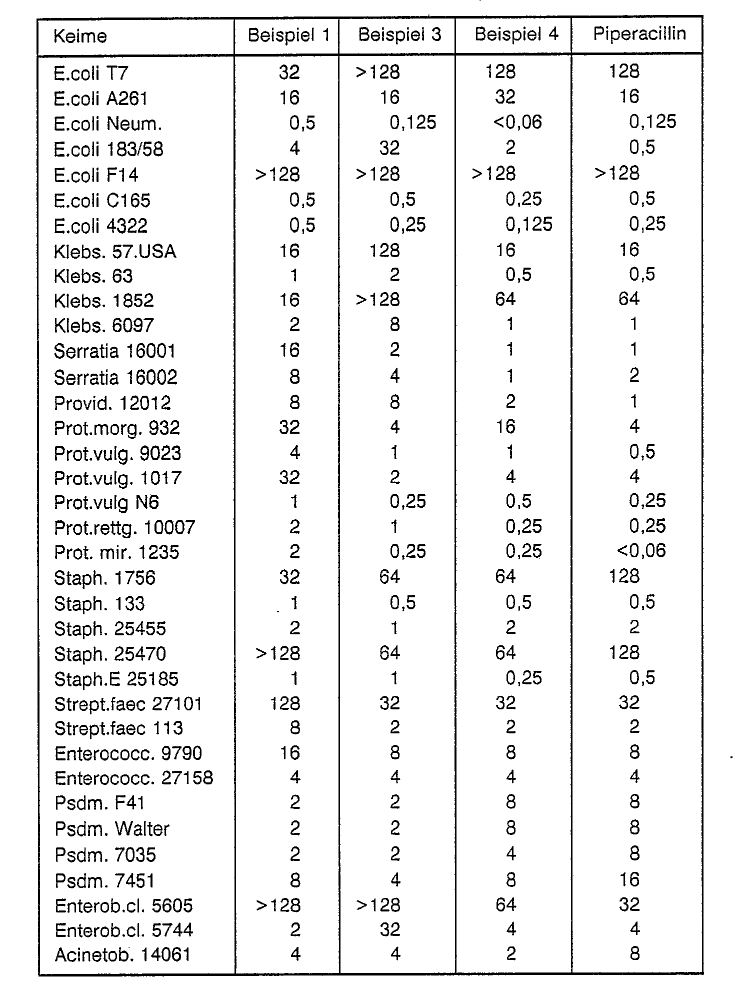

Zur vorliegenden Erfindung gehören pharmazeutische Zubereitungen, die neben nicht-toxischen, inerten pharmazeutisch geeigneten Trägerstoffen eine oder mehrer erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1_{/}3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Träger stoffe enthalten, z.B. Polyethylenglykole, Fette, z.B. Kakaofett und höhere Ester (z.B. Cu-Alkohol mit Ci 5-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe, Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs-und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.- %, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophthalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen.

Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe.in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch eine Förderung des Wachstums und eine Verbesserung der Verwertung des Futters erreicht werden.

Die erfindungsgemäßen Verbindungen können zum Zwecke der Erweiterung des Wirkungsspektrums und um eine Wirkungssteigerung speziell bei β-lactamasebildenden Bakterien zu erzielen mit anderen antimikrobiellen Wirkstoffen und Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotica, wie z.B. Gentamicin, Sisomicin, Kanamicin, Amikacin oder Tobramicin kombiniert werden.

### Herstellungsbeispiele

Die Erfindung wird anhand der folgenden Beispiele weiter erläutert.

### Beispiel 1

D-7-[α-(4-Ethyl-2,3-dioxo-1-piperazino-carbonylamino)-α-(2-aminobenzothiazol-6-yl)glycylamido]-3-(1-methyl-1 H-tetrazol-5-yl-thiomethyl)-3-cephem-4-carbonsäure-Natrium

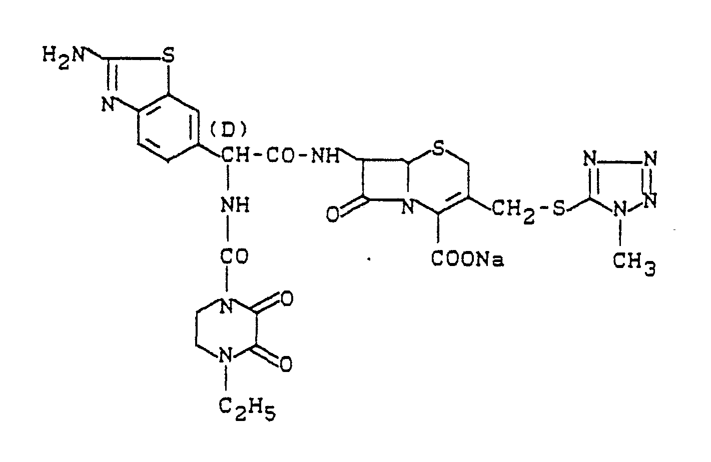

### a) D-7-[2-(Aminobenzthiazol-6-yl)glycyi-amido]-3-(1-methyi-1H-tetrazol-5-yl-thiomethyl)-3-cephem-4-carbonsäure

### Aktivierung der Precursorsäure:

5 g (0,0155 mol) D-α-t-Butyloxycarbonyl-amino-α-(2-aminobenzthiazol-6-yl)-essigsäure werden in 25 ml Di methylformamid in Gegenwart von 2,69 ml (0,0155 mol) Ethyldiisopropylamin bei Raumtemperatur gelöst, bei -50 °C tropfenweise mit 1,20 ml (0,0155 mol) Mesylchlorid versetzt und 40 min. bei -50 °C gerührt.

### Zubereitung der Aminkomponente:

5,96 g (0,0155 mol) 7-Amino-3-(1-methyl-1H-tetrazol-5-yl-thiomethyl)-3-cephem-4-carbonsäure-t-butylester werden in 15 ml Tetrahydrofuran und 20 ml Methylenchlorid gelöst, bei Raumtemperatur mit 2,69 ml (0,0155 mol) Ethyldiisopropylamin versetzt und bei -50 C zum Mesylat der Percursorsäure zugespritzt.

### Kupplung und Isolierung

Nach Zugabe der Aminlösung wird noch 15 min. bei -50° C gerührt; dann läßt man ohne Kältebad in 35 min. die Temperatur auf 20° C ansteigen. Die Reaktionslösung wird in 100 ml Wasser / 800 ml Essigester eingerührt, die wäßrige Phase wird abgetrennt und die organische Phase dreimal mit je 100 ml 0,2 N HCI, zweimal mit je 100 ml NaHCO₃-Lösung und zum Schluß mit Kochsalzlösung gewaschen. Nach Trocknen und Einengen der Essigesterphase wird der Rückstand in 200 ml Trifluoressigsäure (TFE) Methyienchiorid (1:1) aufgenommen und 1 h bei Raumtemperatur gerührt. Anschließend wird das Trifluoressigsäure- .Methylenchlorid-Gemisch im Vakuum abdestilliert und der ölige Rückstand mit Ether versetzt. Das Trifluoracetat wird abgesaugt, mit Ether gewaschen und getrocknet.

Ausbeute: 7,6 g

Das Trifluoressigsäure-Salz wird in 500 ml Wasser und 40 ml Eisessig mittels Ultraschallbad weitgehends in Lösung gebracht. Die Lösung wird auf eine Säule mit 100 ml Amberlite IRA-68 (Acetat-Form) gegeben, mit 1000 ml 10%iger Essigsäure nachgewaschen und lyophilisiert.

Ausbeute: 5 g

Das Lyophilisat wird in 30 ml 50%iger Essigsäure gelöst und an einer Whatman-Säule (Magnum 500 x 40, Partisil C₁₈, 50 µm) zunächst mit 3%iger Essigsäure und danach mit 3% Essigsäure unter Zusatz von 10% Acetonitril chromatographiert.

Ausbeuten:
1. Fraktion
HPLC-Gehalt: 69%
2. Fraktion
HPLC-Gehalt: 95,2%
Hibar 250-4, RP-8, 10 µm,
Laufmittel: 1000 ml Wasser / 30 ml Essigsäure, 254 nm, 2 ml/min.
Retention 9,14
C₁₉H₁₉N₉O₄S₃ x 2H₂0 x CH₃COOH (629,64)
Ber.: C 40,06 H 4,32 N 20,02 S 15,27
Gef.: C 40,4 H 4,1 N 19,7 S 15,3
NMR (DCOOD): 5 = 3,64 (d, 1 H); 3,78 (d, 1 H); 4,09 (s, 3H); 4,24 (d, 1 H); 4,46 (d, 1 H); 5,22 (d, 1 H); 5,74 (s, 1 H); 5,92 (d, 1 H); 7,84 (s, 2H); 8,14 (s, 1 H) ppm.

### b) D-7-(α-(4-Ethyl-2,3-dioxo-1-piperazino-carbonylamino)-α-(2-aminobenzothiazol-6-yl)glycylamido]-3-(1-methyl-1 H-tetrazol-5-yl-thiomethyl)-3-cephem-4-carbonsäure-Natrium

2 g (3,17 mmol; Gehalt 69%) 1a werden in 120 ml Tetrahydrofuram/Wasser (80:20) suspendiert, mit einem Eisbad auf 5°C bis 10° C gekühlt und unter Rühren mit Triethylamin mittels Autotitrator (MemoTitrator DL 40, Mettler) auf pH 8,0 eingestellt. Die erhaltene klare Lösung wird mit 0,972 g (4,75 mmol) 4-Ethyl-2,3-dioxo-1-piperazinocarbonyl-chlorid -gelöst in 60 ml Tetrahydofuran - tropfenweise versetzt. Der pH-Wert wird durch entsprechende Zugabe von 10%igen Triethylamin in Tetrahydrofuran bei pH 7,5 mittels Memotitrator gehalten. Während des 90-minütigen Nachrührens werden noch 100 ml Wasser in mehreren Portionen hinzugefügt. Anschließend wird die Reaktionslösung mit ca. 200 ml Wasser und 600 ml Essigester verdünnt, der pH-Wert auf 2,5 eingestellt und die Essigesterphase abgetrennt. Nach Waschen der organischen Phase mit Kochsalzlösung, Trocknen über Natriumsulfat und Einengen bis zu Trockene erhält man 1,15 g (71,0% der Theorie). Das Material wird an einer präparativen Säule (Hibar 250-25, RP 18, 7 um) chromatographiert (siehe Beispiel 2b) unter Verwandlung des Laufmittelsystems Wasser-Acetonitril mit einem Gradient von 2% bis 20%.
Ausbeute: 550 mg (34% der Theorie)
C₂₆H₂₇N₁₁O₇S₃ x 2H₂O (737,8)
Ber.: C 42,33 H 4,24 N 20,88 S 13,04
Gef.: C 41,9 H 4,1 N 20,15 S 13,8

### Beispiel 2

### D-7-[α-(4-Ethyl-2,3-dioxo-1-piperazino-carbonylamino)-α-(2-aminobenzothiazol-6-yl)glycylamido]-3-acetoxymethyl-3-cephem-4-carbonsäure-Natrium

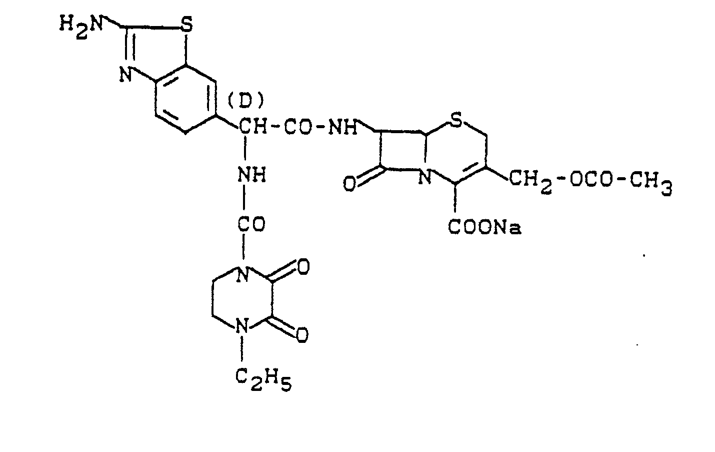

### a) D-7-[(2-Aminobenzothiazol-6-yl)glycylamido]-3-acetoxymethyl-3-cephem-4-carbonsäure

1,62 g (5 mmol) D-a-t-Butyloxycarbonyl-amino-a-(2-aminobenzthiazol-6-yl)-essigsäure werden in 12 ml Dimethylformamid in Gegenwart von 0,871 ml (5 mmol) Ethyldiisopropylamin bei Raumtemperatur gelöst, bei -50 C tropfenweise mit 0,387 ml (5 mmol) Mesylchlorid versetzt und 40 min. bei -50° C gerührt.

### Zubereitung der Aminkomponente:

1,64 g (5 mmol) 7-Amino-3-acetoxy-methyl-3-cephem-4-carbonsäure-t-butylester werden in 12 ml Methylenchlorid gelöst, bei Raumtemperatur mit 0,871 ml (5 mmol) Ethyldiisopropylamin versetzt und sofort in einem Guß zum Mesylat der Precursorsäure geschüttet.

### Kupplung und Isolierung

Nach Zugabe der Aminlösung wird noch 15 min. im Kältebad bei -50° C gerührt. Danach läßt man innerhalb von 45 min. die Temperatur auf 0 C ansteigen. Die Reaktionslösung wird in 400 ml Essigester / 200 ml Wasser eingerührt, die wäßrige Phase abgetrennt und die organische Phase wird zweimal mit 0,2 N HCI gewaschen. Um eine besserer Phasentrennung zu bekommen, wird Methanol zugesetzt. Danach wird die organische Phase zweimal mit gesättigter NaHCO₃-Lösung und Kochsalzlösung gewaschen, getrocknet und bis zu Trockene eingeengt. Der Rückstand wird 20 min. bei Raumtemperatur mit 50 ml Trifluoressigsäure und 0,5 ml Anisol behandelt. Danach wird die Trifluoressigsäure bis zu Trockene eingeengt, der ölige Rückstand mit Ether verrieben und das Trifluoressigsäure-Salz abgesaugt. Ausbeute: 1,56 g

Das Trifluoracetat wird in Wasser Essigsäure (30 ml, 1:1) gelöst und am Adsorberharz HP 20 (500 ml, Dianion, Mitsubishi) chromatographiert. Man eluiert mit Wasser, das kontinuierlich mit einem steigenden Gehalt von Acetonitril (0% bis 40%) und Essigsäure (0% bis 40%) versetzt wird. Man sammelt insgesamt 10 Fraktionen ä 400 ml, wobei die Fraktionen 6 und 7 die gewünschte Verbindung enthalten.

Ausbeute: 473 mg (16,5% der Theorie).
C₁₉H₁₉N₅O₆S₂ x 2H₂0 x CH3COOH (573,56)
Ber.: C 43,98 H 4,74 N 12,21
Gef.: C 43,5 H 4,5 N 12,5
NMR (DCOOD): 5 = 2,16 (s, 3H); 3,45 (d, 1 H); 3,68 (d, 1 H); 4,96 (d, 1 H); 5,17 (d, 1 H); 5,20 (d, 1 H); 5,71 (s, 1 H); 5,92 (d, 1 H); 7,78 (s, 2H); 8,1 (s, 1 H) ppm.
HPLC-Gehalt: 95,5%; Hibar 250-4, RP-8, 10 um;
Laufmittel: 1000 ml H_{z}O/40 ml CH₃CN / 1 ml TFE, 254 nm; 4 ml/min;
Retention: 2,33

### b) D-7-[α-(4-Ethyl-2,3-dioxo-1-piperazinocarbonylamino)-α-(2-aminobenzothiazol-6-yl)glycylamido]-3-acetoxymethyl-3-cephen-4-carbonsäure-Natrium

0,573 g (1 mmol) 2a werden in 30 ml Tetrahydrofuran / Wasser (80:20) suspendiert, mit einem Eisbad auf 5 C bis 10° C gekühlt und unter magnetischem Rühren mit Triethylamin mittels Autotitrator auf pH 8,0 eingestellt. Die erhaltene klare Lösung wird mit 0,307 g (1,5 mmol) 4-Ethyl-2,3-dioxo-1-piperazinocarbonyl- chlorid in 20 ml Tetrahydofuran tropfenweise versetzt. Der pH-Wert wird durch entsprechende Zugabe von 10%igem Triethylamin in Tetrahydrofuran bei 7,5 gehalten. Während des Nachrührens (90 min.) werden noch 30 ml Wasser zugegeben. Man verdünnt nun die Reaktionslösung mit 50 ml Wasser / 400 ml Essigester, stellt den pH auf 2,5 ein und extrahiert mit Essig ester. Nach dem Waschen mit Kochsalzlösung, Trocknen über Magnesiumsulfat und Einengen der organischen Phase erhält man 200 mg Rohprodukt. Die wäßrige Phase aus der Essigester-Extraktion wird auf pH 5 gestellt und lyophilisiert (Ausbeute: 24,8 g). Beide Substanzen werden in 60 bis 70 ml 10%iger Essigsäure gelöst (Ultraschallbad), unlösliches Material wird abgesaugt, das Filtrat nochmals über ein Millipore-Filter filtriert und an einer Wathman-Säule (Magnum 500 x 40 mm, Partisil C₁₈, 50 um) chromatographiert, wobei zunächst 2000 ml 3%ige Essigsäure, danach 3%ige Essigsäure bei steigendem Gradient von Acetonitril von 10% bis 20% als Laufmittel dienen. Die gewünschte Verbindung wird mit 3%iger Essigsäure unter Zusatz von 20% Acetonitril eluiert.

Ausbeute: 187 mg
Da die eluierte Verbindung Δ2-Isomeres als Nebenprodukt enthält, wird das gesamte Material nochmals an einer Merck-Säule (Hibar 250-25, RP-18, 7 um) mit dem Laufmittel 900 ml Wasser 100 ml Acetonitril 10 ml Eisessig chromatographiert.
Ausbeute: 36 mg
HPLC-Gehalt: 88,8%, Hibar 250-4, RP 8, 10 um,
Laufmittel: 100 ml Acetonitril / 1000 ml Wasser
254 nm, 2 ml/min.
Retention: 5,33
NMR (DMSO): δ = 1,1 (t, 3H); 2,02 (s, 3H); 3,34 -3,48 (q, 2H); 3,56 (breites s, 2H); 3,9 (breites s, 2H); 4,65 (d, 1 H); 4,96 (d, 1 H); 5,02 (d, 1 H); 5,62 (d, 1 H); 5,72 - 5,78 (q, 1 H); 7,27 (s, 2H); 7,51 (s, 2H); 7,64 (s, 1 H); 9,42 (d, 1 H); 9,82 (d, 1 H) ppm.

### Beispiel 3

### D-6-[α-(4-Ethyl-2,3-dioxo-1-piperazino-carbonylamino)-α-(2-aminobenzothiazol-6-yl)-glycylamido]-penicillansäure-Natrium

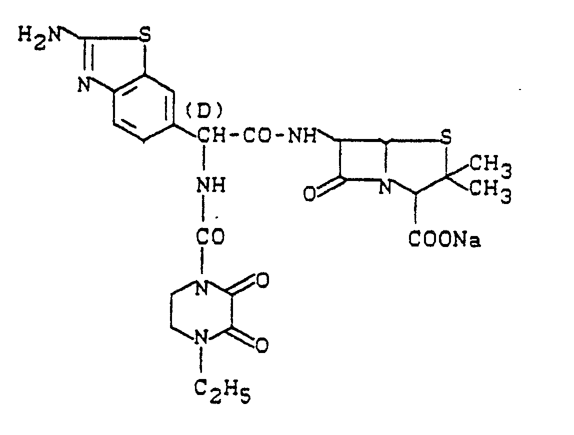

### a) D-α-[(4-Ethyl-2,3-dioxo-piperazin-1-yl)-carbonylamino]-α-(2-aminobenzothiazol-6-yl)essigsäure

Eine Suspension von 12 g (0,0538 mol) D-α-Amino-α-(2-aminobenzothiazol-6-yl)essigsäure in 100 ml Tetrahy drofuran (THF) und 40 ml Wasser wird durch allmähliche Zugabe von 1 N Natriumhydroxid bis zu einem pH von 10,5 in Lösung gebracht und auf +5°C bis +10°C gekühlt. In die Lösung werden 14,3 g (0,0699 mol) 4-Ethyl-2,3-dioxo-1-piperazinocarbonyl-chlorid gelöst in 80 ml Tetrahydrofuran eingetropft. Während dieser Zeit wird der pH der Reaktionslösung durch Zugabe von 2 N Natriumhydroxid auf pH 7,5 bis 8,0 gehalten. Anschließend wird noch 2H bei Raumtemperatur gerührt, der pH-Wert der Lösung auf 5,0 zurückgestellt und die Lösung über eine Säule -gefüllt mit Adsorberharz HP 20 - geschickt. Die Elution erfolgt mit Wasser und 50%igem Aceton. Das Eluat, das die gewünschte Verbindung enthält, wird lyophilisiert.

Ausbeute: 12,8 g (58,2% der Theorie)

C₁₆H₁₇N₅O₄S x H₂O (409,42)

NMR (DMSO): 5 = 1,08 (t, 3H); 3,32 - 3.44 (q, 2H); 3,56 (m, 2H); 3,9 (m, 2H); 5,32 (d, 1 H); 7,2 (dd, 1 H); 7,26 - 7,33 (t, 1 H); 7,55 (s, 2H); 7,66 (schwaches d, 1 H) ppm.

### b) D-6-[α-(4-Ethyl-2,3-dioxo-1-piperazinocarbonyl-amino]-α-(2-aminobenzothiazol-6-yl)glycylamido]-penicillansäure-Natrium (Mesylchlorid-Aktivierung)

2,5 g (0,0061 mol) 3a werden analog Beispiel 2a mit 1,06 ml (0,0061 mol) Ethyldiisopropylamin und 0,472 ml (0,0061 mol) Mesylchlorid bei -50 C in DMF 40 min. aktiviert und mit 3,47 g (0,00671 mol) D-6-(2-Aminobenzothiazol-6-glycylamido)penicillansäure-acetat-Dihydrat zur Umsetzung gebracht. C₂₄H₂₆N₇NaO₇S₂ x H₂0 (629,65)

Ausbeute: 1,6 g (41,7% der Theorie)

NMR (DMSO): δ = 1,1 (t, 3H); 1,50 (s, 3H); 1,54 (s, 3H); 3,25 -. 3,45 (q, 2H); 3,55 (m, 2H); 3,9 (m, 2H); 4,18 (s, 1 H); 5,38 (d, 1 H); 5,54 (q, 1 H); 5,71 (d, 1 H); 7,26 (t, 1 H); 7,51 (s, 1 H); 7,65 (s, 1 H); 9,27 (d, 1 H); 9,84 (d, 1 H) ppm.

### c) D-6-[α-(4-Ethyl-2,3-dioxo-1-piperazinocarbonyl-amino)-α-(2-aminobenzothiazol-6-yl)glycylamido]-penicillansäure-Natrium (Säurechlorid-Verfahren)

0,220 mg (0,425 mmol) D-6-(2-Aminobenzothiazol-6-yl-glycylamido)-penicillansäure als Acetat-dihydrat (MM = 517,59) werden in 10 ml 80%igem wäßrigen Tetrahydrofuran suspendiert und mittels einer 10%igen Lösung von Triethylamin in Tetrahydrofuran bei pH 8,5 in Lösung gebracht. 0,174 g (0,85 mmol) 4-Ethyl-2,3-dioxo-1-piperazinocarbonyl-chlorid gelöst in 2 ml Tetrahydrofuran werden bei 0°C bis +5°C innerhalb von 20 min. zugegeben, wobei der pH bei 7,5 durch entsprechende Zugabe von Triethylamin in Tetrahydrofuran gehalten wird. Danach wird 90 min. bei pH 7,5 nachgerührt, anschließend 100 ml Wasser hinzugegeben und Tetrahydrofuran im Vakuum weitgehend abdestilliert. Die verbleibende wäßrige Lösung wird lyophilisiert. Man erhält 500 mg Lyophilisat, die in 6 ml Wasser ge löst, über ein Ultra-Filter filtriert und anschließend an einer präparativen HPLC-Säule (Hibar 250-25, RP 18, 7 u.m) mittels 5%igem Acetonitril in Wasser chromatographiert werden.

Ausbeute: 165 mg
Das gesamte Material wird anschließend zur Entfernung des Triethylamins in 50 ml Wasser durch Zugabe von Natriumhydrogencarbonat-Lösung gelöst, auf eine Merck-Säule (Hibar 250-25, RP 18, 7 um) gepumpt und dann mit 100 ml 0,1 N Salzsäure und 700 ml Wasser eluiert. Anschließend wird die Säule umgedreht und die Substanz mit 30%igem Acetonitril von der Säule gespült.
Ausbeute: 120 mg (40,5% der Theorie)
C₂₄H₂₇N₇O₇S₂ x 6H₂O (697,752)
HPLC-Gehalt: 96,6%, Hibar 250-4, RP-8, 10 um,
Laufmittel: 0,05 molare Ammoniumacetat-Lösung (1000 ml) / 100 ml Acetonitril,
254 nm, 3 ml/min.
Retention: 9,88
NMR (DMSO): 5 = ₁,₁ (t, 3H); 1,49 (s, 3H); 1,51 (s, 3H); 3,25 - 3,42 (q, 2H); 3,55 (m, 2H); 3,9 (m, 2H); 4,18 (s, 1 H); 5,38 (d, 1 H); 5,52 (q, 1 H); 5,7 (d, 1 H); 7,28 (5, 1 H); 7,51 (s, 1 H); 7,65 (s, 1 H); 9,27 (d, 1 H); 9,83 (d, 1 H) ppm.

### Beispiel 4

### D-6-[α-(2-Oxo-3-furylidenamino-imidazolidin-1-ylcarbonylamino)-α-(2-aminobenzothiazol-6-yl)-glycylamido]-penicillansäure-Natrium

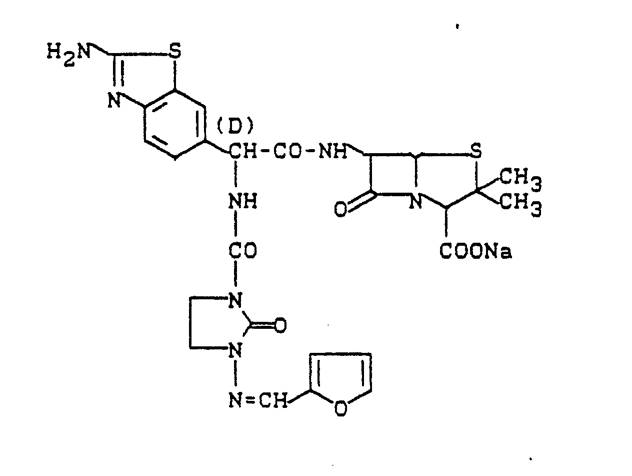

### a) D-α-[(2-Oxo-3-furylidenamino-imidazolin-1-yl)carbonylamino]-α-(2-aminobenzothiazol-6-yl)essigsäure

Eine Suspension von 20 g (0,0896 mol) D-α-Amino-α-(2-aminobenzothiazol-6-yl)-essigsäure in 200 ml Tetrahydrofuran und 120 ml Wasser wird durch allmähliche Zugabe von 2 N Natriumhydroxid bis zu einem pH von 8,0 in Lösung gebracht. Anschließend werden 23,8 g (0,0986 mol) 1-Chlorcarbonyl-2-oxo-3-furylidenaminoimidazolidin portionsweise in die Lösung bei +5°C eingetragen, wobei gleichzeitig der pH-Wert zwischen 7,5 bis 7,8 durch Zugabe von 1 N Natriumhydroxid aufrecht gehalten wird. Man rührt ohne Kühlung ca. 2 h nach und gibt während des Nachrührens 80 ml Tetrahydrofuran und 80 ml Wasser in kleinen Portionen hinzu. Es entsteht allmählich eine klare Lösung, die mit 200 ml Wasser verdünnt und im Vakuum Tetrahydrofuran entfernt wird. Die zurückbleibende Lösung wird einmal mit Essigester extrahiert und die wäßrige Phase bei 0°C mit 2 N Salzsäure auf pH 2,0 angesäuert. Das ausgefallene Produkt wird abgesaugt, mit Wasser gewaschen und im Vakuum getrocknet.
Ausbeute: 24,1 g (55,8% der Theorie)
C₁₈H₁₆N₆O₅Sx3H₂O (482,474)
Ber.: C 44,81 H 4,60 N 17,42 S 6,64
Gef.: C 43,7 H 3,9 N 17,1 S 7,1
NMR (DMSO): S = 3,78 (breites s, 4H); 5,38 (d, 1 H); 6,66 (dd, 1 H); 6,85 (d, 1 H); 7,35 (dd, 1 H); 7,45 - 7,52 (t, 1 H); 7,75 (s, 1 H); 7,83 (s, 2H); 8,85 (breites s, 2H); 9,08 (d, 1 H) ppm.

### b) D-6-[α-(2-Oxo-3-furylidenamino-imidazolidin-1-ylcarbonylamino)-α-(2-aminobenzothiazol-6-yl)-glycylamido]penicillansäure-Natrium

### Aktivierung der Precursorsäure

9,29 g (0,02 mol) 4a (Dihydrat) werden in 40 ml Dimethylformamid gelöst, mit 2,8 ml (0,02 mol) Triethylamin versetzt, bei -50°C 4 Tropfen 3-Dimethylamino-1-propanol und 1,92 ml (0,02 mol) Chlorameisensäureethylester zugegeben und 15 min. bei -40° C gerührt.

### Zubereitung der Aminkomponente:

4,54 g (0,021 mol) 6-Aminopenicillansäure (6-APS) werden in 15 ml Wasser suspendiert und durch tropfen weise Zugabe von 2 N Natriumhydroxid in Lösung gebracht. Die bei Raumtemperatur entstandene Lösung wird auf -10 C gekühlt und mit 20 ml Aceton verdünnt.

### Kupplung und Isolierung

Die gekühle Lösung der 6-Aminopenicillansäure gibt man in einem Guß zu der Lösung des gemischten Anhydrids der Percursorsäure (a) bei -50° C und rührt dann ohne Kühlung 20 min., wobei die Temperatur der Reaktionslösung allmählich bis auf +5°C ansteigt. Die Reaktionslösung wird dann in 400 ml Eiswasser eingerührt, der pH-Wert auf 2,0 eingestellt, das ausgefallene Produkt abgesaugt, mit HzO gewaschen und über Nacht im Vakuum über Phosphorpentoxid getrocknet.
Rohausbeute: 10,5 g
Das Material wird in 50 ml 5%igen Acetonitril unter Zusatz von 60 ml gesättigter Natriumhydrogencarbonat-Lösung (pH 8,6) gelöst, über Kieselgur filtriert und an einer Whatman-Säuie (Magnum M 40, 500 x 40, Merck-Füllmaterial RP 18, 40-68 um) mit Wasser unter Zusatz von Acetonitril mit steigendem Gradient von 5% bis 10% eluiert (Fluß 40 ml/min).
Ausbeute: 3,3 g (22,9% der Theorie)
C₂₆H₂₅N₈NaO₇S₂ x 4H₂O (720,723)
Ber.: C 43,33 H 4,61 N 15,55 S 8,89 Na 3,18
Gef.: C 43,5 H 4,7 N 15,6 S 8,7 Na 3,0
NMR (DMSO): δ = 1,40 (s, 3H); 1,52 (s, 3H); 3,68 -3,87 (m, 4H); 3,9 (s, 1 H); 5,3 (d, 1 H); 5,4 - 5,46 (q, 1 H); 5,74 (d, 1 H); 6,62 (d, 1 H); 6,85 (d, 1 H); 7,29 (t, 2H); 7,53 (s, 2H); 7,65 (s, 1 H); 7,73 (s, 1 H); 7,83 (s, 1 H); 9,08 (d, 1 H); 9,19 (d, 1 H) ppm.
HPLC-Gehalt: 92.4%, Hibar 250-4, RP 8, 10 um,
Laufmittel: 800 ml H₂O/200 ml CH₃CN/3,84 g CH₃C00NH₄.
254 nm, 4 ml/min.
Retention: 3,69

### Beispiel 5

### D-6-[α-(Imidazolidin-2-on-1-ylcarbonylamino)-α-(2-aminobenzothiazöl-6-yl)glycylamido]-penicillansäure-Natrium

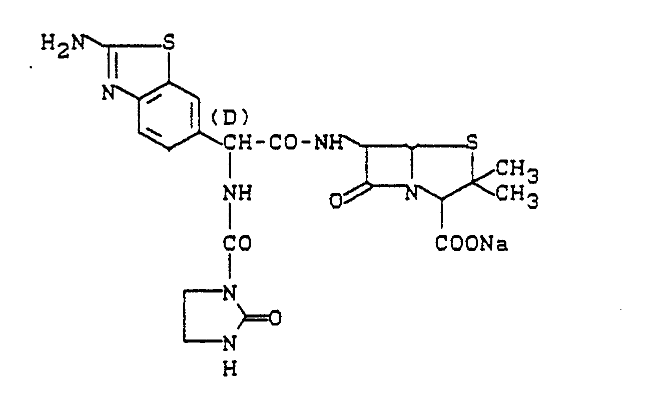

9,4 g (0,0223 mol) D-6-(2-Aminobenzothiazol-6-yl-glycylamido)-penicillansäure werden in 110 ml 80%igem wäßri gem Tetrahydrofuran suspendiert und tropenweise unter Rühren bei +20°C mit soviel Triethylamin versetzt, daß eben eine klare Lösung entsteht und der pH-Wert sich zwischen 7,5 und 8,2 befindet. Man kühlt auf 0^{*} C und tropft 3,3 g (0,0223 mol) N-Chlorcarbonylimidazolidin-2-on gelöst in 20 ml Tetrahydrofuran unter Eiskühlung und heftigem Rühren innerhalb von 30 min. zu, wobei durch gleichzeitig erfolgende Zugabe von Triethylamin der pH-Wert zwischen 7,5 und 8,0 gehalten wird. Man rührt 30 min. bei 0 C und anschließend solange bei Raumtemperatur nach, bis keine Zugabe von Triethylamin zur Aufrechterhaltung des pH-Wertes von 7,5 mehr erforderlich ist. Es werden 100 ml Wasser hinzugefügt und Tetrahydrofuran im Vakuum abdestilliert. Die verbleibende wäßrige Lösung wäscht man einmal mit Ether, überschichtet mit 300 ml Essigester, kühlt auf 0°C und säuert unter Rühren und Eiskühlung mit 1 N Salzsäure auf pH 1,5 - 2,0 an. Man trennt die organische Phase ab, extrahiert das Filtrat nochmals mit 200 ml Essigester und vereinigt die organischen Extrakte. Nach dem Trocknen über Natriumsulfat gibt man 22 ml einer 1-molaren Lösung von Natrium-2-ethyl-hexanoat in methanolhaltigem Ether hinzu, engt die Lösung fast bis zur Trockene ein, nimmt die Restlösung in 25 ml Methanol auf und versetzt mit 400 ml Ether. Das ausgefallene Produkt wird abgesaugt, mit Ether nachgewaschen und getrocknet.
Ausbeute: 6,3 g (47,8% der Theorie)
C₂₁H₂₂N₇NaO₆S₂ x 2H₂0 (591,604)
Ber.: C 42,64 H 4,43 N 16,57 S 10,47
Gef.: C 41,9 H 4,22 N 16,1 S 9,9
NMR (DMSO):S = 1,41 (s, 3H); 1,54 (s, 3H); 3,68 - 3,87 (m, 4H); 4,0 (s, 1 H); 5,3 (d, 1 H); 5,4 -5,46 (q, 1 H); 5,72 (d, 1 H); 7,29 (t, 2H); 7,68 (s, 1 H); 9,08 (d, 1 H); 9,19 (d, 1 H) ppm.

### Beispiel 6

D-6-[α-(3-Methylsulfonyl-imidazolidin-2-on-1-ylcarbonylamino)-α-(2-aminobenzothiazol-6-yl)glycylamido]-penicillansäure-Natrium

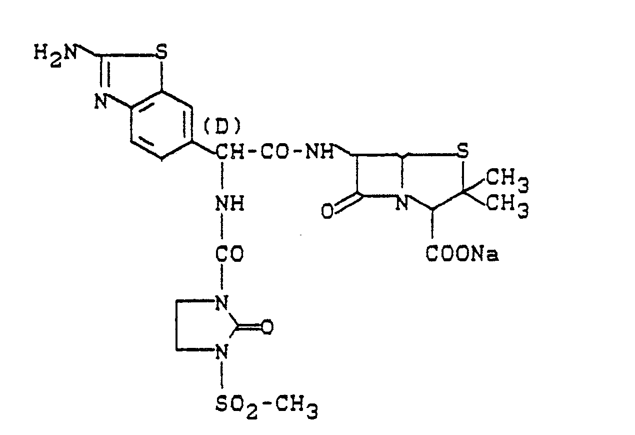

3,5 g (0,0083 mol) D-6-(2-Aminobenzothiazol-6-ylglycylamido)penicillansäure werden in 50 ml 80%igem Tetrahydrofuran suspendiert, mittels der eben notwendigen Menge Triethylamin in Lösung gebracht (pH-Wert 7,8). 1,94 g (0,0096 mol) 1-Chlorcarbonyl-3-methylsulfonyl imidazolidin-2-on fügt man unter Eiskühlung portionsweise hinzu und hält durch gleichzeitig erfolgende Zugabe von 10%igem Triethylamin in Tetrahydrofuran den pH-Wert von 7,5 aufrecht. Anschließend wird ca. 2 h bei einem pH-Bereich von 7,0 -7,5 nachgerührt. Man verdünnt mit 150 ml H₂0, stellt den pH auf 6,5 ein, destilliert Tetrahydrofuran im Vakuum weitgehend ab und wäscht die verbleibende wäßrige Lösung einmal mit Ether.

Das wäßrige Filtrat wird mit Essigester überschichtet und bei 0°C bis 5°C mit 1 N Salzsäure auf pH 2 angesäuert. Die organische Phase wird abgetrennt, mit Kochsalzlösung gewaschen, über Natriumsulfat getrocknet und danach analog Beispiel 5b mittels 1-molarer Natrium-2-ethylhexanoat-Lösung in methanolhaltigem Ether das Natriumsalz isoliert.
Ausbeute: 2,3 g (41,4% der Theorie)
C₂₂H₂₄N₇NaO₈S₃ x 2H₂0 (669,69)
Ber.: C 39,46 H 4,21 N 14,64 Na 3,43 S 14,36
Gef.: C 38,8 H 4,4 N 14,1 Na 3,6 S 13,9

### Beispiel 7

### 7β-{D-α-[(4-Ethyl-2,3-dioxopiperazin-1-yl)carbonylaminol]-α-(2-amino-1 H-benzimidazol-5-ylglycylamido}-7α-formamido-3-(pyridiniomethyi)-3-cephem-4-carbonsäure

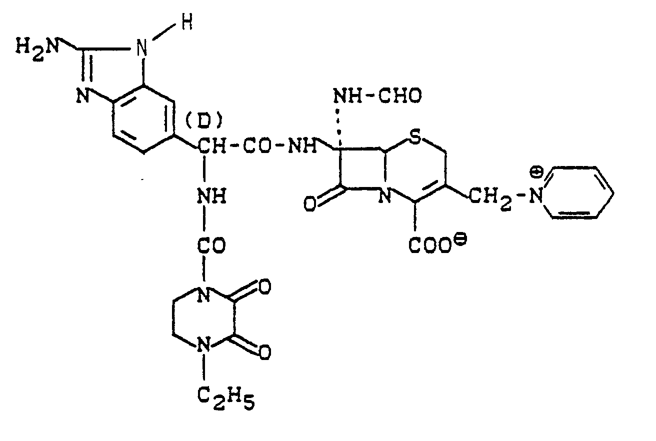

550 mg (1,05 mmol) 7α-Formamido-7β-[D-2-(amino-1H-benzimidazol-5-yl)glycylamido]-3-(pyridiniomethyl)-3-cephem-4-carbonsäure werden analog Beispiellb mit 258 mg (1,26 mmol) 4-Ethyl-2,3-dioxo-1-piperazino-carbonylchlorid umgesetzt. Nach Chromatographie an Adsorberharz HP 20 AG (Dianion, Mitsubishi) mit Wasser/Aceton ( 1:0 - 10:1) erhält man 150 mg Betain.
C₃₀H₃₀N₁₀O₈S x 2H₂0 (762,72)
Ber.: C 49,58 H 4,72 N 19,27 S 4,41
Gef.: C 48.9 H 4,6 N 18,7 S 4,2

### Beispiel 8

### 7β-{D-α-(4-Ethyl-2,3-dioxopiperazin-1-yl)carbonylamino]-α-(2-aminobenzoxazol-5-ylglycylamido}-7-α-formamido-3-[(1,3,4-fhiadiazol-2-yl)thiomethyl]-3-cephem-4-carbonsäure

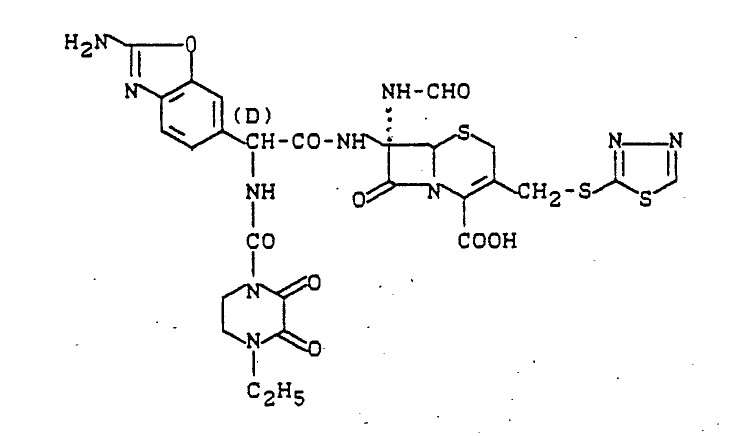

0,720 g (1,92 mmol) D-α[(4-Ethyl-2,3-dioxopiperazin-1-yl)carbonylamino[-α-(2-aminobenzoxazol-5-yl)-essigsäure werden analog Beispiel 2a mit 0,334 ml (1,92 mmol) Ethyldiisopropylamin und 0,149 ml (1,92 mmol) Mesylchlorid 40 min. bei -50 C behandelt und anschließend mit 0,788 g (2,11 mmoi) 7β-Amino-7-α-formamido-3-[(1.3.4-thiadiazol-2-yl)thiomethyl]-3-cephem-4-carbonsäure umgesetzt. Das Rohbetain wird an Adsorberharz OC 1062 (Lewatit BAYER AG) mit dem Laufmittelsystem: Wasser/Aceton (1:1 → 4:1) chromatographiert. Das Eluat, das die gewünschte Verbindung enthält, wird von Aceton befreit, mit 1 N Natriumhydroxid auf pH 7,8 gestellt und die Lösung lyophilisiert.
Ausbeute: 310 mg (21,4% der Theorie)
C₂₇H₂₅N₁₀NaO₉S₂ x 2H₂O (756,714)
Ber.: C 42,86 H 3,86 N 18,51 Na 3,04 S 8,47
Gef.: C 41,9 H 3,5 N 18,2 Na 3,2 S 8,2

### Beispiel 9

### D-7-[α-(4-Ethyl-2,3-dioxo-1-piperazino-carbonylamino)-α-(benzofur-5-yl)glycyl-amido]-3-{[(1,2,5,6-tetrahydro-2-methyl-5,6-dioxo-as-triazin-3-yl)-thio]methyl}3-cephem-4-carbonsäure-Dinatrium

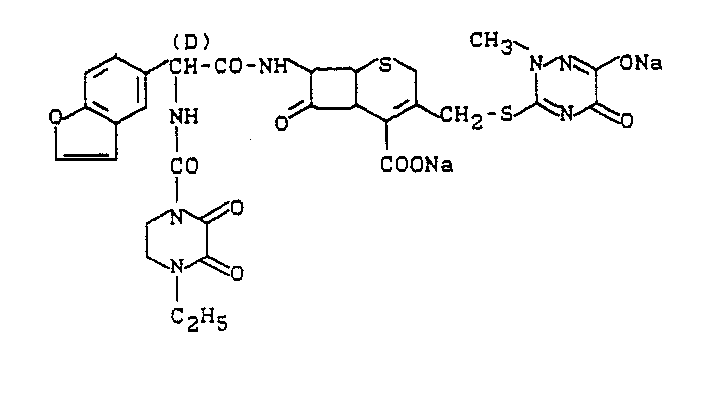

### a) Zubereitung der Precursorsäure

790 mg (2,2 mmol) DL-α-[(4-Ethyl-2,3-dioxo-1-piperazino)-carbonylamino]-α-(benzofur-5-yl)essigsäure werden in 15 ml Dimethylformamid und 5 ml Acetonitril unter Zusatz von 0,31 ml (2.2 mmol) Triethylamin gelöst. Dann kühlt man die Lösung auf -50° C und gibt nacheinander 1 Tropfen 3-Dimethylamino-1-propanol und 0,21 ml (2,2 mmol) Chlorameisensäureethylester hinzu und rührt 30 Minuten bei -50° C.

### b) Zubereitung der Aminkomponente:

817 mg (2,2 mmol) 7-Amino-3-{[1.2.5.6-tetra-hydro-2-methyl-5.6-dioxo-as-triazin-3-yl)-thio]methyl}-3-cephem-4-carbonsäure werden in 10 ml Wasser, 10 ml Dimethylformamid und 4 ml Acetonitril suspendiert und durch Zugabe von 1 N Natronlauge bis pH 7,5 eingestellt, wobei eine klare Lösung entsteht.

### c) Kupplung und Isolierung:

Nach Zugabe der Aminkomponente b) zu der Lösung des gemischten Anhydrids a) bei -50° C läßt man anschließend die Temperatur der Lösung innerhalb von 30 Min. auf 0 C ansteigen (ohne Kältebad).

Danach wird die Lösung teilweise eingeengt, mit ca. 100 ml Wasser und 300 ml Essigester verdünnt, der pH-Wert auf 2,5 eingestellt und die Essigesterphase abgetrennt. Nach Waschen der organischen Phase mit Kochsalzlösung, Trocknen über Natriumsulfat und Einengen bis zur Trockene wird das Rohmaterial an einer präparativen Säule (Hibar 250-25, RP 18, 7 um) unter Verwendung des Laufmittelsystems Wasser_acetonitril-Eisessig (siehe Beispiel 2 b) gereinigt. Das Elual, das diese gewünschte Verbindung enthält, wird von organischem Lösungsmittel befreit mit 1 N Natronlauge auf pH 7.5 gestellt und diese Lösung lyophilisiert..
Ausbeute: 178 mg (10 % der Theorie)
C₂₉H₂₆N₈Na₂O₁₀S₂.3H₂O (810,7)
Ber. : C 42,96 H 3,99 N 13,82 S 7,91
Gef. : C 41,2 H 3,5 N 13,62 S 6,89
IR-Spektrum (Nujol): 1770, 1730, 1660 cm⁻¹

### Beispiel 10

### D-7-[α-(4-Ethyl-2,3-dioxo-1-piperazino-carbonylamino)-α-(2-aminobenzothiazol-6-yl)-glycylamido]-3-{[-(1,2,5,6-tetrahydro-2-methyl-5,6-dioxo-as-triazin-3-yl)-thio]methyl}3-cephem-4-carbonsäure-dinatrium

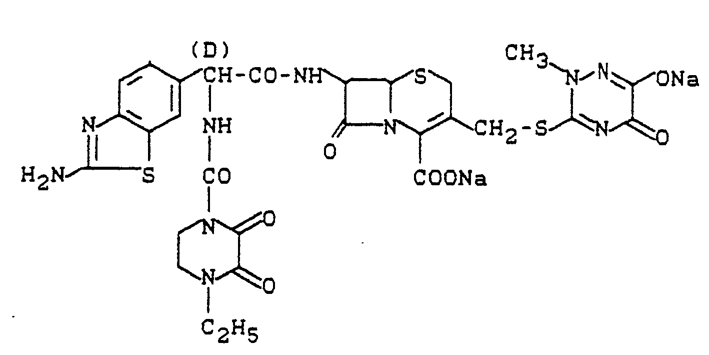

1,25 g (3,1 mmol) D-a-[(4-Ethyl-2,3-dioxo-piperazin-1-yl)carbonylamino]-a-(2-aminobenzothiazol-6-yl)-essigsäure-monohydrat werden analog Beispiel 9 mit 0,434 ml (3,1 mmol) Triethylamin und 0,298 mi (3,1 mmol) Chlorameisensäureethylester bei -50° C aktiviert und anschließend mit 1,15 g (3,1 mmol) 7-Amino-3- {[(1,2,5,6-tetra hydro-2-methyl-5,6-dioxo-as-triazin-3-yl-thio]methyl}-3-cephem-4-carbonsäure. Das Rohprodukt, das in Anlehnung an Beispiel 9 isoliert wird, wird in Wasser unter Zuatz von Ammoniumhydrogencarbonat gelöst und an Adsorberharz L PG 4429 (Lewatit® OC 1062) in wäßrigem Aceton chromatographiert. Ausbeute: 0,95 g (38 % der Theorie).
C₂₈H₂₆N₁₀Na₂O₉S₃.2H₂O (824,78)
Ber. : C 40,78 H 3,67 N 16,98 S 11,66
Gef. : C 41,2 H 3,15 N 15,8 S 11,2
IR-Spektrum (KBr): 1775, 1740, 1695, 1670 cm⁻¹

### Beispiel 11

### D-7-[α-(4-Ethyl-2,3-dioxo-1-piperazino-carbonylamino)-α-(indol-5-yl)-glycylamido]-3-(1-methyl-1H-tetrazol-5-yl-thiomethyl)-3-cephem-4-carbonsäure-Natrium

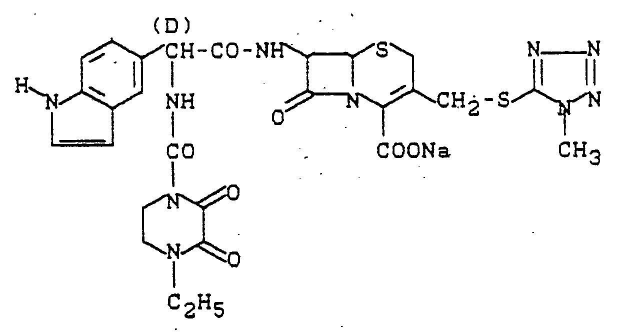

0,86 g (2,4 mmol) D-α-((4-Ethyl-2,3-diaxo-piperazin-1-yl)carbonylamino]-α-(indol-5-yl)essigsäure werden analog Beispiel 9 mit 0,336 ml (2,4 mmol) Triethylamin und 0,230 ml (2,4 mmol) Chlorameisensäureethylester bei -50° C in Dimethylformamid/Acetonitril 30 Min. aktiviert und danach 0,923 g (2,4 mmol) 7-Amino-3-(1-methyl-1 H-tetrazol-5-yl-thiomethyl)-3-cephem-4-carbonsäure-t-butylester gelöst in 8 ml Tetrahydrofuran bei -50° C zum gemischten Anhydrid hinzugegeben.

Nach Kupplung und Isolierung des rohen Cephalosporinderivates erhält man schließlich durch Adsorberharz-Chromatographie an OC 1062 (siehe Beispiel 10) das reine Endprodukt.
Ausbeute: 0,69 g (39,7 % der Theorie)
C₂₇H₂₇N₁₀Na O₇S₂·2H₂O (726,73)
Ber. : C 44,62 H 4,29 N 19,27 S 8,82
Gef. : C 43,8 H 4,05 N 18,90 S 8,60
IR-Spektrum (Nujol): 1780, 1715, 1685-1675 cm⁻¹

### Beispiel 12

### D-7-[α-(4-Ethyl-2,3-dioxo-1-piperazino-carbonylamino)-α-(2-aminobenzoxazol-5-yl)-glycylamido]-3-(1-methyl-1H-tetrazol-5-yl-thiomethyl)-3-cephem-4-carbonsäure-Natrium

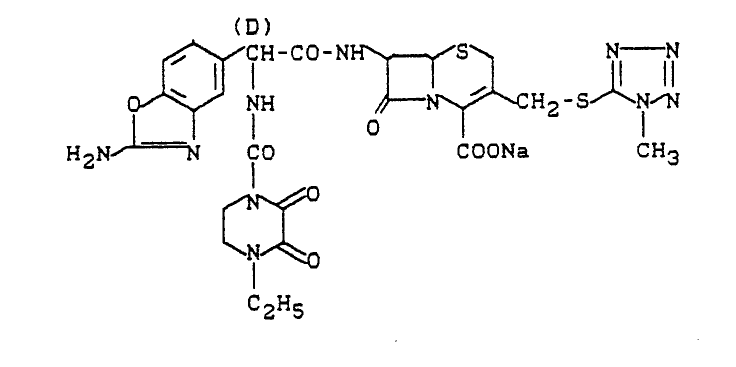

2,1 (5,59 mmol) DL-α-[4-Ethyl-2,3-dioxo-piperazin-1-yl)carbonylamino]-α-(2-aminobenzoxazol-5-yl)-essigsäure werden analog Beispiel 9 mit 0,783 ml (5,59 mmol) Triethylamin und 0,537 ml (5,59 mmol) Chlorameisensäureethylester bei -50 C in Dimethylformamid/Acetonitril 30 Min. lang aktiviert und anschließend mit 1,84 g (5,59 mmol) 7-Amino-3-(1-methyl-1H-tetrazol-5-yl-thiomethyl)-3-cephem-4-carbonsäure umgesetzt, die in 15 ml Wasser, 15 ml Dimethylforamid und 8 ml Acetonitril unter Zusatz von 1 N Natronlauge in Lösung gebracht werden.

Nach Kupplung und Isolierung wird das Rohbetain an Adsorberharz OC 1062 mit dem Laufmittelsystem Wasser/Aceton chromatographiert (siehe Beispiel 8).
Ausbeute: 0,85 g (20,7 % der Theorie)
C₂₆H₂₆N₁₁NaO₈S₂·2H₂O (743,7)
Ber. : C 41,99 H 4,07 N 20,72 S 8,62
Gef. : C 41,51 H 3,55 N 19,90 S 8,40
IR-Spektrum (Nujoi): 1780, 1720-1650 cm-'

### Beispiel 13

### D-7-[α-(4-Ethyl-2,3-dioxo-1-piperazino-carbonylamino)-α-(20-amino-1H-benzimidazol-5-yl)-glycylamido]-3-(1-methyl-1 H-tetrazol-5-yl-thio-methyl)-3-cephem-4-carbonsäure-Natrium

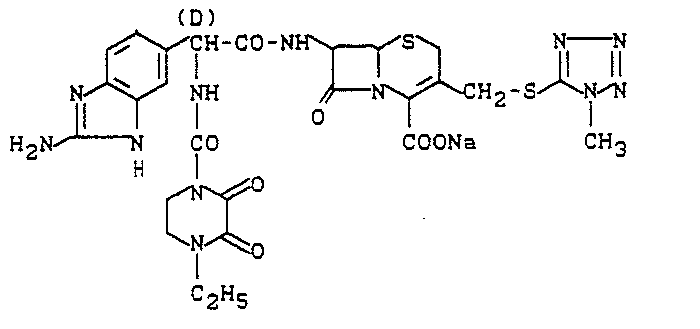

1,32 g(3,53 mmol) DL-α-[(4-Ethyl-2,3-dioxo-piperazin-1-yl)-carbonylamino]-α-(2-amino-1H-benzimidazol-5-yl)-essigsäure werden analog Beispiel 9 mit 0,494 ml (3,53 mmol) Triethylamin und 0,339 ml (3,53 mmol) Chlorameisensäureethylester bei -50 °C in DimethylformamidiAcetonitril 30 Min. aktiviert und anschließend mit 1,36 g (3,53 mmol) 7-Amino-3-(1-methyl-1H-tetrazol-5-yl-thiomethyl)-3-cephem-4-carbonsäure-t-butylester, gelöst in 20 ml Tetrahydrofuran, bei -50 °C umgesetzt. Nach Kupplung und Isolierung des Rohproduktes analog Beispiel 1 erhält man mit Hilfe der präparativen HPLC-Trennung an RP-18 das reine D-isomere Cephalosporinderivat.
Ausbeute: 0,62 g (23,7 % der Theorie)
C₂₆H₂₆N₁₂NaO₇S₂·2H₂O (741,72)
Ber. : C 42,10 H 4,08 N 22,66 S 8,65
Gef. : C 41,54 H 3,75 N 22,40 S 8,41
IR-Spektrum (KBr): 1775, 1720-1660 cm-'

### Beispiel 14

### 7β-{D-α-[(4-Ethyl-2,3-dioxo-piperazin-1-yl)-carbonylamino]-α-(2-methylbenzothiazol-6-yl]glycylamido}-7α-formamido-3-(pyridiniomethyl)-3-cephem-4-carbonsäure

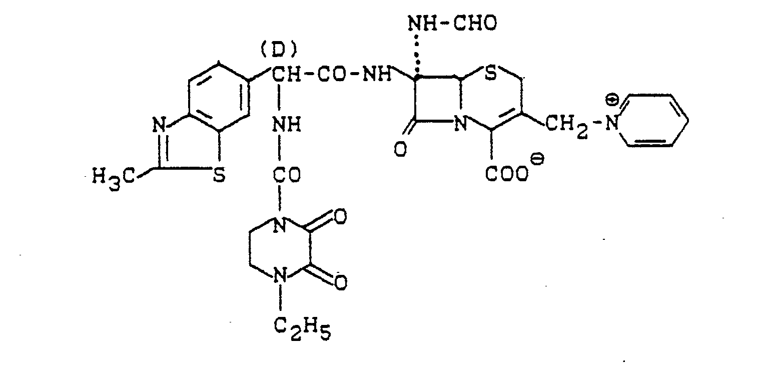

1,83 g (4,69 mmol) DL-α-[(4-Ethyl-2,3-dioxo-piperazin-1-yl)-carbonylamino]-α-(2-methylbenzothiazol-6-yl)-essigsäure werden analog Beispiel 9 mit 0,657 ml (4,69 mmol) Triethylamin und 0,450 ml (4,69 mmol) Chlorameisensäureethylester bei -50 °C in Dimethylformamid/Acetonitril 30 Min. aktiviert und anschließend mit 1,57 g (4,69 mmol) 7α-Formamido-7β-amino-3-(pyridiniomethyl)-3-cephem-4-carbonsäure umgesetzt, die in 10 ml Wasser, 15 ml Dimethylformamid und 8 ml AcetonitriJ unter Zusatz von 1 N Natronlauge in Lösung gebracht werden. Nach Kupplung und Isolierung wird aus dem Rohmaterial durch präparative HPLC-Trennung an RP-18 in Anlehnung an Beispiel 1 das D-Isomer erhalten.
Ausbeute: 0,81 g (23,8 % der Theorie)
C₃₁H₃₀N₈O₈S₂·H₂O (724,78)
Ber.: C 50,55 H 4,52 N 15,72 S 9,0
Gef.: C 49,62 H 4,2 N 15,45 S 8,55
IR-Spektrum (KBr): 1770, 1730, 1660-1630 cm⁻¹.

## Patentansprüche

1. Phenylglycin-β-Lactam-Antibiotika der allgemeinen Formel (I) 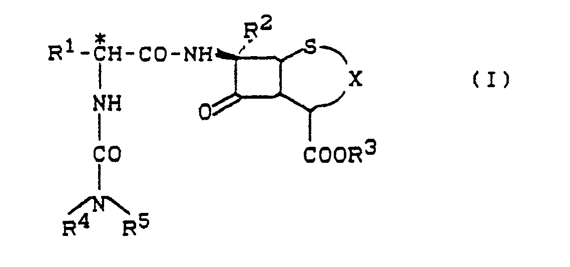 in welcher
R¹ - für einen Rest der Formel 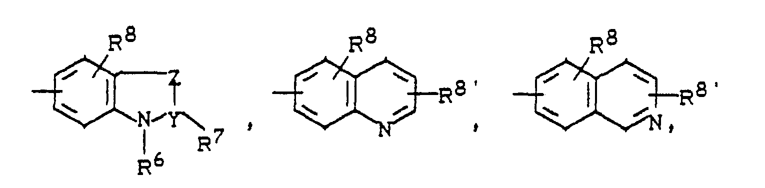 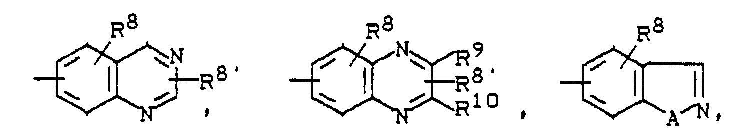 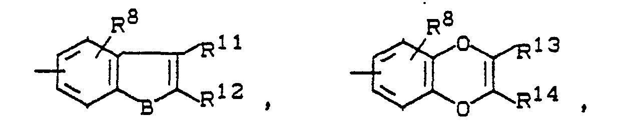 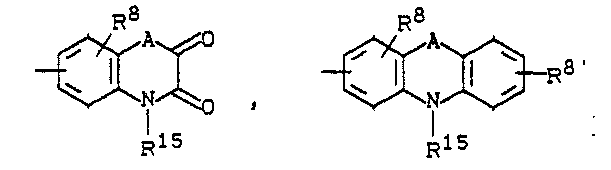 oder 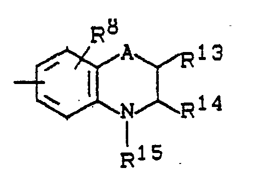 steht, worin
Y - für N oder CR¹⁶ steht,
oder die Gruppierung
Y-R^{7 -} für >= 0 oder >=N-R⁷ steht,
Z - für 0, S oder -NR¹⁷ steht,
A - für 0, S oder -NR¹⁸ steht,
B - Für 0 oder -NR¹⁵ steht,
_{R}^{s} - f_{ü}r Wasserstoff oder
- für Hydroxy oder Amino steht, oder
- für geradkettiges, verzweigtes oder cyclisches, gegebenenfalls durch Halogen, gegebenenfalls substituiertes Amino, Hydroxy, Cyano oder C₆-C₁₀-Aryl substituiertes Alkyl oder Alkenyl mit bis zu 10 Kohlenstoffatomen steht, oder
- für gegebenenfalls substituiertes C₆-C₁₀-Aryl steht,
R^{7 -} für Wasserstoff steht, oder
- für gegebenenfalls substituiertes C₆-C₁₀-Aryl steht, oder
- für geradkettiges, verzweigtes oder cyclisches Alkyl oder Alkenyl mit bis zu 10 Kohlenstoffatomen steht, das gegebenenfalls substituiert ist durch Halogen, Hydroxy, Alkoxy oder Alkoxycarbonyl mit jeweils bis zu 6 Kohlenstoffatomen, Cyano, Carboxy, gegebenenfalls substituiertes Cs-Cio-Aryl, Sulfo oder durch eine gegebenenfalls substituierte Aminogruppe,
oder
R⁶ und R⁷ gemeinsam eine Doppelbindung vervollständigen,
R^{s}, R^{8'} gleich oder verschieden sind und
- für Wasserstoff stehen, oder
- für geradkettiges, verzweigtes oder cyclisches Alkyl, Alkoxy oder Alkylthio mit jeweils bis zu 8 Kohlenstoffatomen stehen, oder
- für Trifluormethyl oder Trifluormethoxy stehen, oder
- für Hydroxy, Mercapto, Nitro, Cyano oder Halogen stehen, oder
- für eine gegebenenfalls substituierte Aminogruppe stehen,
R⁹, R¹⁰ gleich oder verschieden sind und
- für Wasserstoff stehen, oder
- für gegebenenfalls substituiertes C₆-C₁₀-Aryl stehen, oder
- für eine gegebenenfalls substituierte Aminogruppe stehen, oder
- für Hydroxy stehen, oder
- für geradkettiges, verzweigtes oder cyclisches Alkoxy mit bis zu 8 Kohlenstoffatomen stehen, oder
- für Acyl oder Acyloxy mit jeweils bis zu 7 Kohlenstoffatomen stehen, oder
- für geradkettiges, verzweigtes oder cyclisches, gegebenenfalls substituiertes Alkyl mit bis zu 12 Kohlenstoffatomen stehen,
R", R¹² gleich oder verschieden sind und
- für Wasserstoff stehen, oder
- für gegebenenfalls substituiertes C₆-C₁₀-Aryl stehen, oder
- für Heterocyclyl stehen, oder
- für Hydroxy stehen, oder
- für eine gegebenenfalls substituierte Aminogruppe stehen, oder
- für geradkettiges, verzweigtes oder cyclisches Alkoxy mit bis zu 8 Kohlenstoffatomen, oder
- für Acyl oder Acyloxy mit jeweils bis zu 8 Kohlenstoffatomen stehen, oder
- für Alkoxycarbonyl mit bis zu 8 Kohlenstoffatomen stehen, oder
- für gegebenenfalls substituiertes geradkettiges, verzweigtes oder cyclisches Alkyl mit bis zu 12 Kohlenstoffatomen stehen,
oder
R" und R¹² zusammen für die Gruppierung der Formel 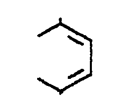 stehen,
R'³, R¹⁴ gleich oder verschieden sind und
- für Wasserstoff stehen, oder
- für gegebenenfalls substituiertes geradkettiges, verzweigtes oder cyclisches Alkyl mit bis zu 12 Kohlenstoffatomen stehen, oder
- für gegebenenfalls substituiertes C₆-C₁₀-Aryl stehen, oder
- für Alkoxycarbonyl mit bis zu 8 Kohlenstoffatomen stehen,
R¹⁵ für Wasserstoff, oder
- für gegebenenfalls substituiertes C₆-C₁₀-Aryl steht, oder
- für geradkettiges, verzweigtes oder cyclisches Alkyl mit bis zu 8 Kohlenstoffatomen steht,
R¹⁶ die gleiche Bedeutung hat wie R⁷ und außerdem
- für Halogen, oder
- für geradkettiges, verzweigtes oder cyclisches Alkoxy oder Alkylthio mit jeweils bis zu 8 Kohlenstoffatomen steht, oder
- für eine gegebenenfalls substituierte Aminogruppe steht, oder
- für geradkettiges, verzweigtes oder cyclisches Alkylsulfonyl mit bis zu 8 Kohlenstoffatomen steht, oder
- für Phosphono, Sulfo oder Sulfamoyl steht, oder
- für Mercapto, Hydroxy, Phenylthio oder Phenoxy steht, oder
- für Guanidino, Amidino, Hydrazino oder Hydroxylamino steht, oder
- für gegebenenfalls substituiertes Heterocyclyl steht, oder
- für gegebenenfalls substituiertes Heterocyclyloxy oder Heterocyclylthio steht,
R¹⁷ die gleiche Bedeutung hat wie R¹⁶, jedoch nicht mit R⁷ eine Doppelbindung vervollständigt,
oder
R¹⁶ und R¹⁷ gemeinsam für eine gegebenenfalls durch Sauerstoff oder Schwefel unterbrochene C₂-C₄-Methylenkette steht
und
R¹⁸ die gleiche Bedeutung hat wie R¹⁵ und mit diesem gleich oder verschieden sein kann,
R^{2 -} für Wasserstoff, oder
- für geradkettiges, verzweigtes oder cyclisches Alkoxy oder Alkylthio mit jeweils bis zu 5 Kohlenstoffatomen steht, oder
- für eine gegebenenfalls substituierte Aminogruppe steht, oder
- für Formamido steht,
R^{3 -} für Wasserstoff, oder
- für eine Carboxyschutzgruppe, oder
- für einen in vivo spaltbaren Esterrest steht,
- für eine Gruppe der Formel 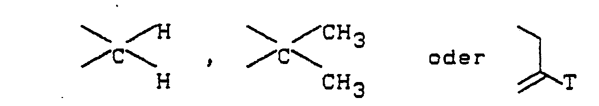 steht, oder die Gruppierung 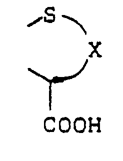 für die Gruppierung 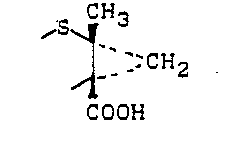 steht, worin
T - Wasserstoff oder Halogen bedeutet, oder
- geradkettiges, verzweigtes oder cyclisches Alkoxy oder Alkylthio mit jeweils bis zu 6 Kohlenstoffatomen bedeutet, oder
- geradkettiges, verzweigtes oder cyclisches Alkyl, Alkenyl oder Alkinyl mit jeweils bis zu 7 Kohlenstoffatomen bedeutet, die gegebenenfalls substituiert sein können durch Halogen, Hydroxy, Alkoxy oder Alkylthio mit bis zu 5 Kohlenstoffatomen, Aminocarbonyloxy, Acyloxy mit bis zu 10 Kohlenstoffatomen, oder durch einen Pyridiniumrest, der ein- oder mehrfach substituiert sein kann, oder durch einen Rest der Formel 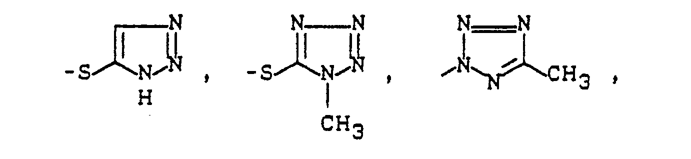 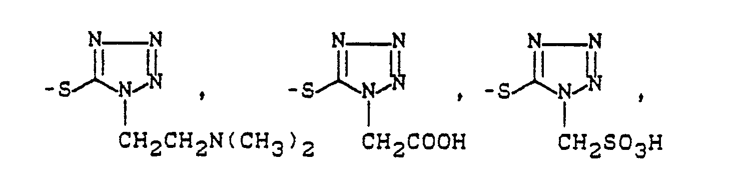 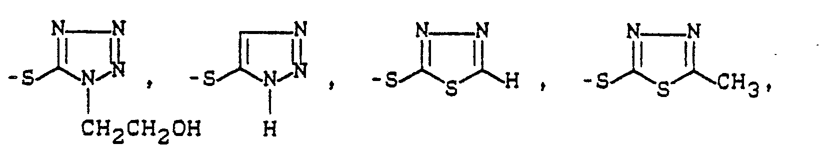 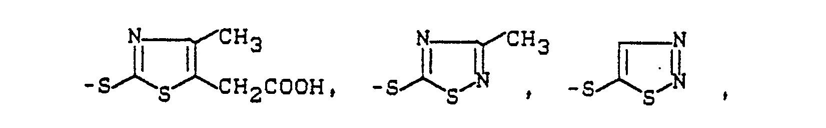 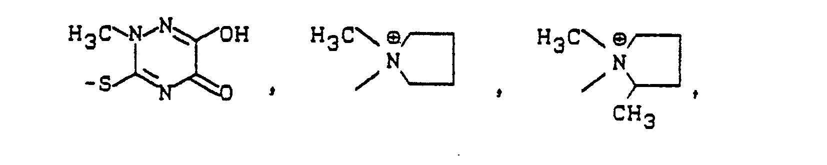 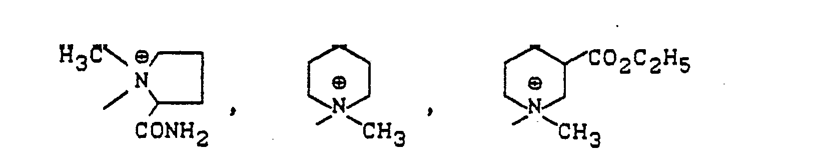 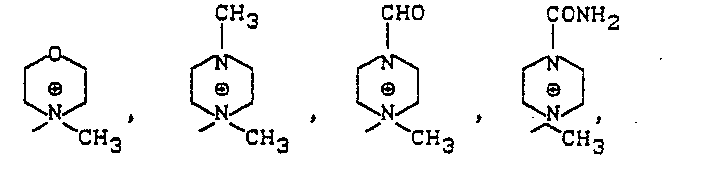 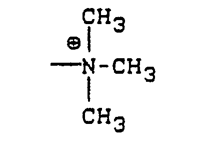 steht,
R⁴ - für Wasserstoff steht, oder
- für geradkettiges, verzweigtes oder cyclisches Alkyl mit bis zu 8 Kohlenstoffatomen steht, oder
- für gegebenenfalls substituiertes C₆-C₁₀-Aryl steht,
R^{S -} für Wasserstoff steht, oder
- für geradkettiges, verzweigtes oder cyclisches Alkyl mit bis zu 8 Kohlenstoffatomen steht, oder
- für einen 5- bis 6-gliedrigen heterocyclischen Rest steht, der als Heteroatome ein bis zwei Stickstoffatome, ein Sauerstoff und/oder ein Schwefelatom enthalten kann und der mehrfach gleich oder verschieden substituiert sein kann durch geradkettiges, verzweigtes oder cyclisches Alkyl, Alkenyl, Alkinyl mit jeweils bis zu 6 Kohlenstoffatomen, Cycloalkyl mit 3 bis 7 Kohlenstoffatomen, Alkoxy, Alkenyloxy, Alkylthio, Alkenylthio, Alkylsulfonyl oder Alkenylsulfonyl mit jeweils bis zu 6 Kohlenstoffatomen, durch Aryl, Aryloxy, Arylthio, Arylsulfonyl mit jeweils 6 bis 10 Kohlenstoffatomen, durch eine gegebenenfalls substituierte Aminogruppe, durch Oxo, Hydroxy, Mercapto, Cyano, Nitro, durch Alkylimino mit bis zu 6 Kohlenstoffatomen oder Arylimino mit 6 bis 10 Kohlenstoffatomen,
oder
R⁴ und R⁵ zusammen mit dem Stickstoffatom einen Ring der Formel 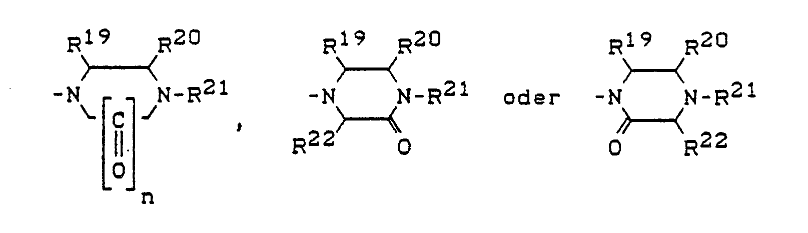 bildet, worin
R'⁹, R²⁰ gleich oder verschieden sind und Wasserstoff, geradkettiges, verzweigtes oder cyclisches Alkyl oder Alkoxy mit jeweils bis zu 4 Kohlenstoffatomen, Hydroxy, Amino, C₆-Cio-Aryl oder Halogen bedeuten, oder
- die Gruppierung der Formel 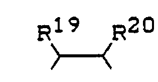 eine Gruppierung der Formel 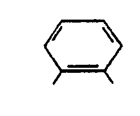 bedeutet,
R²¹⁻ Wasserstoff bedeutet, oder
- geradkettiges oder verzweigtes Alkyl oder Alkenyl mit jeweils bis zu 10 Kohlenstoffatomen bedeutet, oder
- Cycloalkyl mit 3 bis 7 Kohlenstoffatomen bedeutet, wobei bei den genannten Resten ein oder zwei CH₂-Gruppen durch CO, CS, CO₂, 0 oder S ersetzt sein können, oder
- gegebenenfalls substituiertes C₆-C₁₀-Aryl oder C₇-C₁₄-Aralkyl bedeutet, oder
- einen heterocyclischen Rest der Reihe Thienyl, Furyl, Pyridyl, Pyrimidyl, Oxazolyl, Thiazolyl, Oxadiazolyl, Thiadiazolyl, Benzoxazolyl, Benzthiazolyl, Benzoxadiazolyl, Benzthiadiazolyl, Chinolyl, Isochinolyl oder Indolyl bedeutet, wobei die genannten Heterocyclen bis zu 3-fach gleich oder verschieden substituiert sein können durch geradkettiges, verzweigtes oder cyclisches Alkyl, Alkoxy oder Alkylthio, Alkylsulfonyl mit jeweils bis zu 8 Kohlenstoffatomen, durch Trifluormethyl, Trifluormethoxy, Halogen, Nitro, Cyano, C_{G}-Cio-Aryl oder C₇-C₁₄-Aralkyl, oder R²¹⁻ eine Gruppe der Formel 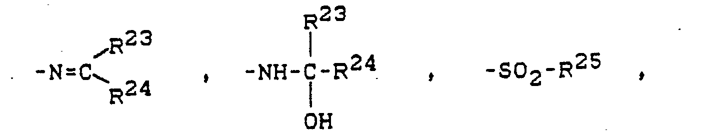 -COR²⁵ oder -CSR²⁵ bedeutet,
R22- Wasserstoff oder geradkettiges, verzweigtes oder cyclisches Alkyl mit bis zu 6 Kohlenstoffatomen bedeutet,
R²³, R²⁴ gleich oder verschieden sind und Wasserstoff, Cyano, Carboxy, Alkoxycarbonyl mit bis zu 6 Kohlenstoffatomen bedeuten, oder
- geradkettiges oder verzweigtes Alkyl oder Alkenyl mit jeweils bis zu 8 Kohlenstoffatomen bedeuten, oder
- Cycloalkyl mit 3 bis 7 Kohlenstoffatomen bedeuten, oder
- gegebenenfalls substituiertes C₆-C₁₀-Aryl oder C₇-C₁₄-Aralkyl bedeuten, oder
- einen Heterocyclus der Reihe Furyl, Thienyl, Pyridyl, Pyrimidyl, Oxazolyl, Thiazolyl, Oxadiazolyl, Thiadiazolyl, Benzoxazolyl oder Benzthiazolyl bedeuten, wobei die genannten Heterocyclen bis zu 3-fach gleich oder verschieden substituiert sein können durch geradkettiges, verzweigtes oder cyclisches Alkyl, Alkoxy oder Alkylthio mit jeweils bis zu 6 Kohlenstoffatomen, durch Halogen, Nitro, Cyano, Trifluormethyl oder Trifluormethoxy,
oder
R²³ und R²⁴ gemeinsam mit dem Kohlenstoffatom einen 3- bis 7-gliedrigen heterocyclischen Ring bilden, der als Heteroatome bis zu zwei Stickstoffe, ein Sauerstoff und/oder ein Schwefelatom enthalten kann, der gesättigt oder ungesättigt und benzokondensiert sein kann und der bis zu 3-fach gleich oder verschieden substituiert sein kann durch geradkettiges oder verzweigtes Alkyl oder Alkoxy mit jeweils bis zu 8 Kohlenstoffatomen, durch Cycloalkyl mit 3 bis 7 Kohlenstoffatomen, durch Halogen, Cyano, Nitro, Trifluormethyl oder Trifluormethoxy,
und
R²⁵⁻ Hydroxy, geradkettiges oder verzweigtes Alkyl, Alkenyl oder Alkoxy mit jeweils bis zu 8 Kohlenstoffatomen bedeutet, oder
- Cycloalkyl mit 3 bis 7 Kohlenstoffatomen bedeutet, oder
- gegebenenfalls substituiertes C₆-C_{10 o}-Aryl oder C₇-C₁₀ -Aralkyl bedeutet, oder
- eine gegebenenfalls substituierte Aminogruppe bedeutet, oder
- einen heterocyclischen Ring der Reihe Furyl, Thienyl, Pyridyl, Pyrimidyl, Thiazolyl, Oxazolyl, Thiadiazolyl oder Oxadiazolyl bedeutet, wobei die genannten heterocyclischen Reste bis zu 3-fach gleich oder verschieden substituiert sein können durch geradkettiges oder verzweigtes Alkyl, Alkoxy oder Alkylthio mit jeweils bis zu 6 Kohlenstoffatomen, durch Halogen, Nitro, Cyano, Trifluormethyl oder Trifluormethoxy
und deren Salze.

2. Verfahren zur Herstellung der heteroanellierten Phenylglycin-ß-Lactam-Antibiotika der allgemeinen Formel (I) nach Anspruchl, dadurch gekennzeichnet, daß man
A) Carbonsäuren der allgemeinen Formel (III) 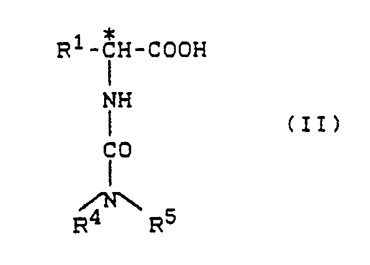 in welcher
R¹, R⁴ und R³ die im Anspruch 1 angegebene Bedeutung haben,
nach Aktivierung der Carboxylgruppe durch Überführung in ein gemischtes Anhydrid oder durch Überführen in das Säurehalogenid oder durch Überführen in einen aktivierten Ester, gegebenenfalls in Anwesenheit von N-Hydroxybenztriazol,
mit den ß-Lactamaminen der allgemeinen Formel (III), 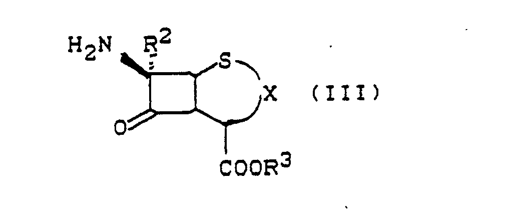 in welcher
R², R³ und X die in Anspruch 1 angegebene Bedeutung haben,
umsetzt,
dann gegebenenfalls Schutzgruppen abspaltet und die gewünschten Salze oder aus den Salzen die freien Säuren herstellt,
oder daß
B) Verbingungen der allgemeinen Formel (VI) 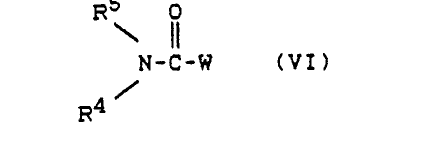 in welcher
R⁴ und R⁵ die oben angegebene Bedeutung haben,
und
W - für Halogen, Azid oder eine nucleofuge Abgangsgruppe steht,
in Gegenwart eines Lösemittels und gegebenenfalls eines Säurebindemittels bei Temperaturen von etwa -20° C bis etwa +50° C mit Verbindungen der allgemeinen Formel (VII) 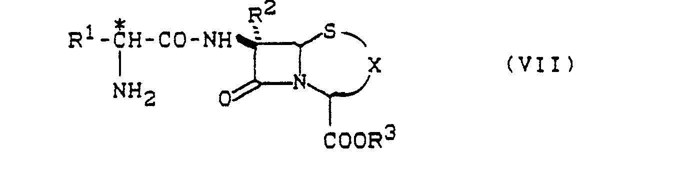 in welcher
R¹, R², R³ und X die oben angegebene Bedeutung haben,
umsetzt,
die erhaltenen β-Lactam-Antibiotika gegebenenfalls nach Abspaltung von Schutzgruppen, gegebenenfalls in ihre nicht-toxisch pharmazeutisch verträglichen Salze überführt, oder aus den gegebenenfalls erhaltenen Salze die freien Säuren herstellt.

3. Arzneimittel, enthaltend Verbindungen der Formel (I) nach Anspruch 1.

4. Verbindungen der Formel (I) nach Anspruch 1 zur Verwendung in einem Verfahren zur therapeutischen Behandlung des menschlichen oder tierischen Körpers.

5. Verwendung von Verbindungen der Formel (I) nach Anspruch 1 zur Herstellung von Arzneimitteln.
